# EUROPEAN PATENT APPLICATION

(11) **EP 4 083 035 A1**
(43) Date of publication of application: **02.11.2022**
(21) Application number: 20904733.1
(22) Date of filing: 17.12.2020
(51) Int. Cl.: C07D 405/14, C07D 495/04, C07D 405/04, C07D 491/048, H01L 51/00, H01L 51/50

(54) **HETEROCYCLIC COMPOUND, ORGANIC LIGHT-EMITTING DIODE COMPRISING SAME, AND COMPOSITION FOR ORGANIC LAYER OF ORGANIC LIGHT-EMITTING DIODE**

(30) Priority: 27.12.2019 KR 20190175840
(71) Applicant: LT Materials Co., Ltd., Yongin-si, Gyeonggi-do 17118 (KR)
(72) Inventor: PARK, Min-Ji, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2020/018560
(87) International publication number: WO 2021/132984

(57) **Abstract**

The present application provides a heterocyclic compound, and an organic light emitting device containing the heterocyclic compound in an organic material layer.

## Description

### [Technical Field]

The present specification claims priority to and the benefits of Korean Patent Application No. 10-2019-0178540, filed with the Korean Intellectual Property Office on December 27, 2019, the entire contents of which are incorporated herein by reference.

The present specification relates to a heterocyclic compound, an organic light emitting device comprising the same, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

(Patent Document 1) US Patent No. 4,356,429

### [Disclosure]

### [Technical Problem]

The present specification is directed to providing a heterocyclic compound, an organic light emitting device comprising the same, and a composition for an organic material layer of an organic light emitting device.

### [Technical Solution]

One embodiment of the present application provides a heterocyclic compound represented by the following Chemical Formula 1.

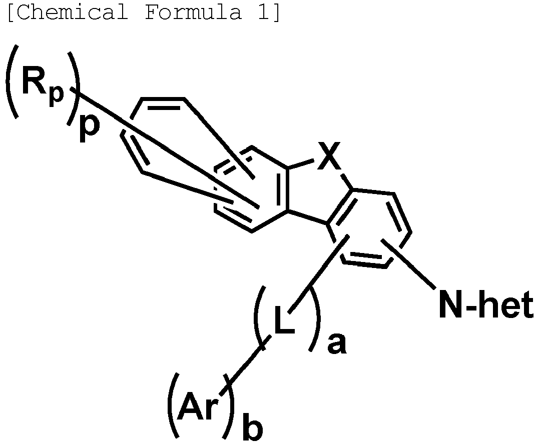

In Chemical Formula 1,
L is a direct bond; a substituted or unsubstituted arylene group having 6 to 60 carbon atoms; or a substituted or unsubstituted heteroarylene group having 2 to 60 carbon atoms,
X is O; or S,
N-het is a monocyclic or polycyclic heterocyclic group substituted or unsubstituted, and comprising one or more Ns,
Ar is a substituted or unsubstituted aryl group having 6 to 60 carbon atoms; a substituted or unsubstituted heteroaryl group having 2 to 60 carbon atoms; or an amine group unsubstituted or substituted with one or more selected from the group consisting of a substituted or unsubstituted aryl group having 6 to 60 carbon atoms and a substituted or unsubstituted heteroaryl group having 2 to 60 carbon atoms,
Rₚ is hydrogen; deuterium; a halogen group; a cyano group; a substituted or unsubstituted alkyl group having 1 to 30 carbon atoms; a substituted or unsubstituted cycloalkyl group having 3 to 30 carbon atoms; a substituted or unsubstituted aryl group having 6 to 40 carbon atoms; or a substituted or unsubstituted heteroaryl group having 2 to 40 carbon atoms,
a is an integer of 0 to 3, and when a is 2 or greater, substituents in the parentheses are the same as or different from each other,
b is an integer of 0 to 4, and when b is 2 or greater, substituents in the parentheses are the same as or different from each other, and
p is an integer of 0 to 6, and when p is 2 or greater, substituents in the parentheses are the same as or different from each other.

In addition, one embodiment of the present application provides an organic light emitting device comprising a first electrode; a second electrode; and one or more organic material layers provided between the first electrode and the second electrode, wherein one or more layers of the organic material layers comprise the heterocyclic compound represented by Chemical Formula 1.

Lastly, one embodiment of the present application provides a composition for an organic material layer of an organic light emitting device, the composition comprising two or more types of the heterocyclic compound represented by Chemical Formula 1.

### [Advantageous Effects]

A heterocyclic compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like.

Specifically, when using the heterocyclic compound represented by Chemical Formula 1 in the organic material layer, a driving voltage of the device can be lowered, light efficiency can be enhanced, and lifetime properties of the device can be enhanced.

### [Description of Drawings]

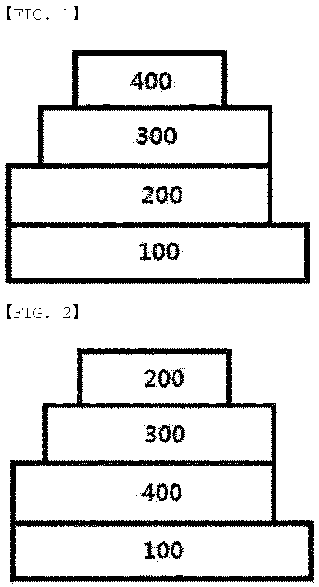

FIG. 1 to FIG. 3 are diagrams each illustrating a lamination structure of an organic light emitting device according to one embodiment of the present application.

### [Mode for Disclosure]

Hereinafter, the present specification will be described in more detail.

In the present specification, a certain part "comprising" certain constituents means capable of further comprising other constituents, and does not exclude other constituents unless particularly stated on the contrary.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of a linear or branched alkyl group having 1 to 60 carbon atoms; a linear or branched alkenyl group having 2 to 60 carbon atoms; a linear or branched alkynyl group having 2 to 60 carbon atoms; a monocyclic or polycyclic cycloalkyl group having 3 to 60 carbon atoms; a monocyclic or polycyclic heterocycloalkyl group having 2 to 60 carbon atoms; a monocyclic or polycyclic aryl group having 6 to 60 carbon atoms; a monocyclic or polycyclic heteroaryl group having 2 to 60 carbon atoms; a silyl group; a phosphine oxide group; and an amine group, or being unsubstituted, or being substituted with a substituent linking two or more substituents selected from among the substituents illustrated above, or being unsubstituted.

More specifically, "substituted or unsubstituted" in the present specification means being substituted with one or more substituents selected from the group consisting of a monocyclic or polycyclic aryl group having 6 to 60 carbon atoms; or a monocyclic or polycyclic heteroaryl group having 2 to 60 carbon atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the following spiro group may include any one of groups of the following structural formulae.

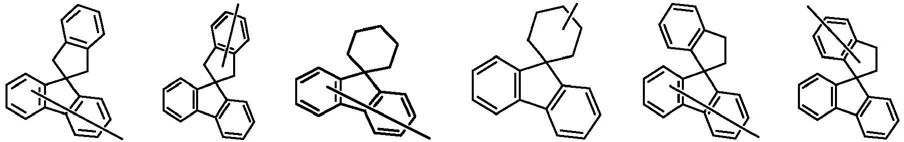

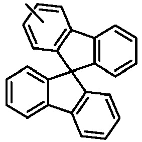

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 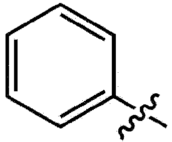 means that the total number of substituents that the phenyl group may have is 5 (T1 in the formula), and the number of deuterium among these is 1 (T2 in the formula) . In other words, having a deuterium content of 20% in a phenyl group may be represented by the following structural formulae.

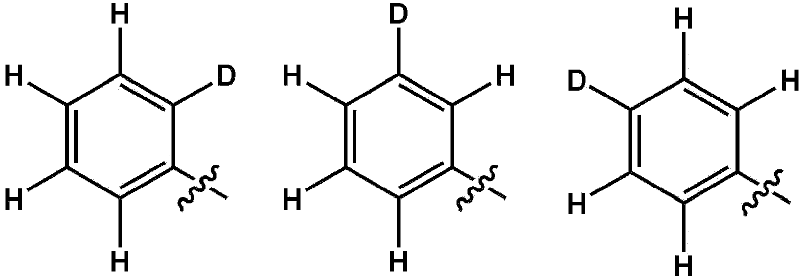

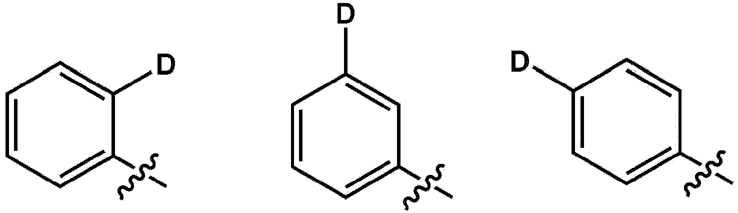

In addition, in one embodiment of the present application, "a phenyl group having a deuterium content of 0%" may mean a phenyl group that does not include a deuterium atom, that is, a phenyl group that has 5 hydrogen atoms.

One embodiment of the present application provides a heterocyclic compound represented by the following Chemical Formula 1.

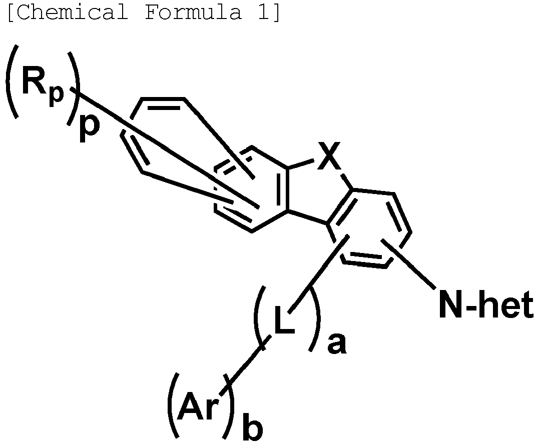

In Chemical Formula 1,
L is a direct bond; a substituted or unsubstituted arylene group having 6 to 60 carbon atoms; or a substituted or unsubstituted heteroarylene group having 2 to 60 carbon atoms,
X is O; or S,
N-het is a monocyclic or polycyclic heterocyclic group substituted or unsubstituted, and comprising one or more Ns,
Ar is a substituted or unsubstituted aryl group having 6 to 60 carbon atoms; a substituted or unsubstituted heteroaryl group having 2 to 60 carbon atoms; or an amine group unsubstituted or substituted with one or more selected from the group consisting of a substituted or unsubstituted aryl group having 6 to 60 carbon atoms and a substituted or unsubstituted heteroaryl group having 2 to 60 carbon atoms,
Rₚ is hydrogen; deuterium; a halogen group; a cyano group; a substituted or unsubstituted alkyl group having 1 to 30 carbon atoms; a substituted or unsubstituted cycloalkyl group having 3 to 30 carbon atoms; a substituted or unsubstituted aryl group having 6 to 40 carbon atoms; or a substituted or unsubstituted heteroaryl group having 2 to 40 carbon atoms,
a is an integer of 0 to 3, and when a is 2 or greater, substituents in the parentheses are the same as or different from each other,
b is an integer of 0 to 4, and when b is 2 or greater, substituents in the parentheses are the same as or different from each other, and
p is an integer of 0 to 6, and when p is 2 or greater, substituents in the parentheses are the same as or different from each other.

The heterocyclic compound represented by Chemical Formula 1 has, while having high thermal stability, proper molecular weight and band gap by N-het being linked without a linker. Using such a compound in a light emitting layer of an organic light emitting device prevents losses of electrons and holes helping to form an effective recombination zone of the electrons and the holes. When increasing recombination efficiency as above, current efficiency increases, and, when using the heterocyclic compound represented by Chemical Formula 1 as an organic material in an organic light emitting device, high efficiency and an increase in lifetime may be expected in the organic light emitting device.

In one embodiment of the present application, Chemical Formula 1 may be represented by any one of the following Chemical Formulae 1-1 to 1-3.

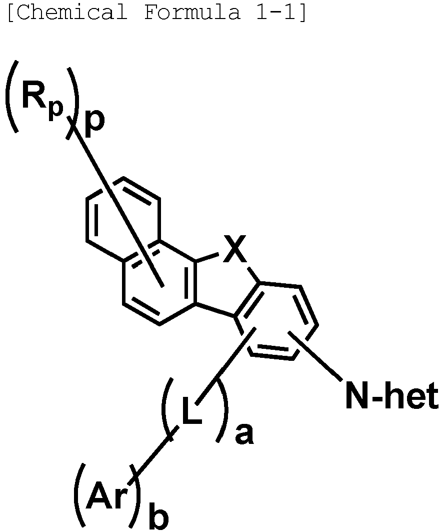

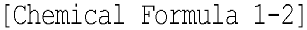

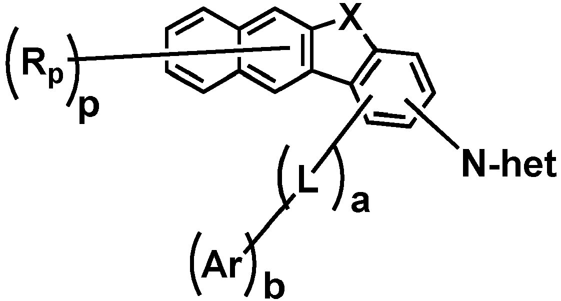

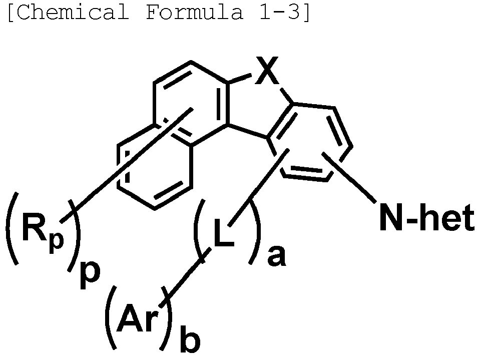

In Chemical Formulae 1-1 to 1-3,
each substituent has the same definition as in Chemical Formula 1.

In one embodiment of the present application, L of Chemical Formula 1 may be a direct bond; a substituted or unsubstituted arylene group having 6 to 60 carbon atoms; or a substituted or unsubstituted heteroarylene group having 2 to 60 carbon atoms.

In another embodiment, L may be a direct bond; a substituted or unsubstituted arylene group having 6 to 40 carbon atoms; or a substituted or unsubstituted heteroarylene group having 2 to 40 carbon atoms.

In another embodiment, L may be a direct bond; a substituted or unsubstituted arylene group having 6 to 20 carbon atoms; or a substituted or unsubstituted heteroarylene group having 2 to 20 carbon atoms.

In another embodiment, L may be a direct bond; a substituted or unsubstituted phenylene group; a substituted or unsubstituted naphthylene group; or a substituted or unsubstituted heteroarylene group having 2 to 20 carbon atoms.

In another embodiment, L may be a direct bond; a substituted or unsubstituted phenylene group; or a substituted or unsubstituted naphthylene group.

In another embodiment, L may be a direct bond; a phenylene group; or a naphthylene group.

In another embodiment, L is a direct bond.

In another embodiment, L is a phenylene group.

In another embodiment, L is a naphthylene group.

In one embodiment of the present application, X of Chemical Formula 1 may be O; or S.

In one embodiment of the present application, X is O.

In one embodiment of the present application, X is S.

In one embodiment of the present application, N-het of Chemical Formula 1 may be a monocyclic or polycyclic heterocyclic group substituted or unsubstituted, and comprising one or more Ns.

In another embodiment, N-het may be a monocyclic or polycyclic heterocyclic group substituted or unsubstituted, and comprising one or more and three or less Ns.

In another embodiment, N-het may be a monocyclic or polycyclic heterocyclic group having 2 to 60 carbon atoms unsubstituted or substituted with one or more substituents selected from the group consisting of an aryl group having 6 to 60 carbon atoms and a heteroaryl group having 2 to 60 carbon atoms, and comprising one or more Ns.

In another embodiment, N-het may be a monocyclic or polycyclic heterocyclic group having 2 to 60 carbon atoms unsubstituted or substituted with one or more substituents selected from the group consisting of an aryl group having 6 to 60 carbon atoms and a heteroaryl group having 2 to 60 carbon atoms, and comprising one or more and three or less Ns.

In another embodiment, N-het may be represented by the following Chemical Formula A.

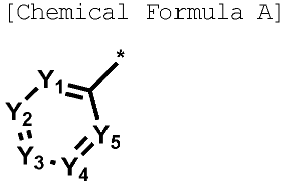

In Chemical Formula A,
* is a position at which Chemical Formula 1 and N-het bond,
Y₁ to Y₅ are the same as or different from each other and each independently N or CRa, at least one or more of Y₁ to Y₅ are N, and when there are two or more CRas, Ras are the same as or different from each other, and
Ra is selected from the group consisting of hydrogen; deuterium; a substituted or unsubstituted aryl group having 6 to 60 carbon atoms; and a substituted or unsubstituted heteroaryl group having 2 to 60 carbon atoms, or two or more groups adjacent to each other may bond to each other to form a substituted or unsubstituted aromatic hydrocarbon ring having 6 to 60 carbon atoms or a substituted or unsubstituted heteroring having 2 to 60 carbon atoms.

In one embodiment of the present application, Chemical Formula A may be represented by any one of the following Chemical Formulae A-1 to A-7.

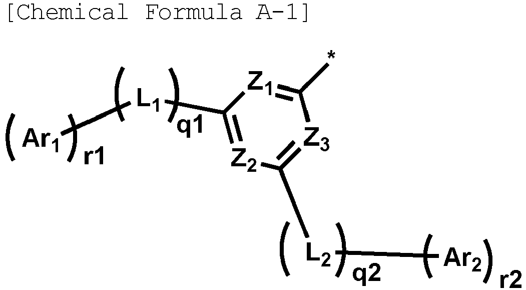

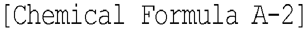

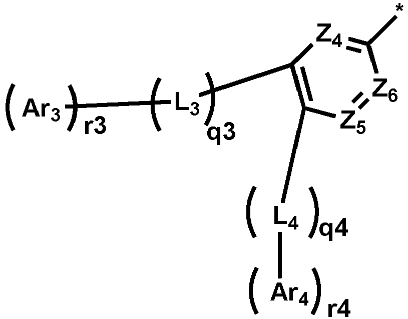

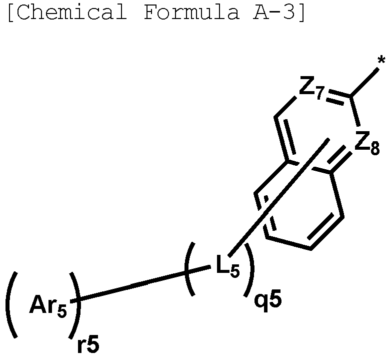

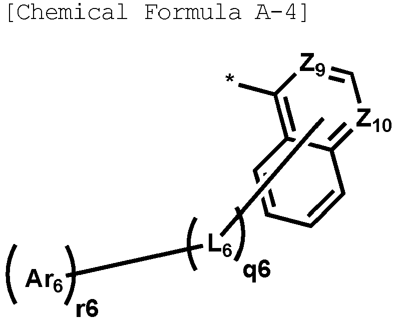

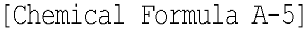

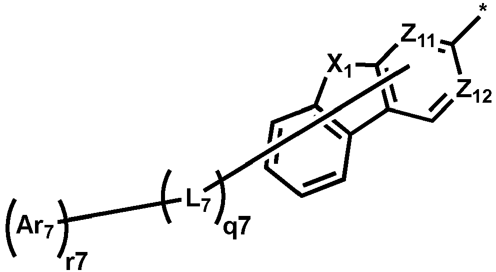

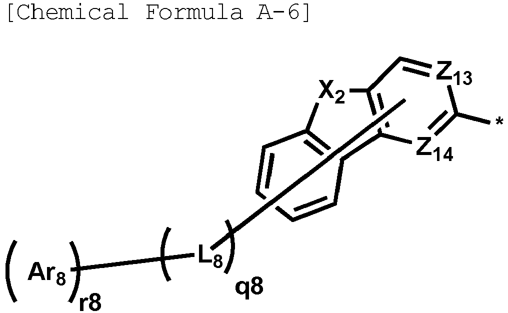

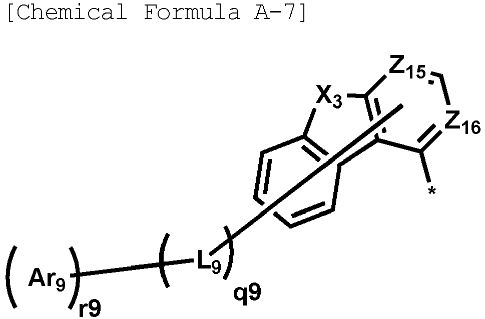

In Chemical Formulae A-1 to A-7,
* is a position at which Chemical Formula 1 and N-het bond,
Z₁ to Z₁₆ are the same as or different from each other and each independently N or CH, at least one or more of Z₁ to Z₃ are N, at least one or more of Z₄ to Z₆ are N, at least one or more of Z₇ and Z₈ are N, at least one or more of Z₉ and Z₁₀ are N, at least one or more of Z₁₁ and Z₁₂ are N, at least one or more of Z₁₃ and Z₁₄ are N, and at least one or more of Z₁₅ and Z₁₆ are N,
L₁ to Lg are the same as or different from each other, and each independently a direct bond; a substituted or unsubstituted arylene group having 6 to 60 carbon atoms; or a substituted or unsubstituted heteroarylene group having 2 to 60 carbon atoms,
Ar₁ to Arg are the same as or different from each other, and each independently hydrogen; deuterium; a substituted or unsubstituted aryl group having 6 to 60 carbon atoms; or a substituted or unsubstituted heteroaryl group having 2 to 60 carbon atoms,
X₁ to X₃ are the same as or different from each other, and each independently O; or S,
q1 to q9 are each independently an integer of 0 to 2, and when q1 to q9 are each 2, substituents in the parentheses are the same as or different from each other, and
r1 to r4 are each independently an integer of 1 to 3, r5 to r9 are each independently an integer of 1 to 5, and when r1 to q9 are each 2 or greater, substituents in the parentheses are the same as or different from each other.

In one embodiment of the present application, Z₁ to Z₃ of Chemical Formula A-1 are the same as or different from each other and each independently N or CH, and at least one or more of Z₁ to Z₃ may be N.

In one embodiment of the present application, Z₁ is N, and Z₂ and Z₃ are CH.

In one embodiment of the present application, Z₂ is N, and Z₁ and Z₃ are CH.

In one embodiment of the present application, Z₃ is N, and Z₁ and Z₂ are CH.

In one embodiment of the present application, Z₁ is CH, and Z₂ and Z₃ are N.

In one embodiment of the present application, Z₂ is CH, and Z₁ and Z₃ are N.

In one embodiment of the present application, Z₃ is CH, and Z₁ and Z₂ are N.

In one embodiment of the present application, Z₁ to Z₃ are N.

In one embodiment of the present application, Z₄ to Z₆ of Chemical Formula A-2 are the same as or different from each other and each independently N or CH, and at least one or more of Z₄ to Z₆ may be N.

In one embodiment of the present application, Z₄ is N, and Z₅ and Z₆ are CH.

In one embodiment of the present application, Z₅ is N, and Z₄ and Z₆ are CH.

In one embodiment of the present application, Z₆ is N, and Z₄ and Z₅ are CH.

In one embodiment of the present application, Z₄ is CH, and Z₅ and Z₆ are N.

In one embodiment of the present application, Z₅ is CH, and Z₄ and Z₆ are N.

In one embodiment of the present application, Z₆ is CH, and Z₄ and Z₅ are N.

In one embodiment of the present application, Z₄ to Z₆ are N.

In one embodiment of the present application, Z₇ and Z₈ of Chemical Formula A-3 are the same as or different from each other and each independently N or CH, and at least one or more of Z₇ and Z₈ may be N.

In one embodiment of the present application, Z₇ is N, and Z₈ is CH.

In one embodiment of the present application, Z₈ is N, and Z₇ is CH.

In one embodiment of the present application, Z₇ and Z₈ are N.

In one embodiment of the present application, Z₉ and Z₁₀ of Chemical Formula A-4 are the same as or different from each other and each independently N or CH, and at least one or more of Z₉ and Z₁₀ may be N.

In one embodiment of the present application, Z₉ is N, and Z₁₀ is CH.

In one embodiment of the present application, Z₁₀ is N, and Z₉ is CH.

In one embodiment of the present application, Z₉ and Z₁₀ are N.

In one embodiment of the present application, Z₁₁ and Z₁₂ of Chemical Formula A-5 are the same as or different from each other and each independently N or CH, and at least one or more of Z₁₁ and Z₁₂ may be N.

In one embodiment of the present application, Z₁₁ is N, and Z₁₂ is CH.

In one embodiment of the present application, Z₁₂ is N, and Z₁₁ is CH.

In one embodiment of the present application, Z₁₁ and Z₁₂ are N.

In one embodiment of the present application, Z₁₃ and Z₁₄ of Chemical Formula A-6 are the same as or different from each other and each independently N or CH, and at least one or more of Z₁₃ and Z₁₄ may be N.

In one embodiment of the present application, Z₁₃ is N, and Z₁₄ is CH.

In one embodiment of the present application, Z₁₄ is N, and Z₁₃ is CH.

In one embodiment of the present application, Z₁₃ and Z₁₄ are N.

In one embodiment of the present application, Z₁₅ and Z₁₆ of Chemical Formula A-7 are the same as or different from each other and each independently N or CH, and at least one or more of Z₁₅ and Z₁₆ may be N.

In one embodiment of the present application, Z₁₅ is N, and Z₁₆ is CH.

In one embodiment of the present application, Z₁₆ is N, and Z₁₅ is CH.

In one embodiment of the present application, Z₁₅ and Z₁₆ are N.

In one embodiment of the present application, L₁ to L₉ of Chemical Formulae A-1 to A-7 are the same as or different from each other, and may be each independently a direct bond; a substituted or unsubstituted arylene group having 6 to 60 carbon atoms; or a substituted or unsubstituted heteroarylene group having 2 to 60 carbon atoms.

In one embodiment of the present application, L₁ to L₉ are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L₁ to L₉ are the same as or different from each other, and may be each independently a direct bond; a substituted or unsubstituted arylene group having 6 to 20 carbon atoms; or a substituted or unsubstituted heteroarylene group having 2 to 20 carbon atoms.

In one embodiment of the present application, L₁ to L₉ are the same as or different from each other, and may be each independently a direct bond; or a substituted or unsubstituted arylene group having 6 to 20 carbon atoms.

In one embodiment of the present application, L₁ to L₉ are the same as or different from each other, and may be each independently a direct bond; a substituted or unsubstituted phenylene group; or a substituted or unsubstituted naphthylene group.

In one embodiment of the present application, L₁ to L₉ are the same as or different from each other, and may be each independently a direct bond; a phenylene group; or a naphthylene group.

In another embodiment, L₁ is a direct bond.

In another embodiment, L₁ is a phenylene group.

In another embodiment, L₁ is a naphthylene group.

In another embodiment, L₂ is a direct bond.

In another embodiment, L₂ is a phenylene group.

In another embodiment, L₂ is a naphthylene group.

In another embodiment, L₃ is a direct bond.

In another embodiment, L₃ is a phenylene group.

In another embodiment, L₃ is a naphthylene group.

In another embodiment, L₄ is a direct bond.

In another embodiment, L₄ is a phenylene group.

In another embodiment, L₄ is a naphthylene group.

In another embodiment, L₅ is a direct bond.

In another embodiment, L₅ is a phenylene group.

In another embodiment, L₅ is a naphthylene group.

In another embodiment, L₆ is a direct bond.

In another embodiment, L₆ is a phenylene group.

In another embodiment, L₆ is a naphthylene group.

In another embodiment, L₇ is a direct bond.

In another embodiment, L₇ is a phenylene group.

In another embodiment, L₇ is a naphthylene group.

In another embodiment, L₈ is a direct bond.

In another embodiment, L₈ is a phenylene group.

In another embodiment, L₈ is a naphthylene group.

In another embodiment, Lg is a direct bond.

In another embodiment, Lg is a phenylene group.

In another embodiment, Lg is a naphthylene group.

In one embodiment of the present application, X₁ to X₃ of Chemical Formulae A-5 to A-7 are the same as or different from each other, and may be each independently O; or S.

In one embodiment of the present application, X₁ may be O; or S.

In one embodiment of the present application, X₁ is O.

In one embodiment of the present application, X₁ is S.

In one embodiment of the present application, X₂ may be O; or S.

In one embodiment of the present application, X₂ is O.

In one embodiment of the present application, X₂ is S.

In one embodiment of the present application, X₃ may be O; or S.

In one embodiment of the present application, X₃ is O.

In one embodiment of the present application, X₃ is S.

In one embodiment of the present application, q1 to q9 of Chemical Formulae A-1 to A-7 are each independently an integer of 0 to 2, and when q1 to q9 are each 2, substituents in the parentheses may be the same as or different from each other.

In one embodiment of the present application, Ar₁ to Arg of Chemical Formulae A-1 to A-7 are the same as or different from each other, and may be each independently hydrogen; deuterium; a substituted or unsubstituted aryl group having 6 to 60 carbon atoms; or a substituted or unsubstituted heteroaryl group having 2 to 60 carbon atoms.

In one embodiment of the present application, Ar₁ to Arg are the same as or different from each other, and may be each independently a substituted or unsubstituted aryl group having 6 to 60 carbon atoms; or a substituted or unsubstituted heteroaryl group having 2 to 60 carbon atoms.

In one embodiment of the present application, Ar₁ to Arg are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar₁ to Arg are the same as or different from each other, and may be each independently a substituted or unsubstituted phenyl group; a substituted or unsubstituted biphenyl group; a substituted or unsubstituted terphenyl group; a substituted or unsubstituted triphenyl group; a substituted or unsubstituted dibenzofuran group; a substituted or unsubstituted dibenzothiophene group; or a substituted or unsubstituted carbazolyl group.

In one embodiment of the present application, Ar₁ to Arg are the same as or different from each other, and may be each independently a phenyl group; a biphenyl group; a terphenyl group; a triphenyl group; a dibenzofuran group; a dibenzothiophene group; or a carbazolyl group.

In one embodiment of the present application, r1 to r4 of Chemical Formulae A-1 to A-7 are each independently an integer of 1 to 3, r5 to r8 are each independently an integer of 1 to 5, and when r1 to r9 are each 2 or greater, substituents in the parentheses are the same as or different from each other.

In another embodiment, N-het may be a pyridyl group; a pyrimidyl group; a pyridazinyl group; a pyrazolyl group; a triazinyl group; a quinolyl group; an isoquinolyl group; a quinazolinyl group; an isoquinazolinyl group; a quinozolilyl group; a naphthyridyl group; a benzothiazole group; or a benzo[4,5]thieno[2,3-d]pyrimidine group unsubstituted or substituted with one or more substituents selected from the group consisting of an aryl group having 6 to 60 carbon atoms and a heteroaryl group having 2 to 60 carbon atoms.

In one embodiment of the present application, Ar of Chemical Formula 1 may be a substituted or unsubstituted aryl group having 6 to 60 carbon atoms; a substituted or unsubstituted heteroaryl group having 2 to 60 carbon atoms; or an amine group unsubstituted or substituted with one or more selected from the group consisting of a substituted or unsubstituted aryl group having 6 to 60 carbon atoms and a substituted or unsubstituted heteroaryl group having 2 to 60 carbon atoms.

When Ar of Chemical Formula 1 is an amine group, stronger donor properties are obtained, and using the compound in an organic material layer of an organic light emitting device may further improve efficiency of the organic light emitting device by resolving a hole trap phenomenon of a dopant by further helping with hole injection.

In addition, the compound in which Ar of Chemical Formula 1 is an amine group has lower HOMO and LUMO levels, and therefore, using the compound with a compound in which Ar of Chemical Formula 1 is an aryl group or a heteroaryl group in an organic material layer of an organic light emitting device may further improve efficiency and lifetime of the organic light emitting device by having an energy level difference advantageous for exciplex formation.

In one embodiment of the present application, Ar may be a substituted or unsubstituted aryl group having 6 to 60 carbon atoms; a substituted or unsubstituted heteroaryl group having 2 to 60 carbon atoms; or a group represented by the following Chemical Formula B.

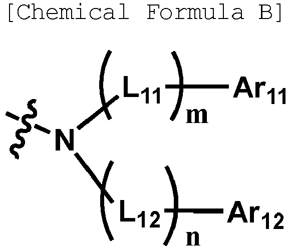

In Chemical Formula B,
L₁₁ and L₁₂ are the same as or different from each other, and each independently a direct bond; a substituted or unsubstituted arylene group having 6 to 40 carbon atoms; or a substituted or unsubstituted heteroarylene group having 2 to 40 carbon atoms,
Ar₁₁ and Ar₁₂ are the same as or different from each other, and each independently selected from the group consisting of a substituted or unsubstituted aryl group having 6 to 40 carbon atoms; and a substituted or unsubstituted heteroaryl group having 2 to 40 carbon atoms, or Ar₁₁ and Ar₁₂ bond to each other to form a substituted or unsubstituted aromatic hydrocarbon ring having 6 to 60 carbon atoms or a substituted or unsubstituted heteroring having 2 to 60 carbon atoms,
m and n are each 0 or 1, and 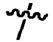
means a position bonding to L of Chemical Formula 1.

In one embodiment of the present application, Chemical Formula B may be represented by any one of the following Chemical Formulae B-1 to B-5.

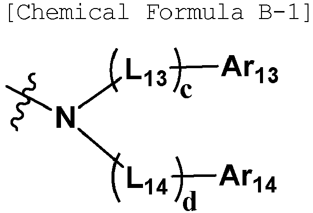

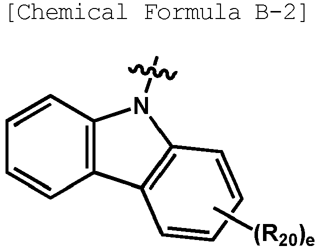

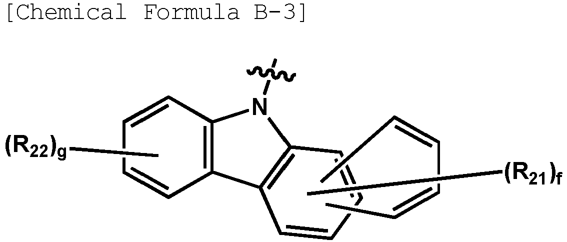

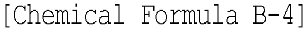

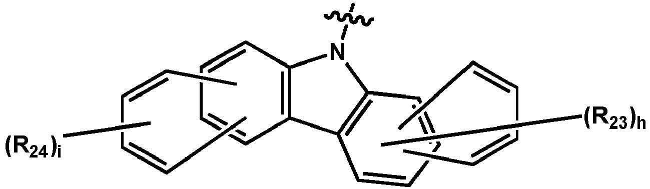

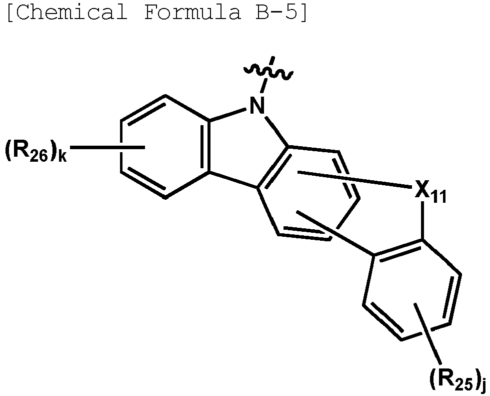

In Chemical Formulae B-1 to B-5,
L₁₃ and L₁₄ are the same as or different from each other, and each independently a direct bond; a substituted or unsubstituted arylene group having 6 to 40 carbon atoms; or a substituted or unsubstituted heteroarylene group having 2 to 40 carbon atoms,
Ar₁₃ and Ar₁₄ are the same as or different from each other, and each independently a substituted or unsubstituted aryl group having 6 to 40 carbon atoms; or a substituted or unsubstituted heteroaryl group having 2 to 40 carbon atoms,
R₂₀ to R₂₆ are each independently hydrogen; deuterium; a halogen group; a cyano group; a substituted or unsubstituted aryl group having 6 to 20 carbon atoms; or a substituted or unsubstituted heteroaryl group having 2 to 20 carbon atoms,
X₁₁ is O; S; or NRc, and Rc is a substituted or unsubstituted aryl group having 6 to 20 carbon atoms,
c and d are each 0 or 1,
e, g, j and k are each an integer of 0 to 4, f, h and i are each an integer of 0 to 6, and when e to k are each 2 or greater, substituents in the parentheses are the same as or different from each other, and 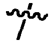
means a position bonding to L of Chemical Formula 1.

In one embodiment of the present application, L₁₃ and L₁₄ of Chemical Formula B-1 are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L₁₃ and L₁₄ of Chemical Formula B-1 are the same as or different from each other, and may be each independently a direct bond; a substituted or unsubstituted arylene group having 6 to 20 carbon atoms; or a substituted or unsubstituted heteroarylene group having 2 to 20 carbon atoms.

In one embodiment of the present application, L₁₃ and L₁₄ are the same as or different from each other, and may be each independently a direct bond; or a substituted or unsubstituted arylene group having 6 to 20 carbon atoms.

In one embodiment of the present application, L₁₃ and L₁₄ are the same as or different from each other, and may be each independently a direct bond; a substituted or unsubstituted phenylene group; a substituted or unsubstituted biphenylene group; or a substituted or unsubstituted naphthylene group.

In one embodiment of the present application, L₁₃ and L₁₄ are the same as or different from each other, and may be each independently a direct bond; a phenylene group; a biphenylene group; or a naphthylene group.

In one embodiment of the present application, L₁₃ is a direct bond.

In one embodiment of the present application, L₁₃ is a phenylene group.

In one embodiment of the present application, L₁₃ is a biphenylene group.

In one embodiment of the present application, L₁₃ is a naphthylene group.

In one embodiment of the present application, L₁₄ is a direct bond.

In one embodiment of the present application, L₁₄ is a phenylene group.

In one embodiment of the present application, L₁₄ is a biphenylene group.

In one embodiment of the present application, L₁₄ is a naphthylene group.

In one embodiment of the present application, Ar₁₃ and Ar₁₄ of Chemical Formula B-1 are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar₁₃ and Ar₁₄ are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar₁₃ and Ar₁₄ are the same as or different from each other, and may be each independently a substituted or unsubstituted phenyl group; a substituted or unsubstituted biphenyl group; a substituted or unsubstituted naphthyl group; a substituted or unsubstituted fluorenyl group; a substituted or unsubstituted dibenzothiophene group; or a substituted or unsubstituted dibenzofuran group.

In one embodiment of the present application, Ar₁₃ and Ar₁₄ are the same as or different from each other, and may be each independently a substituted or unsubstituted phenyl group; a substituted or unsubstituted biphenyl group; a substituted or unsubstituted naphthyl group; a fluorenyl group unsubstituted or substituted with one or more selected from the group consisting of a phenyl group and an alkyl group having 1 to 10 carbon atoms; a substituted or unsubstituted dibenzothiophene group; or a substituted or unsubstituted dibenzofuran group.

In one embodiment of the present application, Ar₁₃ and Ar₁₄ are the same as or different from each other, and may be each independently a substituted or unsubstituted phenyl group; a substituted or unsubstituted biphenyl group; a substituted or unsubstituted naphthyl group; a fluorenyl group unsubstituted or substituted with one or more selected from the group consisting of a phenyl group and a methyl group; a substituted or unsubstituted dibenzothiophene group; or a substituted or unsubstituted dibenzofuran group.

In one embodiment of the present application, Ar₁₃ and Ar₁₄ are the same as or different from each other, and may be each independently a phenyl group; a biphenyl group; a naphthyl group; a fluorenyl group unsubstituted or substituted with one or more selected from the group consisting of a phenyl group and a methyl group; a substituted or unsubstituted dibenzothiophene group; or a substituted or unsubstituted dibenzofuran group.

In one embodiment of the present application, c and d of Chemical Formula B-1 may each be 0 or 1.

In one embodiment of the present application, R₂₀ to R₂₆ of Chemical Formulae B-1 to B-5 may be each independently hydrogen; deuterium; a halogen group; a cyano group; a substituted or unsubstituted aryl group having 6 to 20 carbon atoms; or a substituted or unsubstituted heteroaryl group having 2 to 20 carbon atoms.

In one embodiment of the present application, R₂₀ to R₂₆ may be each independently hydrogen; deuterium; or a substituted or unsubstituted aryl group having 6 to 20 carbon atoms.

In one embodiment of the present application, R₂₀ to R₂₆ may be each independently hydrogen; deuterium; or a substituted or unsubstituted phenyl group.

In one embodiment of the present application, R₂₀ to R₂₆ may be each independently hydrogen; or a phenyl group.

In one embodiment of the present application, R₂₀ may be hydrogen; or a phenyl group.

In one embodiment of the present application, X₁₁ of Chemical Formula B-5 is O; S; or NRc, and Rc may be a substituted or unsubstituted aryl group having 6 to 20 carbon atoms.

In one embodiment of the present application, X₁₁ is O; S; or NRc, and Rc may be a phenyl group.

In one embodiment of the present application, X₁₁ is O.

In one embodiment of the present application, X₁₁ is S.

In one embodiment of the present application, X₁₁ is NRc, and Rc is a phenyl group.

In one embodiment of the present application, X of Chemical Formula 1 may be O; or S.

In one embodiment of the present application, X is O.

In one embodiment of the present application, X is S.

In one embodiment of the present application, a of Chemical Formula 1 is an integer of 0 to 3, and when a is 2 or greater, substituents in the parentheses are the same as or different from each other.

In one embodiment of the present application, a is 3.

In one embodiment of the present application, a is 2.

In one embodiment of the present application, a is 1.

In one embodiment of the present application, a is 0.

In one embodiment of the present application, b of Chemical Formula 1 is an integer of 0 to 4, and when b is 2 or greater, substituents in the parentheses are the same as or different from each other.

In one embodiment of the present application, b is 4.

In one embodiment of the present application, b is 3.

In one embodiment of the present application, b is 2.

In one embodiment of the present application, b is 1.

In one embodiment of the present application, b is 0.

In one embodiment of the present application, Rₚ of Chemical Formula 1 may be hydrogen; deuterium; a halogen group; a cyano group; a substituted or unsubstituted alkyl group having 1 to 30 carbon atoms; a substituted or unsubstituted cycloalkyl group having 3 to 30 carbon atoms; a substituted or unsubstituted aryl group having 6 to 40 carbon atoms; or a substituted or unsubstituted heteroaryl group having 6 to 40 carbon atoms.

In one embodiment of the present application, Rₚ of Chemical Formula 1 is hydrogen.

In one embodiment of the present application, p of Chemical Formula 1 is an integer of 0 to 6, and when p is 2, substituents in the parentheses are the same as or different from each other.

In one embodiment of the present application, p is 6.

In one embodiment of the present application, p is 5.

In one embodiment of the present application, p is 4.

In one embodiment of the present application, p is 3.

In one embodiment of the present application, p is 2.

In one embodiment of the present application, p is 1.

In one embodiment of the present application, p is 0.

In the heterocyclic compound provided in one embodiment of the present application, Chemical Formula 1 is represented by any one of the following compounds.

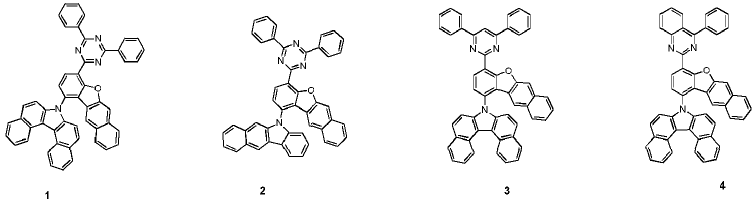

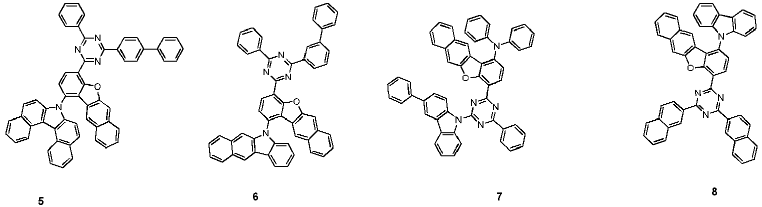

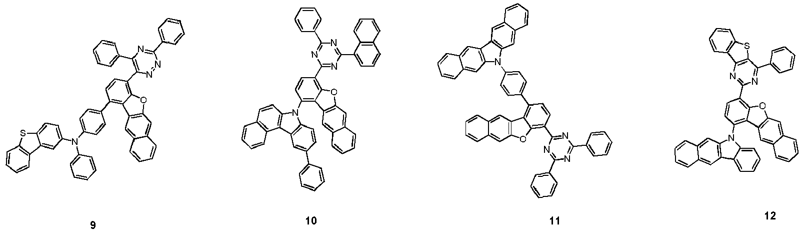

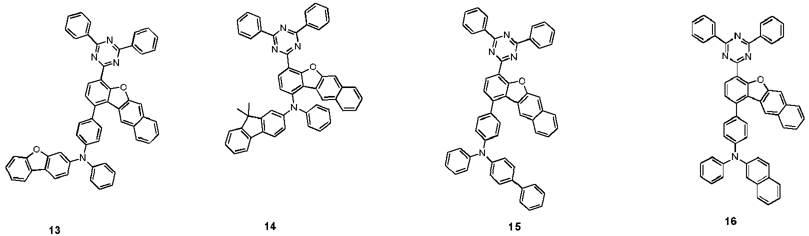

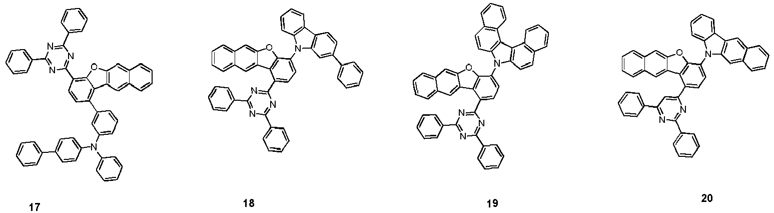

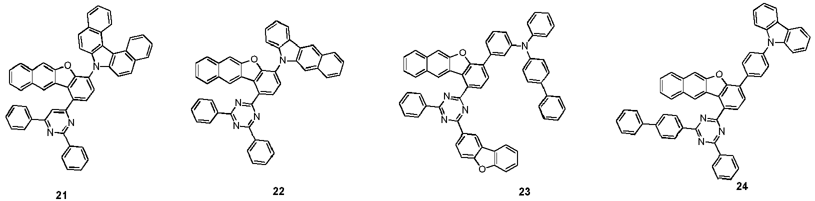

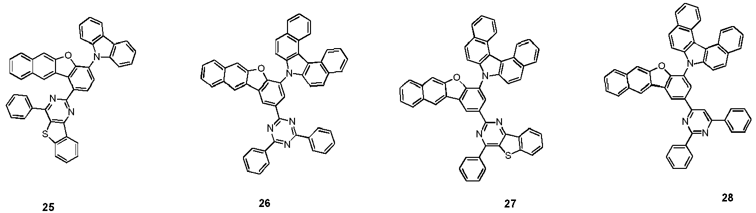

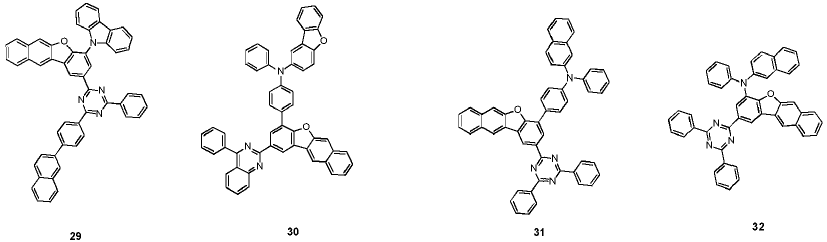

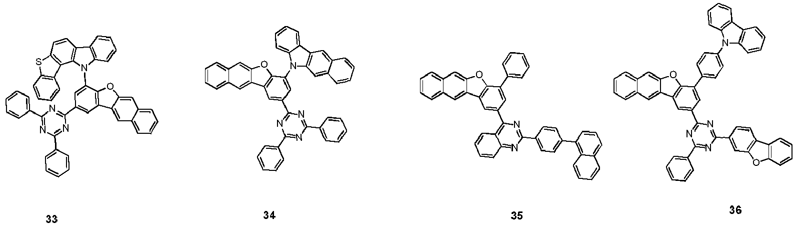

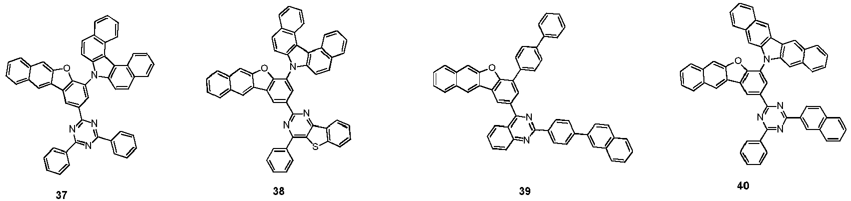

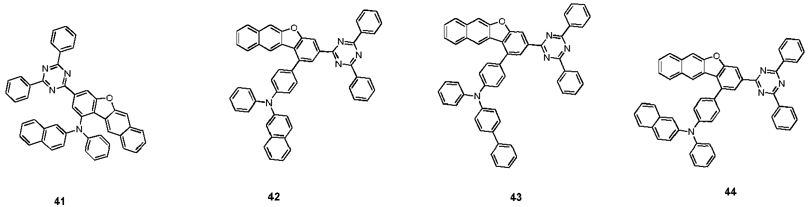

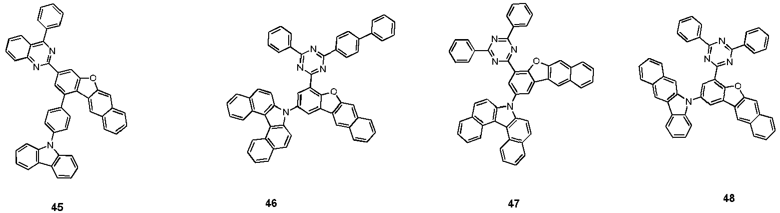

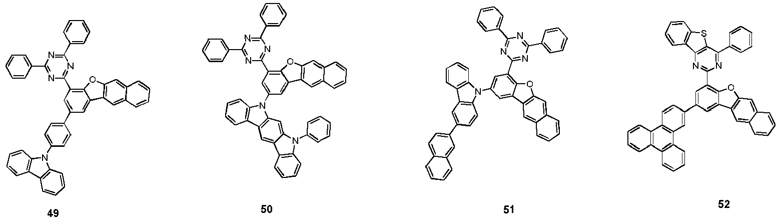

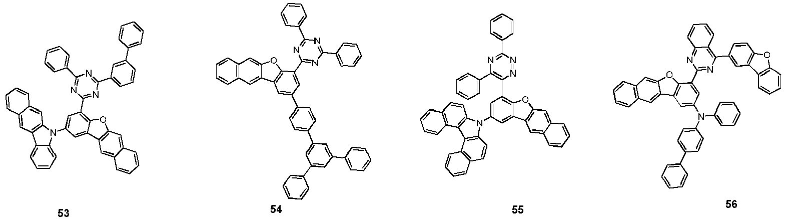

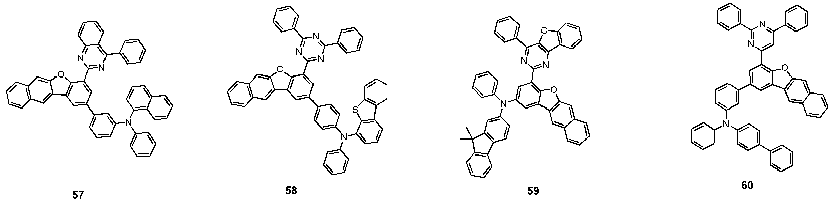

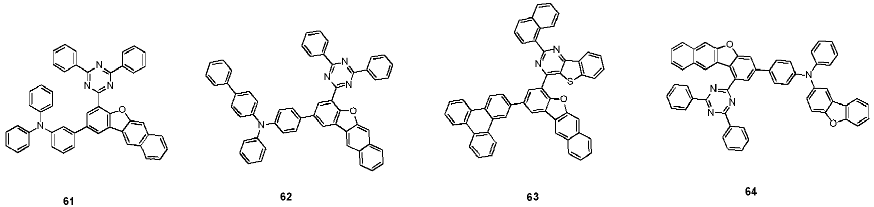

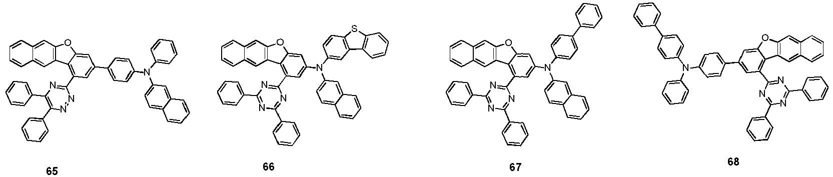

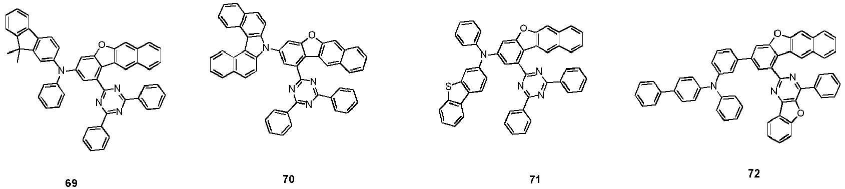

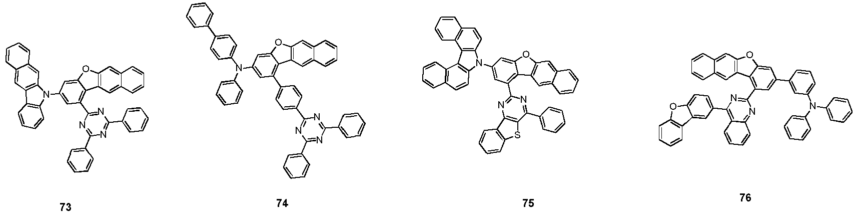

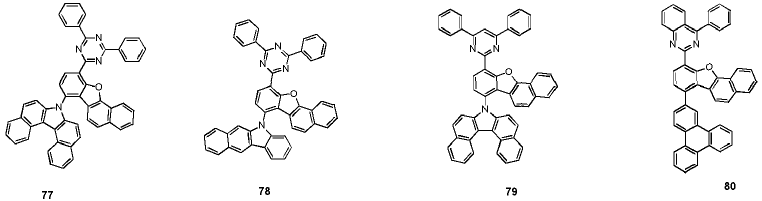

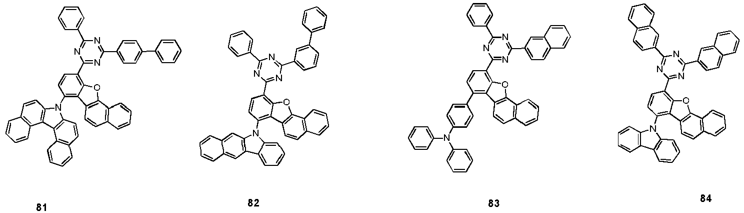

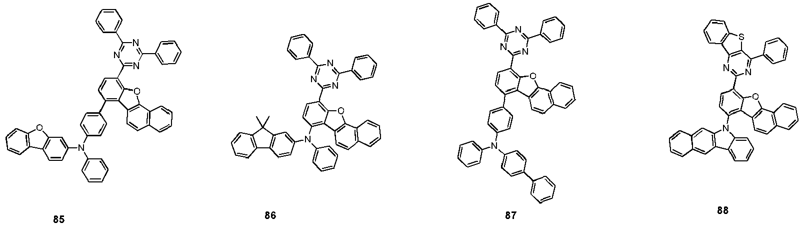

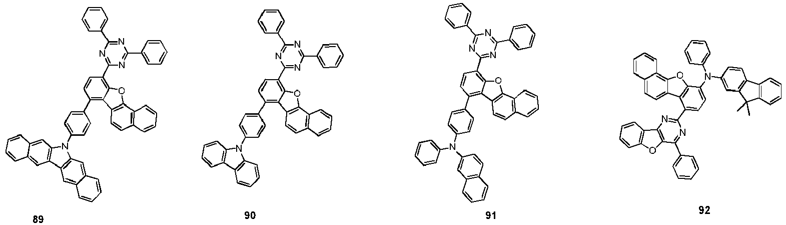

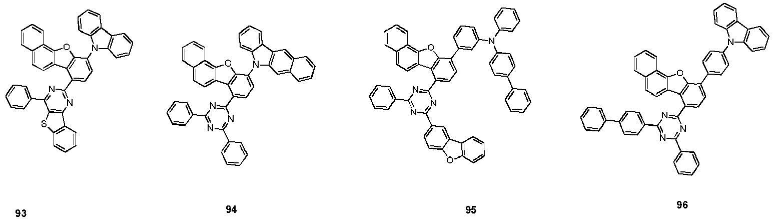

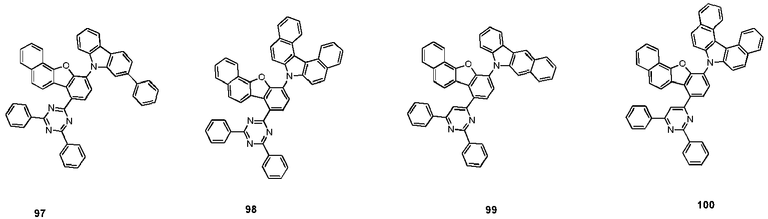

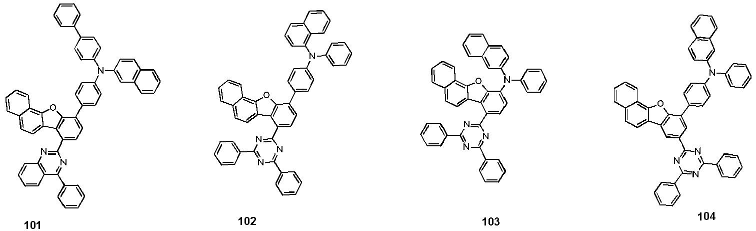

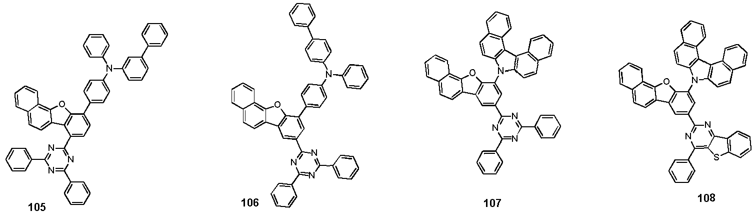

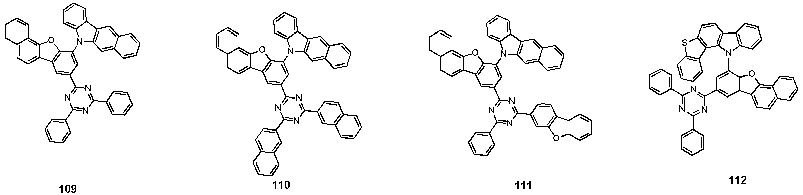

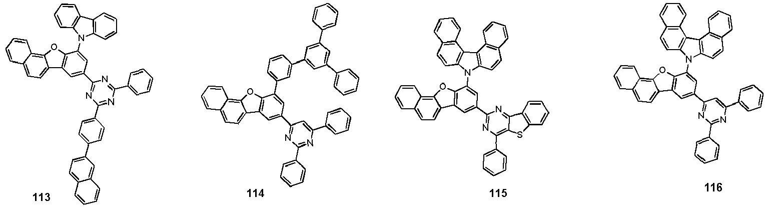

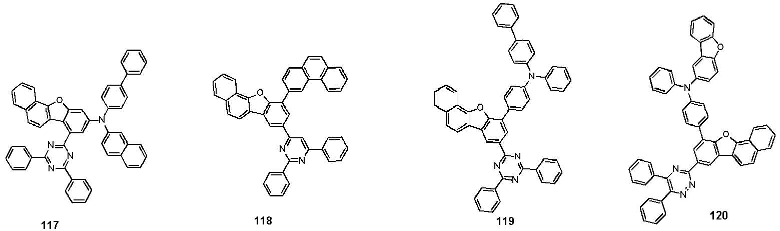

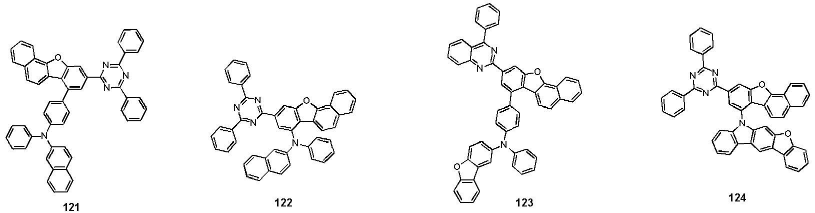

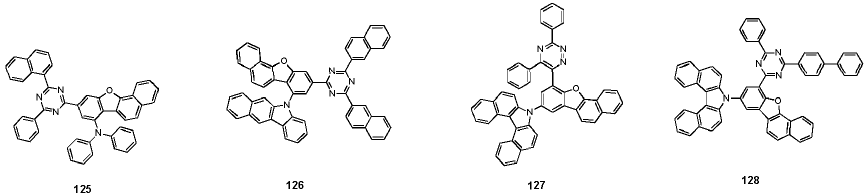

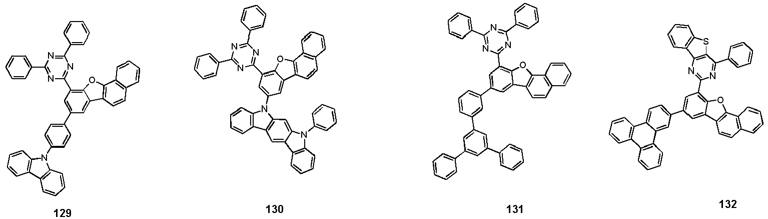

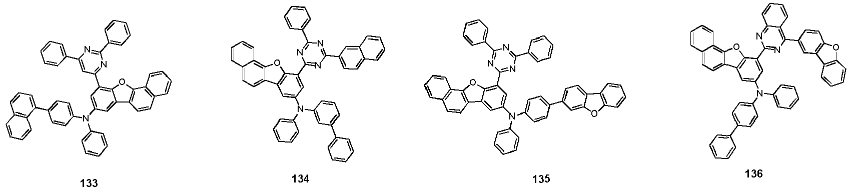

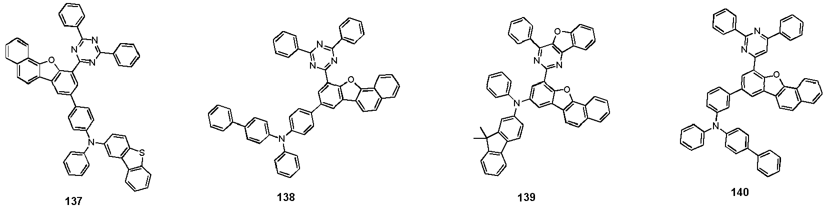

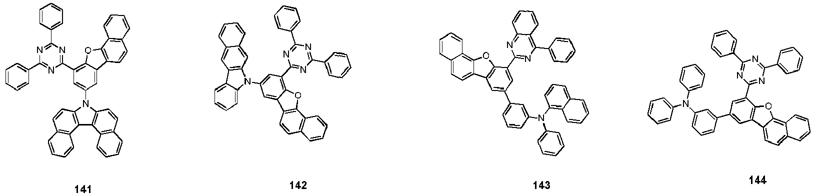

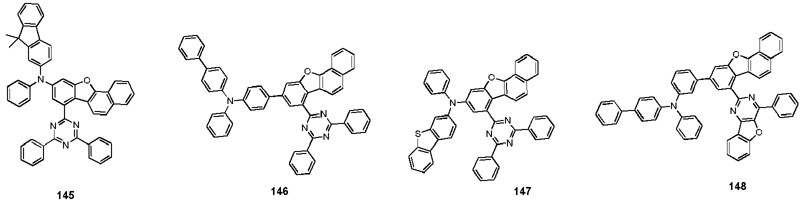

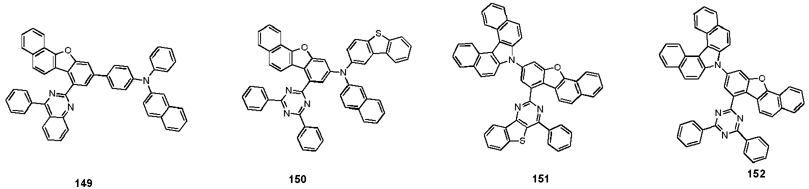

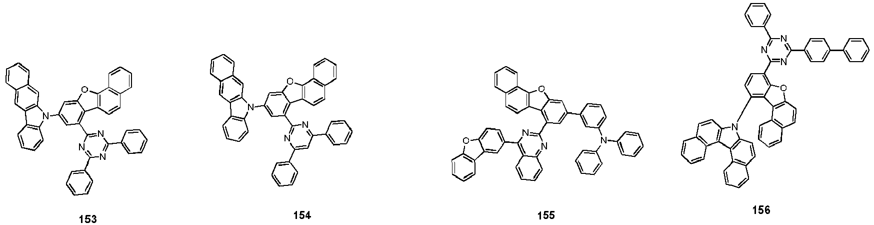

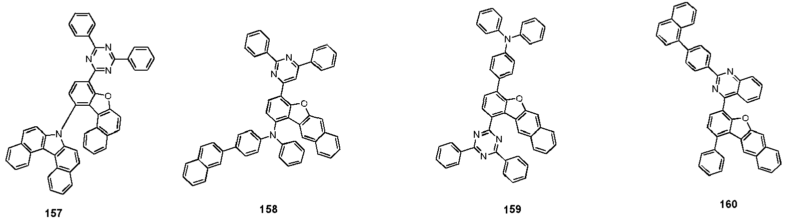

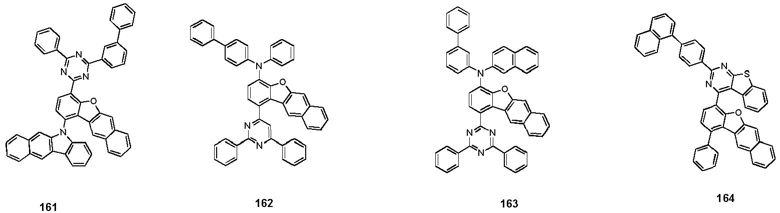

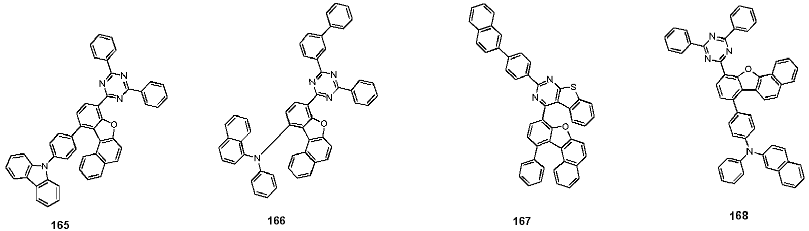

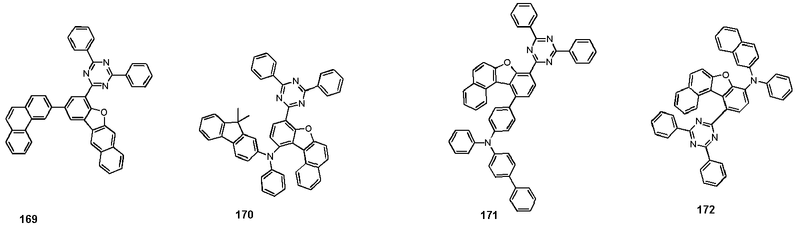

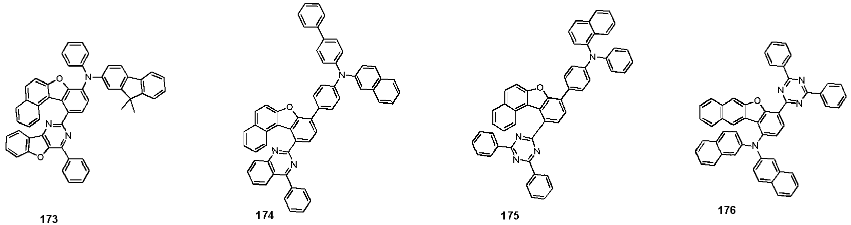

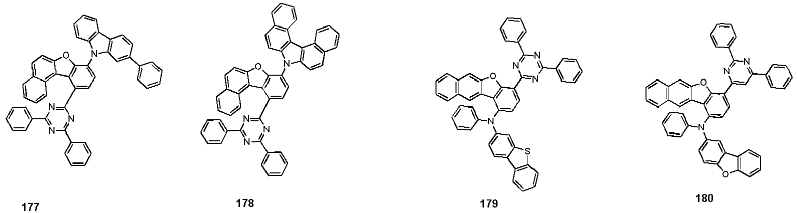

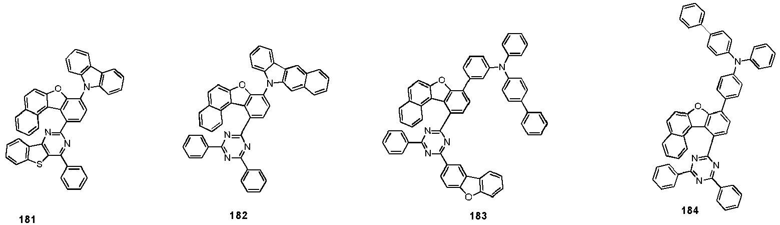

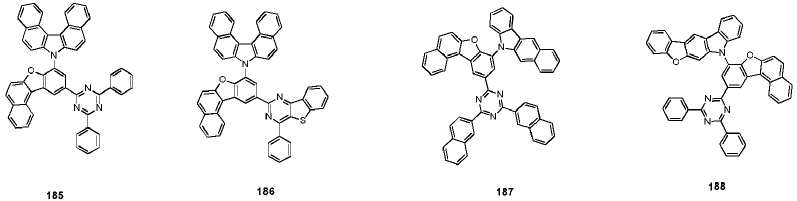

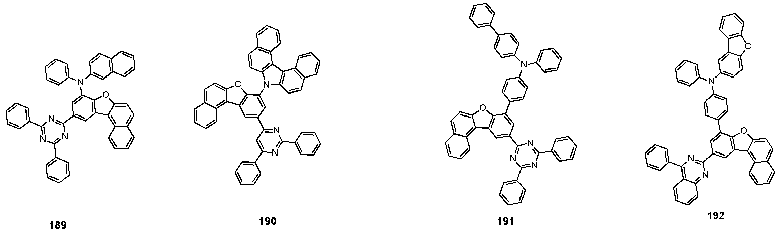

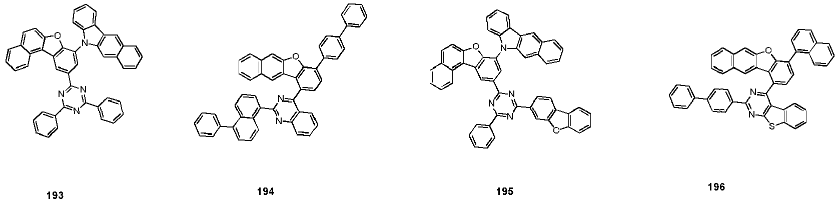

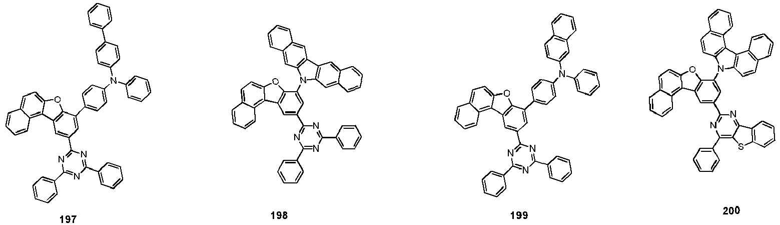

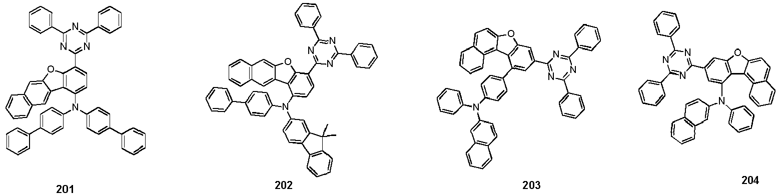

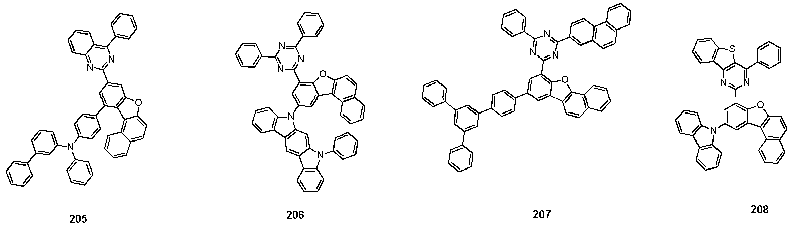

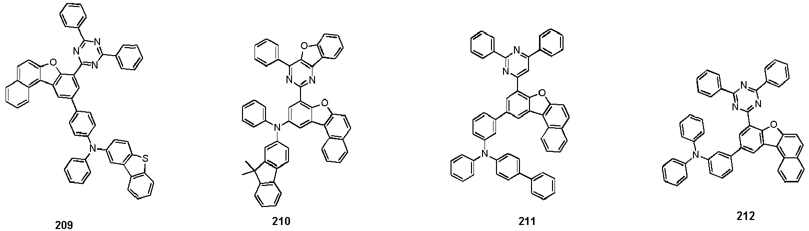

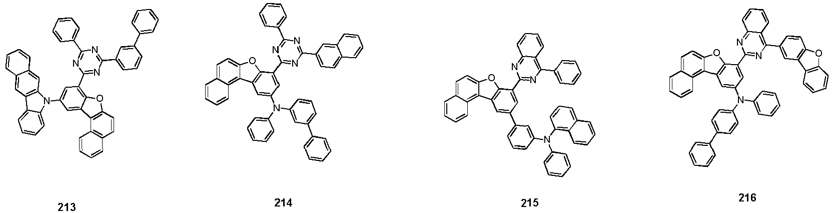

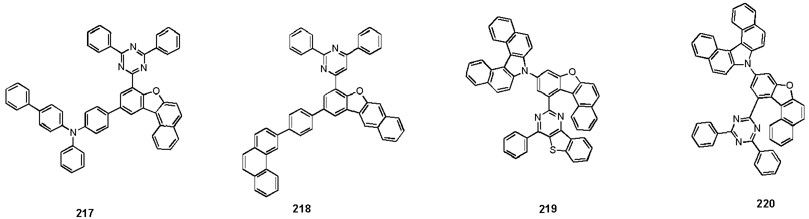

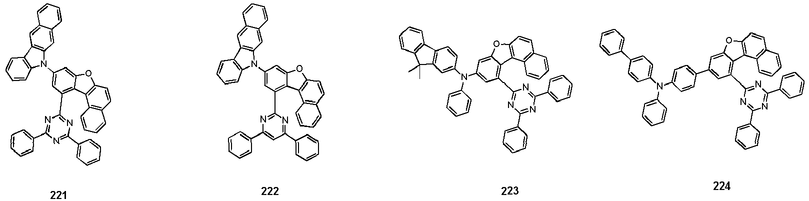

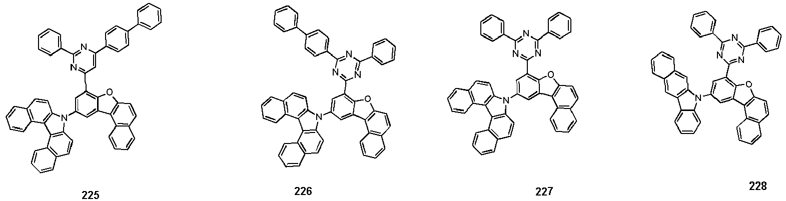

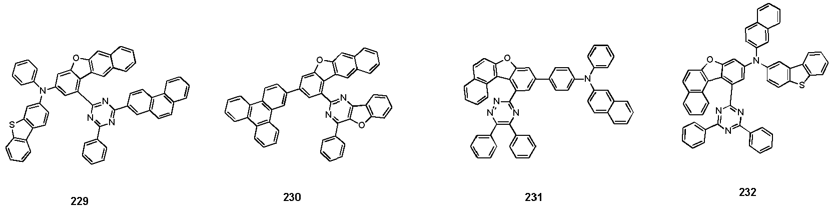

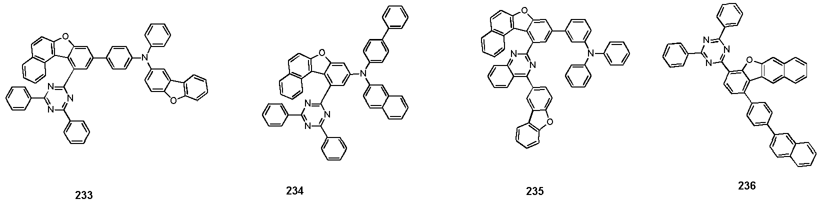

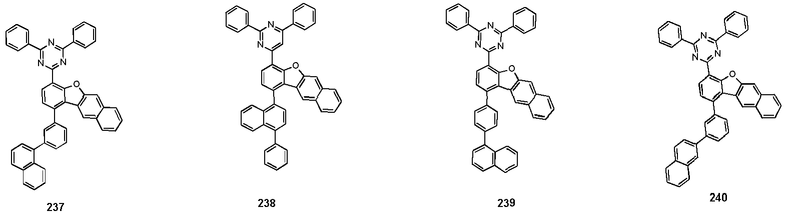

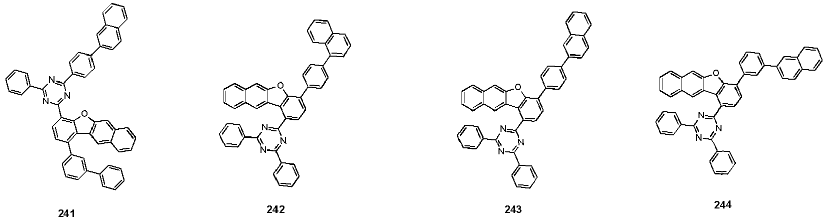

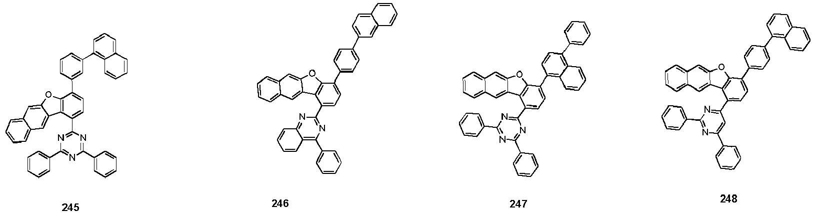

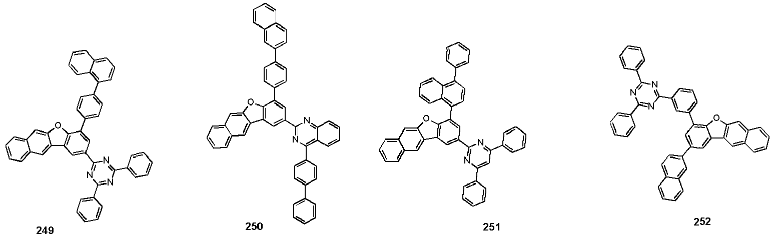

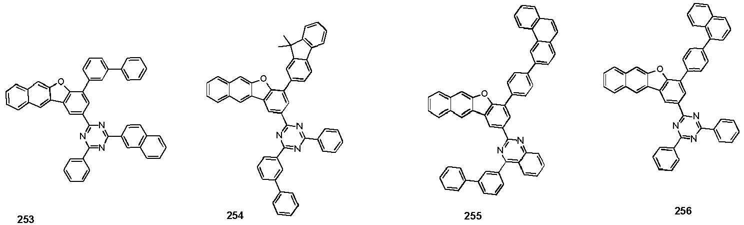

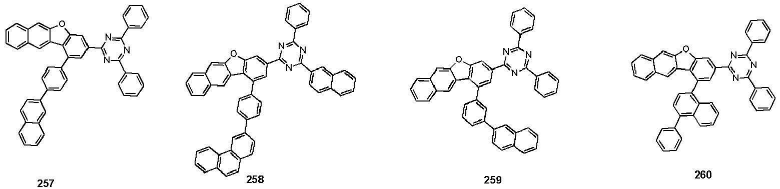

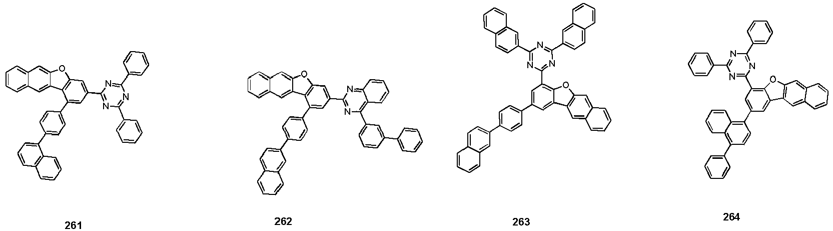

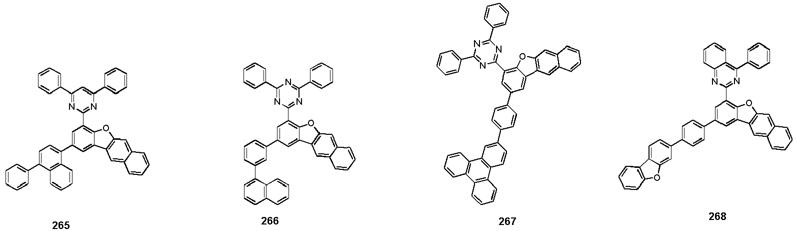

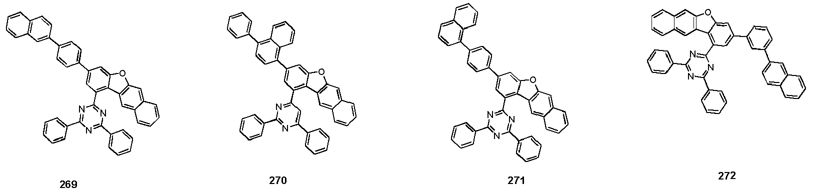

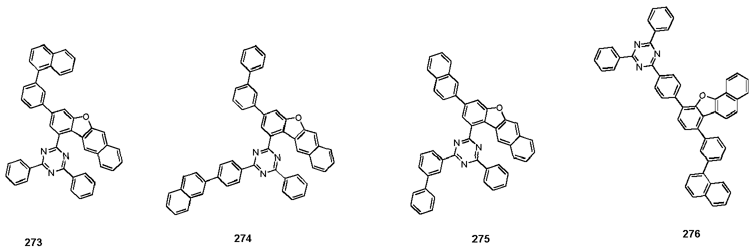

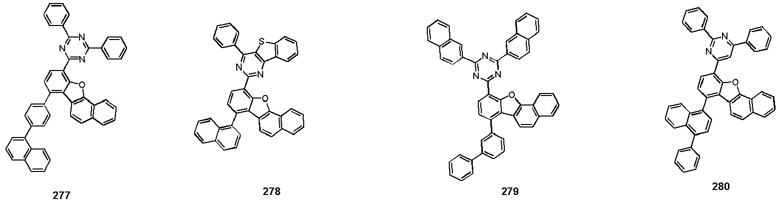

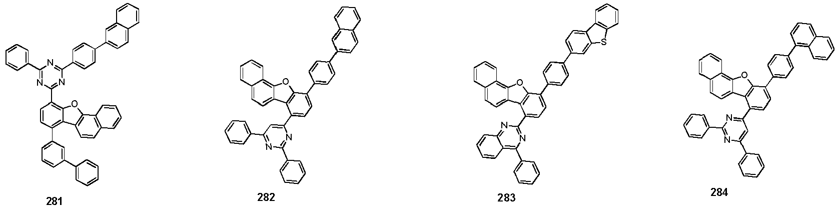

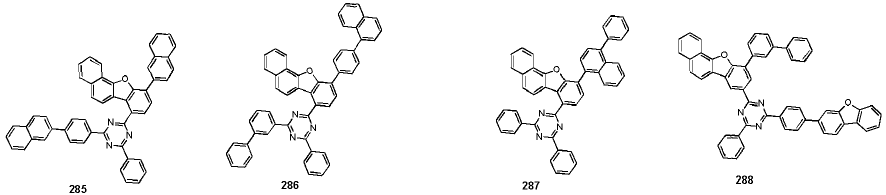

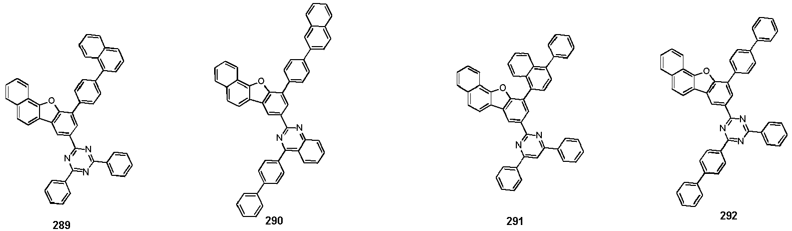

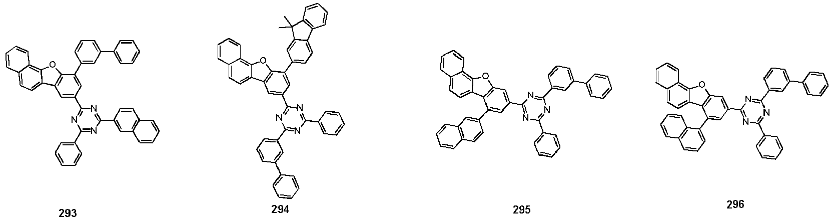

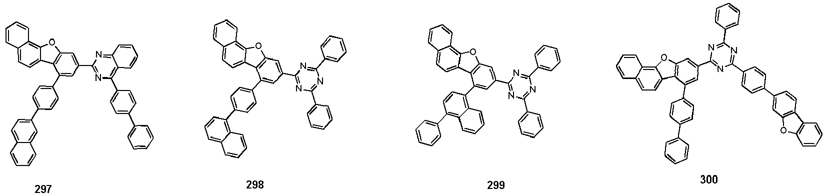

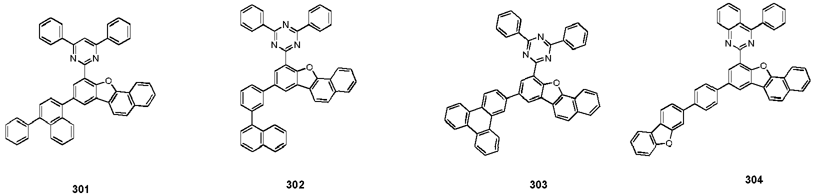

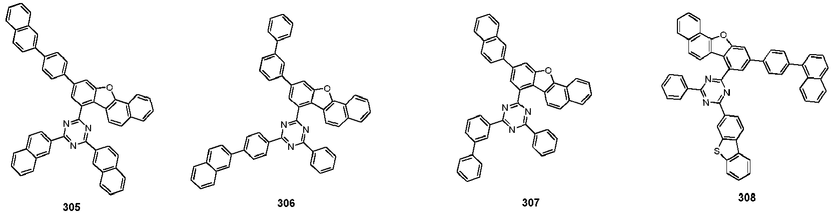

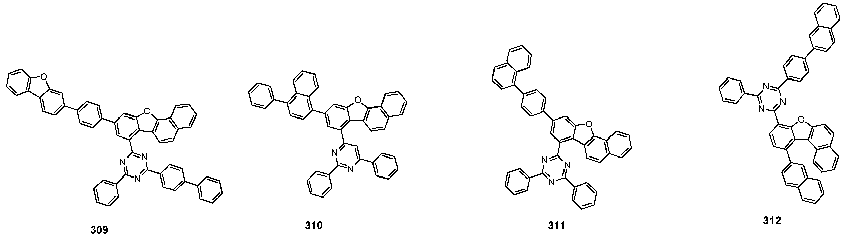

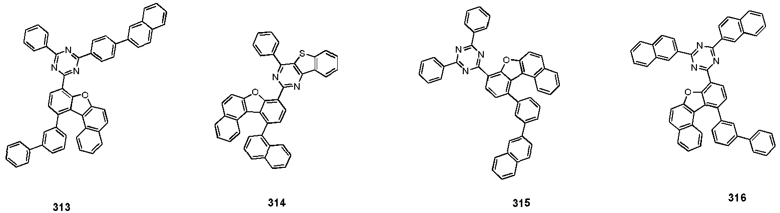

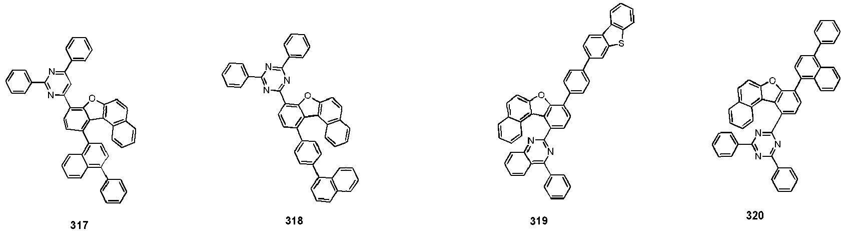

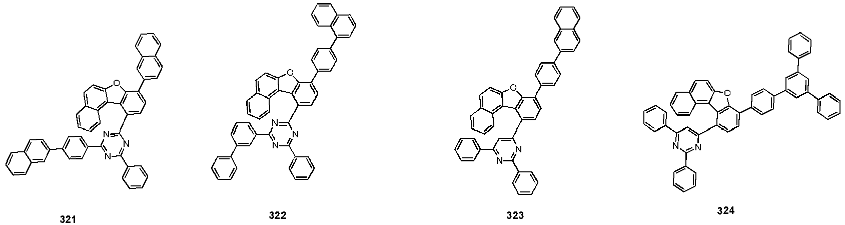

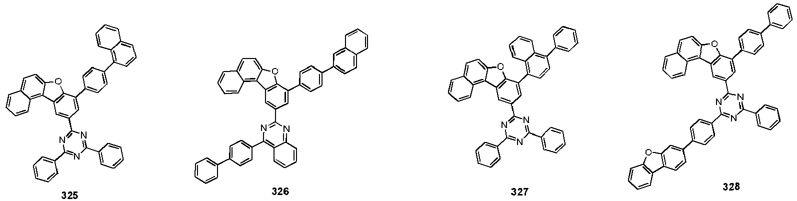

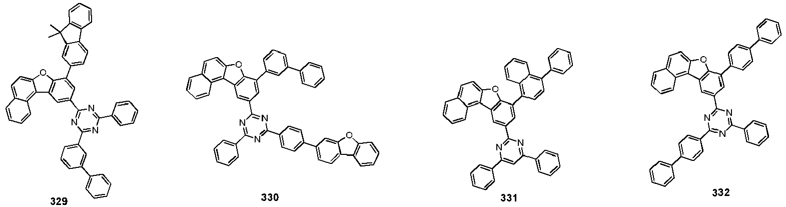

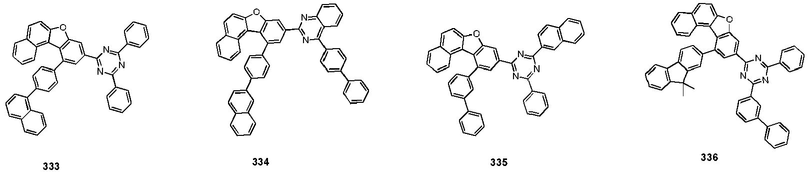

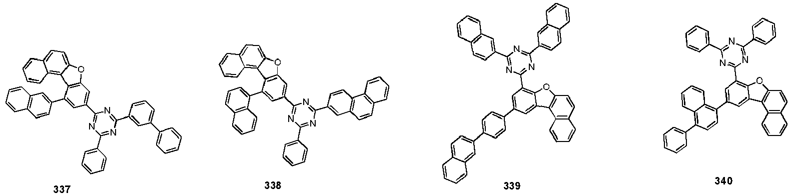

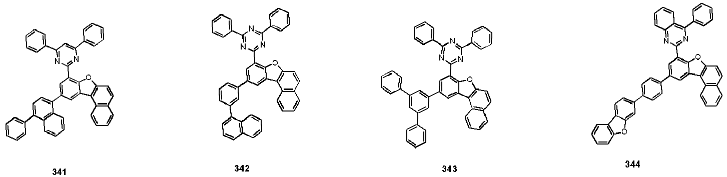

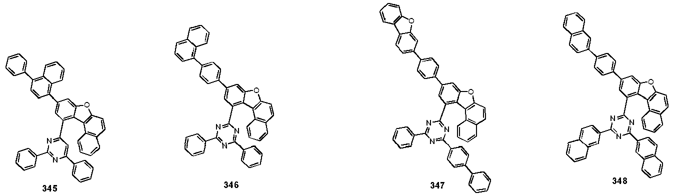

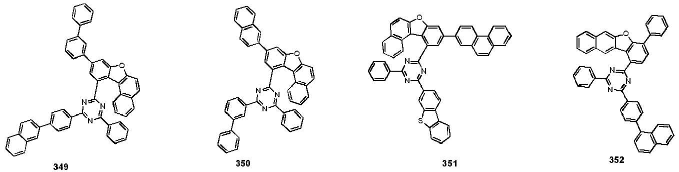

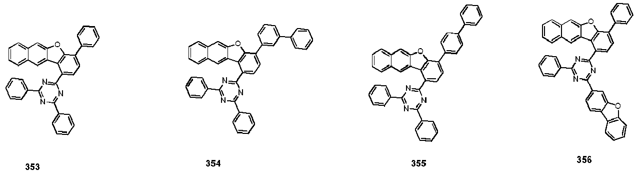

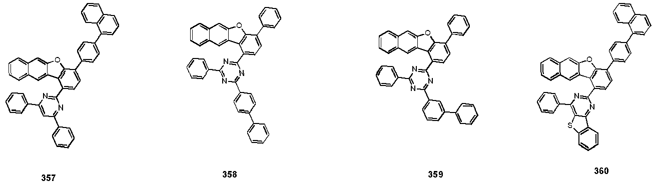

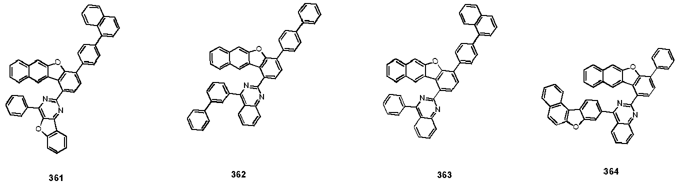

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg) and thereby has superior thermal stability. Such an increase in the thermal stability becomes an important factor in providing driving stability to a device.

The heterocyclic compound according to one embodiment of the present application may be prepared using a multi-step chemical reaction. Some intermediate compounds are prepared first, and from the intermediate compounds, the compound of Chemical Formula 1 may be prepared. More specifically, the heterocyclic compound according to one embodiment of the present application may be prepared based on preparation examples to describe later.

Another embodiment of the present application provides an organic light emitting device comprising the heterocyclic compound represented by Chemical Formula 1. The "organic light emitting device" may be expressed in terms such as an "organic light emitting diode", an "OLED", an "OLED device" and an "organic electroluminescent device".

One embodiment of the present application provides an organic light emitting device comprising a first electrode; a second electrode; and one or more organic material layers provided between the first electrode and the second electrode, wherein one or more layers of the organic material layers comprise the heterocyclic compound represented by Chemical Formula 1.

In one embodiment of the present application, the first electrode may be an anode, and the second electrode may be a cathode.

In another embodiment of the present application, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device.

In another embodiment of the present application, the organic light emitting device may be a green organic light emitting device, and the heterocyclic compound according to Chemical Formula 1 may be used as a material of the green organic light emitting device.

In another embodiment of the present application, the organic light emitting device may be a red organic light emitting device, and the heterocyclic compound according to Chemical Formula 1 may be used as a material of the red organic light emitting device.

Specific descriptions on the heterocyclic compound represented by Chemical Formula 1 are the same as the descriptions provided above.

The organic light emitting device of the present application may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present application may be formed in a single layer structure, but may be formed in a multilayer structure in which two or more organic material layers are laminated. For example, the organic light emitting device of the present disclosure may have a structure comprising a hole injection layer, a hole transfer layer, a hole auxiliary layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present application, the organic material layer comprises a light emitting layer, and the light emitting layer may comprise the heterocyclic compound. Using the heterocyclic compound in the light emitting layer spatially separates HOMO (Highest Occupied Molecular Orbital) and LUMO (Lowest Unoccupied Molecular Orbital) allowing strong charge transfer, and accordingly, superior driving, efficiency and lifetime may be obtained in the organic light emitting device.

The organic light emitting device of the present disclosure may further comprise one, two or more layers selected from the group consisting of a light emitting layer, a hole injection layer, a hole transfer layer, an electron injection layer, an electron transfer layer, a hole auxiliary layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

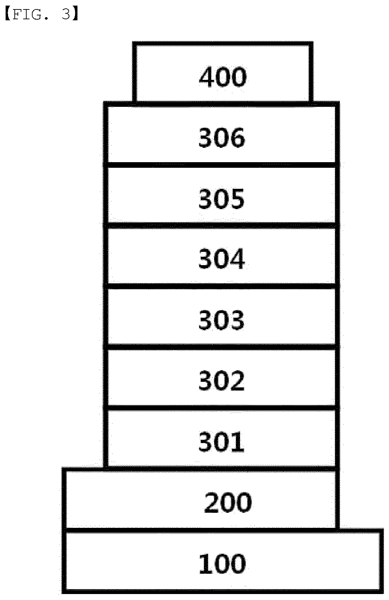

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be comprised, and other necessary functional layers may be further added.

The organic material layer comprising the compound represented by Chemical Formula 1 may further comprise other materials as necessary.

In the organic light emitting device according to one embodiment of the present application, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

In the organic light emitting device of the present application, the organic material layer comprises a light emitting layer, and the light emitting layer may comprise the heterocyclic compound as a host material of a light emitting material.

In the organic light emitting device of the present application, the light emitting layer may comprise two or more host materials, and at least one of the host materials may comprise the heterocyclic compound as a host material of a light emitting material.

In the organic light emitting device of the present application, the light emitting layer may use two or more host materials after pre-mixing, and at least one of the two or more host materials may comprise the heterocyclic compound as a host material of a light emitting material.

The pre-mixing means mixing the two or more host materials of the light emitting layer in advance in one source of supply before depositing on the organic material layer.

In the organic light emitting device of the present application, the light emitting layer may comprise two or more host materials, the two or more host materials each comprise one or more p-type host materials and n-type host materials, and at least one of the host materials may comprise the heterocyclic compound as a host material of a light emitting material. In this case, the organic light emitting device may have superior driving, efficiency and lifetime.

In the organic light emitting device of the present application, the light emitting layer may comprise two or more types of the heterocyclic compound represented by Chemical Formula 1.

In the organic light emitting device of the present application, the light emitting layer may comprise two types of the heterocyclic compound represented by Chemical Formula 1.

When using two or more types of the heterocyclic compound represented by Chemical Formula 1 in the light emitting layer, device performance may be enhanced by forming an exciplex.

In one embodiment of the present application, two or more types of the heterocyclic compound represented by Chemical Formula 1 may be used as a host material at the same time.

One embodiment of the present application provides a composition for an organic material layer of an organic light emitting device, the composition comprising two or more types of the heterocyclic compound represented by Chemical Formula 1.

One embodiment of the present application provides a composition for an organic material layer of an organic light emitting device, the composition comprising two types of the heterocyclic compound represented by Chemical Formula 1.

In the composition, the two types of the heterocyclic compound represented by Chemical Formula 1 may have a weight ratio of 1:10 to 10:1, 1:8 to 8:1, 1:5 to 5:1 or 1:2 to 2:1, however, the weight ratio is not limited thereto.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### [Preparation Example 1] Preparation of Compound 1

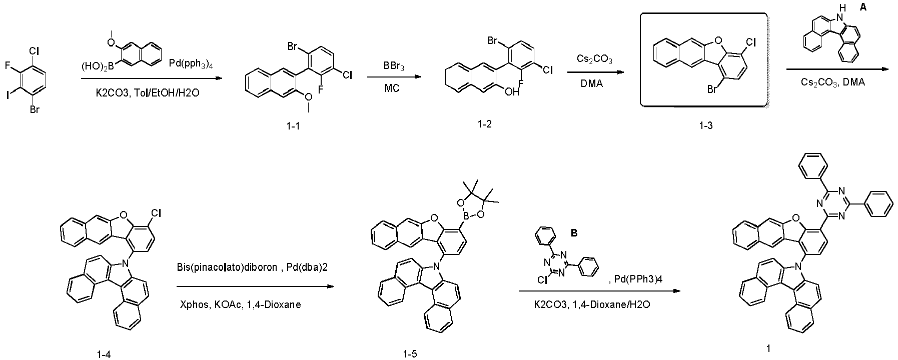

### 1) Preparation of Compound 1-1

In a one neck round bottom flask (one neck r.b.f), a mixture of 1-bromo-4-chloro-3-fluoro-2-iodobenzene (50 g, 150 mmol), (3-methoxynaphthalen-2-yl)boronic acid (30 g, 150 mmol), tetrakis(triphenylphosphine)palladium(0) (17 g, 15 mmol), potassium carbonate (62 g, 450 mmol) and toluene/ethanol/water (500 ml/100 ml/100 ml) was stirred under reflux at 110°C. After that, the result was extracted with dichloromethane, dried with MgSO₄, and then purified by silica gel column chromatography to obtain Compound 1-1 (48 g, 88%).

### 2) Preparation of Compound 1-2

In a one neck round bottom flask (one neck r.b.f), a mixture of Compound 1-1 (48 g, 132 mmol) and dichloromethane (1000 ml) was cooled to 0°C. After adding BBr₃ (26 mL, 270 mmol) dropwise thereto, the temperature was raised to room temperature, and the result was stirred for 2 hours. After that, the reaction was terminated using distilled water, and the result was extracted with dichloromethane, dried with MgSO₄, and purified by silica gel column chromatography to obtain Compound 1-2 (37 g, 80%).

### 3) Preparation of Compound 1-3

In a one neck round bottom flask (one neck r.b.f), a dimethylacetamide (400 ml) mixture of Compound 1-2 (37 g, 106 mmol) and Cs₂CO₃ (172 g, 530 mmol) was stirred at 120°C. After that, the mixture was cooled to room temperature, then filtered, and, after removing the solvent of the filtrate, purified by silica gel column chromatography to obtain Compound 1-3 (32 g, 88%) .

### 4) Preparation of Compound 1-4

In a one neck round bottom flask (one neck r.b.f), a dimethylacetamide (100 ml) mixture of Compound 1-3 (10 g, 30 mmol), 7H-dibenzo[c,g]carbazole (8 g, 30 mmol) and Cs₂CO₃ (48 g, 150 mmol) was stirred for 12 hours under reflux at 170°C. After that, the mixture was cooled to room temperature, then filtered, and, after removing the solvent of the filtrate, purified by silica gel column chromatography to obtain Compound 1-4 (11.3 g, 73%) .

### 5) Preparation of Compound 1-5

In a one neck round bottom flask (one neck r.b.f), a 1,4-dioxane (100 ml) mixture of Compound 1-4 (11.8 g, 22 mmol), bis(pinacolato)diboron (12 g, 44 mmol), XPhos (2.0 g, 4.4 mmol), potassium acetate (6.4 g, 66 mmol) and Pd(dba)₂ (1.2 g, 2.2 mmol) was stirred under reflux at 140°C. After that, the result was extracted with dichloromethane and then concentrated, and produced solids were purified by recrystallization using dichloromethane/MeOH to obtain Compound 1-5 (12.8 g, 96%).

### 6) Preparation of Compound 1

In a one neck round bottom flask (one neck r.b.f), a mixture of Compound 1-5 (12.8 g, 21.1 mmol), 2-chloro-4,6-diphenyl-1,3,5-triazine (5.8 g, 22 mmol), tetrakis(triphenylphosphine)palladium(0) (2.4 g, 2.11 mmol), potassium carbonate (8.7 g, 63.3 mmol) and 1,4-dioxane/water (120 ml/24 ml) was stirred for 3 hours under reflux at 120°C. Solids precipitated during the reaction were filtered while hot, and purified using 1,4-dioxane, distilled water and MeOH to obtain Compound 1 (10.5 g, 70%).

Target compounds of the following Table 1 were synthesized in the same manner as in Preparation of Compound 1 of Preparation Example 1 except that A of the following Table 1 was used instead of 7H-dibenzo[c,g]carbazole, and B of the following Table 1 was used instead of 2-chloro-4,6-diphenyl-1,3,5-triazine.

**[Table 1]**

| Compo und | A | B | Target Compound | Yield (%) |
|---|---|---|---|---|
| 14 | 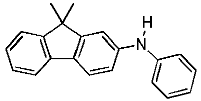 | 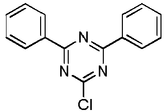 | 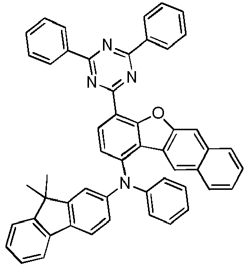 | 67 |

In Preparation of Compound 1-3 of Preparation Example 1, positions of -Br and -Cl in Compound 1-3 differ depending on -Br and -Cl of the 1-bromo-4-chloro-3-fluoro-2-iodobenzene, and intermediates to be synthesized accordingly are as follows.

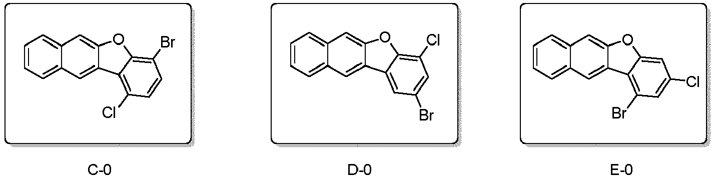

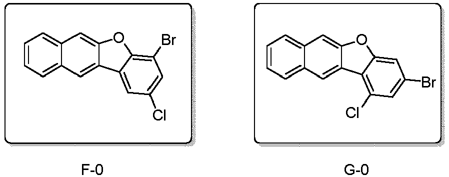

Target compounds of the following Table 2 were synthesized in the same manner as in Preparation of Compound 1 of Preparation Example 1 except that one of Intermediates C-0, D-0, E-0, F-0 and G-0 was used as described in the following Table 2 instead of Compound 1-3, and A and B of the following Table 2 were used.

**[Table 2]**

| Compou nd | Inter media te | A | B | Target Compound | Yiel d (%) |
|---|---|---|---|---|---|
| 21 | F-0 | 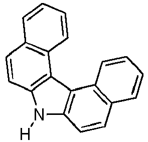 | 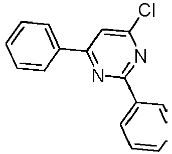 | 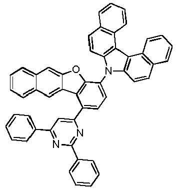 | 71 |
| 22 | C-0 | 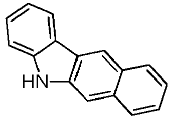 | 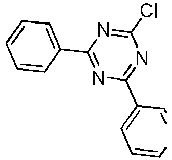 | 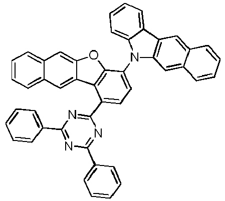 | 78 |
| 34 | D-0 | 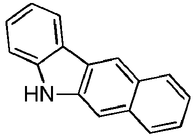 | 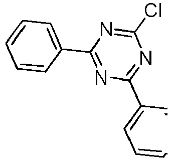 | 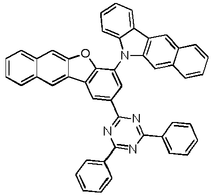 | 82 |
| 41 | E-0 | 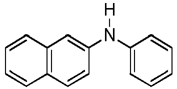 | 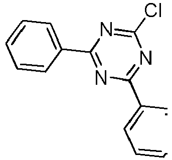 | 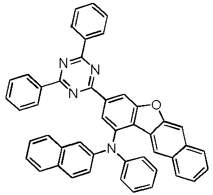 | 75 |
| 67 | G-0 | 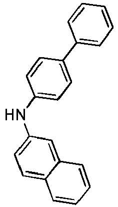 | 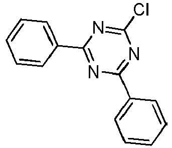 | 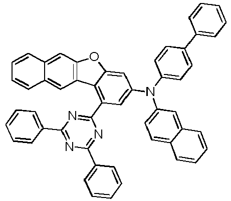 | 62 |

### [Preparation Example 2] Preparation of Compound 9

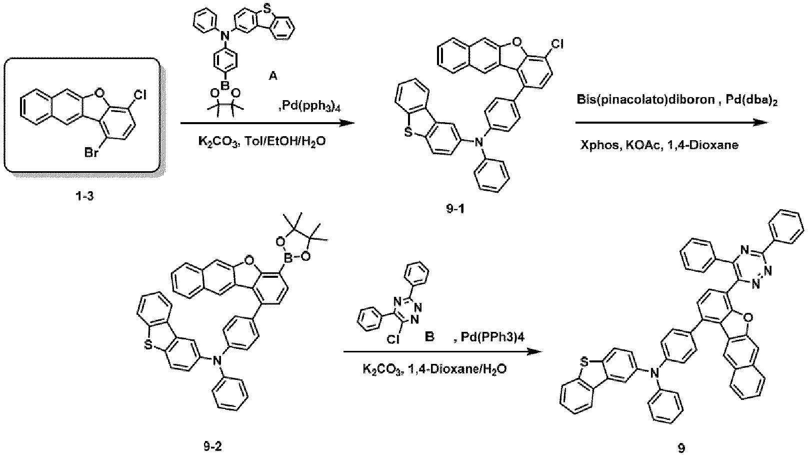

### 1) Preparation of Compound 9-1

In a one neck round bottom flask (one neck r.b.f), a mixture of Compound 1-3 (30 g, 90 mmol), N-phenyl-N-(4-(4,4,5,5-tetramethyl-1,3,2-dioxaborolan-2-yl)phenyl)dibenzo[b,d]thiophen-2-amine (42 g, 90 mmol), tetrakis(triphenylphosphine)palladium(0) (10 g, 9 mmol), potassium carbonate (37 g, 270 mmol) and toluene/ethanol/water (300 ml/60 ml/60 ml) was stirred under reflux at 110°C. After that, the result was extracted with dichloromethane, dried with MgSO₄, and then purified by silica gel column chromatography to obtain Compound 9-1 (66 g, 92%).

### 2) Preparation of Compound 9-2

In a one neck round bottom flask (one neck r.b.f), a 1,4-dioxane (100 ml) mixture of Compound 9-1 (10 g, 16 mmol), bis(pinacolato)diboron (8 g, 32 mmol), XPhos (1.5 g, 3.2 mmol), potassium acetate (4.7 g, 48 mmol) and Pd(dba)₂ (0.9 g, 1.6 mmol) was stirred under reflux at 140°C. After that, the result was extracted with dichloromethane and then concentrated, and produced solids were purified by recrystallization using dichloromethane/MeOH to obtain Compound 9-2 (8.8 g, 88%).

### 3) Preparation of Compound 9

In a one neck round bottom flask (one neck r.b.f), a mixture of Compound 9-2 (8.8 g, 14 mmol), 6-chloro-3,5-diphenyl-1,2,4-triazine (3.7 g, 14 mmol), tetrakis(triphenylphosphine)palladium(0) (1.6 g, 1.4 mmol), potassium carbonate (5.7 g, 42 mmol) and 1,4-dioxane/water (100 ml/20 ml) was stirred for 3 hours under reflux at 120°C. After that, solids precipitated during the reaction were filtered while hot, and purified using 1,4-dioxane, distilled water and MeOH to obtain Compound 9 (9.0 g, 81%).

Target compounds of the following Table 3 were synthesized in the same manner as in Preparation of Compound 9 of Preparation Example 2 except that A of the following Table 3 was used instead of N-phenyl-N-(4-(4,4,5,5-tetramethyl-1,3,2-dioxaborolan-2-yl)phenyl)dibenzo[b,d]thiophen-2-amine, and B of the following Table 3 was used instead of 6-chloro-3,5-diphenyl-1,2,4-triazine.

**[Table 3]**

| Compound | A | B | Target Compound | Yield (%) |
|---|---|---|---|---|
| 13 | 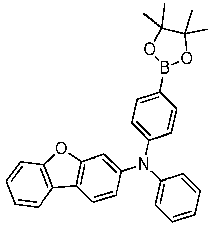 | 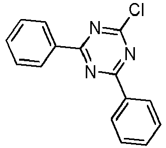 | 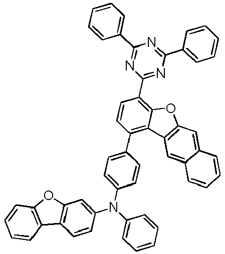 | 78 |

Similarly, the following intermediates may be used instead of Compound 1-3 in Preparation Example 2.

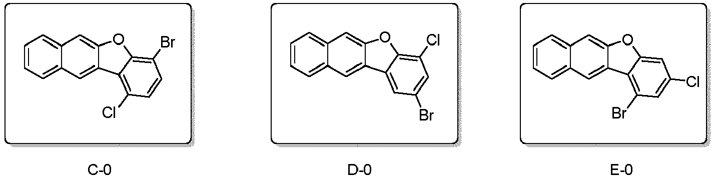

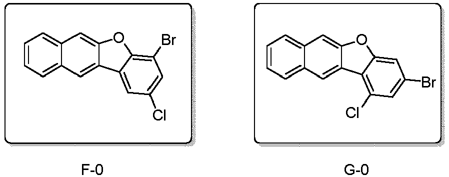

Target compounds of the following Table 4 were synthesized in the same manner as in Preparation of Compound 9 of Preparation Example 2 except that one of Intermediates C-0, D-0, E-0, F-0 and G-0 was used as described in the following Table 4 instead of Compound 1-3, and A and B of the following Table 4 were used.

**[Table 4]**

| Com pou nd | Inter media te | A | B | Target Compound | Yie ld (%) |
|---|---|---|---|---|---|
| 23 | C-0 | 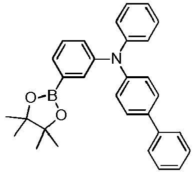 | 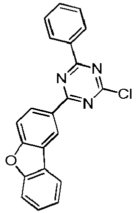 | 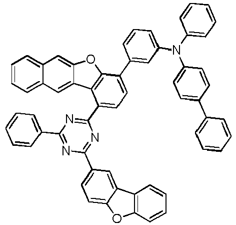 | 81 |
| 30 | F-0 | 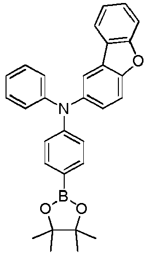 | 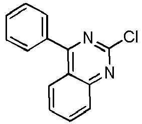 | 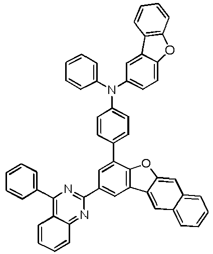 | 86 |
| 31 | F-0 | 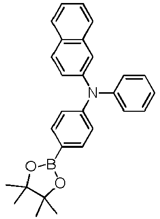 | 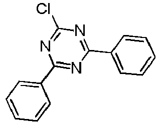 | 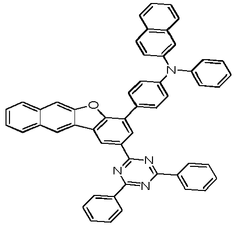 | 78 |
| 44 | E-0 | 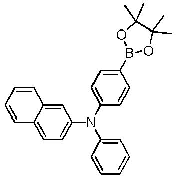 | 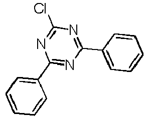 | 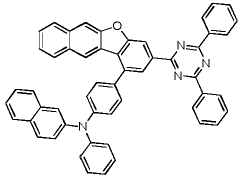 | 74 |
| 57 | D-0 | 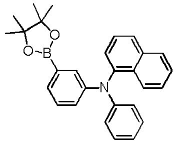 | 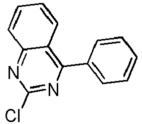 | 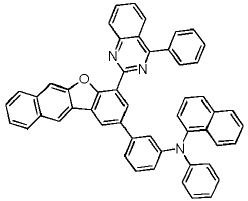 | 68 |
| 64 | G-0 | 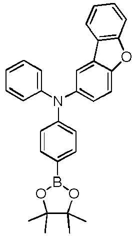 | 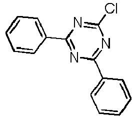 | 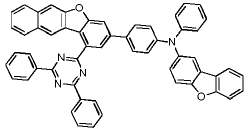 | 73 |
| 243 | C-0 | 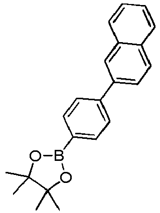 | 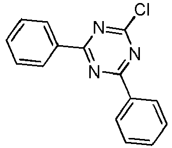 | 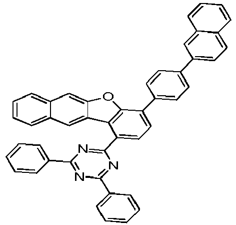 | 65 |
| 271 | G-0 | 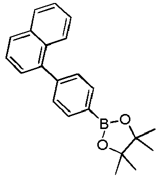 | 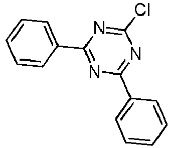 | 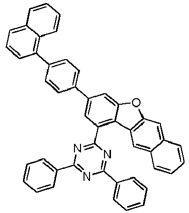 | 68 |
| 353 | C-0 | 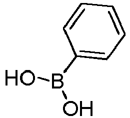 | 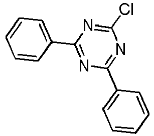 | 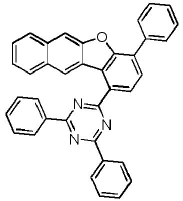 | 75 |
| 356 | C-0 | 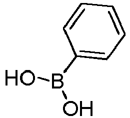 | 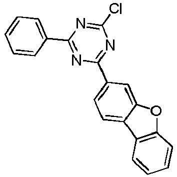 | 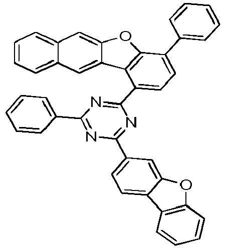 | 65 |
| 354 | C-0 | 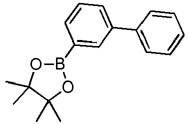 | 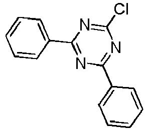 | 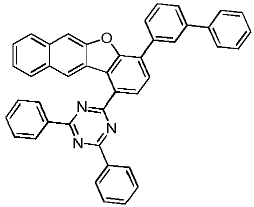 | 78 |
| 360 | C-0 | 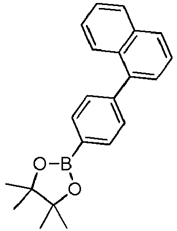 | 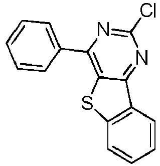 | 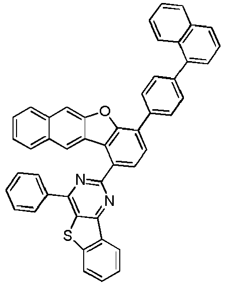 | 81 |
| 363 | C-0 | 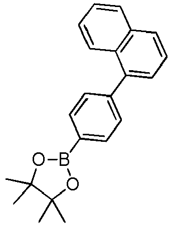 | 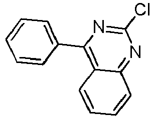 | 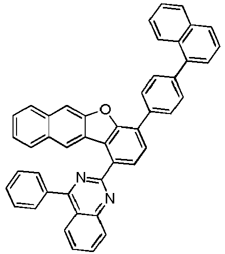 | 77 |

### [Preparation Example 3] Preparation of Compound 77

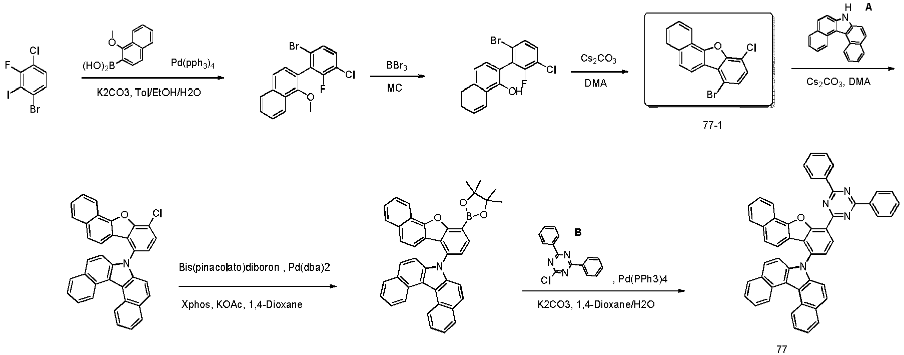

### 1) Preparation of Compound 77-1

Compound 77-1 was prepared in the same manner as in Preparation of Compound 1-3 of Preparation Example 1 except that (1-methoxynaphthalen-2-yl)boronic acid was used instead of (3-methoxynaphthalen-2-yl)boronic acid.

### 2) Preparation of Compound 77

Compound 77 (8.3 g, 72%) was prepared in the same manner as in Preparation of Compound 1 of Preparation Example 1 except that Compound 77-1 was used instead of Compound 1-3.

Target compounds of the following Table 5 were synthesized in the same manner as in Preparation of Compound 77 of Preparation Example 3 except that A of the following Table 5 was used instead of 7H-dibenzo[c,g]carbazole, and B of the following Table 5 was used instead of 2-chloro-4,6-diphenyl-1,3,5-triazine.

**[Table 5]**

| Compound | A | B | Target Compound | Yield (%) |
|---|---|---|---|---|
| 78 | 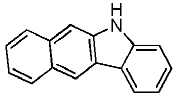 | 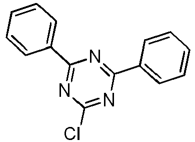 | 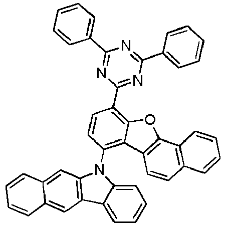 | 91 |

In Preparation Example 3, positions of -Br and -Cl in Compound 1-3 differ depending on -Br and -Cl of the 1-bromo-4-chloro-3-fluoro-2-iodobenzene, and intermediates to be synthesized accordingly are as follows.

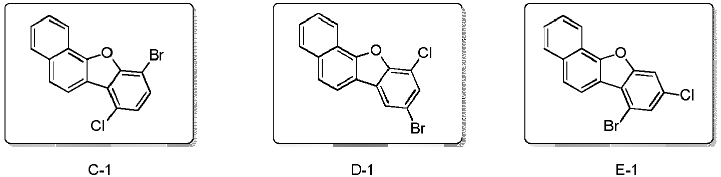

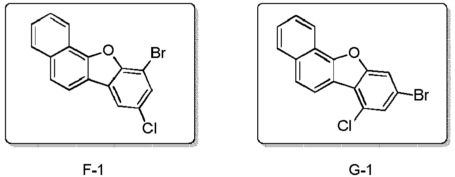

Target compounds of the following Table 6 were synthesized in the same manner as in Preparation of Compound 77 of Preparation Example 3 except that one of Intermediates C-1, D-1, E-1, F-1 and G-1 was used as described in the following Table 6 instead of Compound 77-1, and A and B of the following Table 6 were used.

**[Table 6]**

| Compo und | Interme diate | A | B | Target Compound | Yield (%) |
|---|---|---|---|---|---|
| 103 | C-1 | 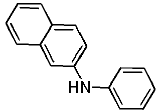 | 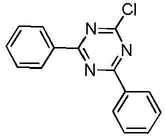 | 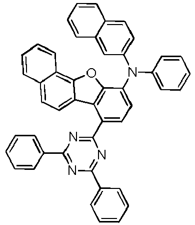 | 83 |
| 107 | F-1 | 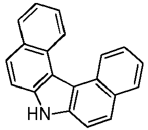 | 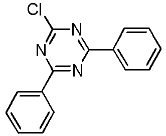 | 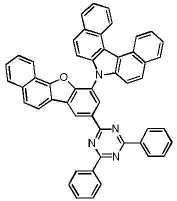 | 80 |
| 117 | G-1 | 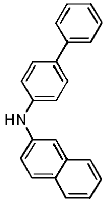 | 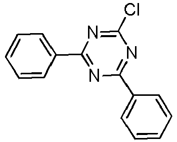 | 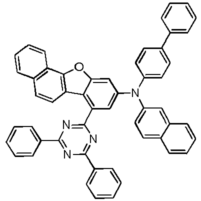 | 79 |
| 122 | E-1 | 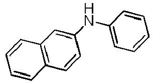 | 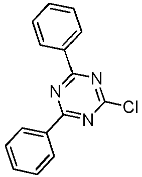 | 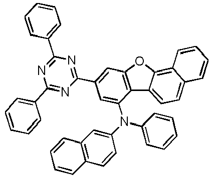 | 68 |
| 130 | D-1 | 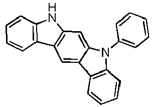 | 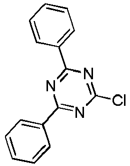 | 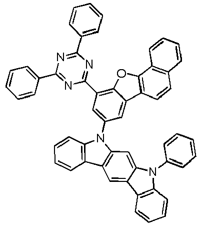 | 74 |
| 153 | G-1 | 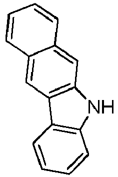 | 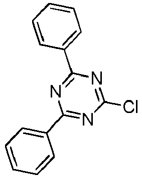 | 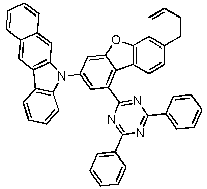 | 67 |

### [Preparation Example 4] Preparation of Compound 90

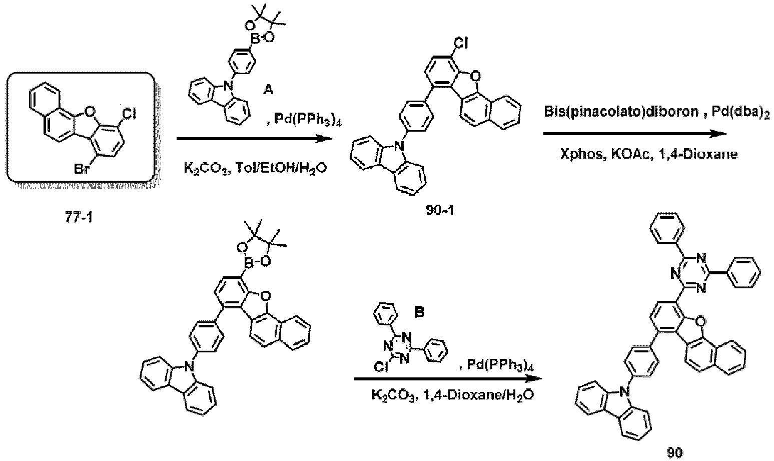

### 1) Preparation of Compound 90-1

Compound 90-1 was obtained in the same manner as in Preparation Example 2 except that Compound 77-1 of Preparation Example 3 was used instead of Compound 9-1, and 9-(4-(4,4,5,5-tetramethyl-1,3,2-dioxaborolan-2-yl)phenyl)-9H-carbazole was used instead of N-phenyl-N-(4-(4,4,5,5-tetramethyl-1,3,2-dioxaborolan-2-yl)phenyl)dibenzo[b,d]thiophen-2-amine.

### 2) Preparation of Compound 90

Compound 90 (13.2 g, 72%) was obtained in the same manner as in Preparation Example 2 except that Compound 90-1 was used instead of Compound 9-1.

The following target compounds were synthesized in the same manner as in Preparation of Compound 90 of Preparation Example 4 except that A and B of the following Table 7 were used.

**[Table 7]**

| Compound | A | B | Target Compound | Yield (%) |
|---|---|---|---|---|
| 85 | 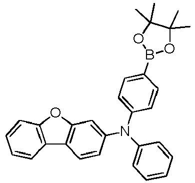 | 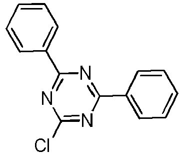 | 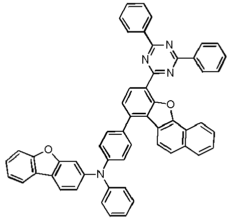 | 71 |

Similarly, the following intermediates may be used instead of Compound 77-1 in Preparation Example 4.

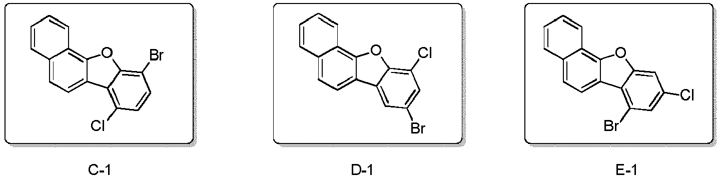

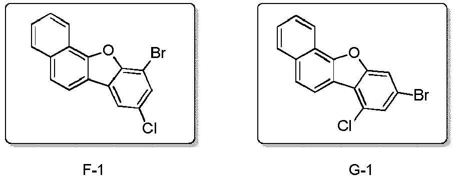

Target compounds of the following Table 8 were synthesized in the same manner as in Preparation of Compound 90 of Preparation Example 4 except that one of Intermediates C-1, D-1, E-1, F-1 and G-1 was used as described in the following Table 8 instead of Compound 77-1, and A and B of the following Table 8 were used.

**[Table 8]**

| Compo und | Inter media te | A | B | Target Compound | Yield (%) |
|---|---|---|---|---|---|
| 95 | C-1 | 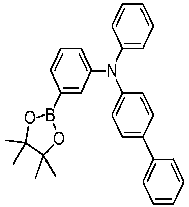 | 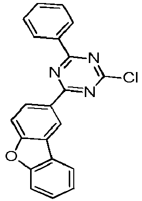 | 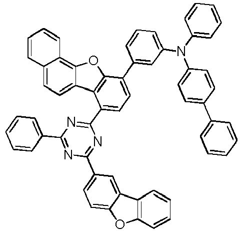 | 88 |
| 119 | F-1 | 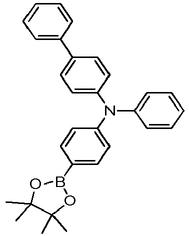 | 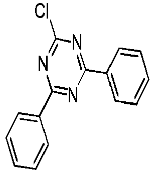 | 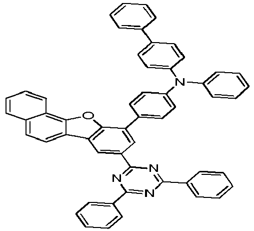 | 85 |
| 123 | E-1 | 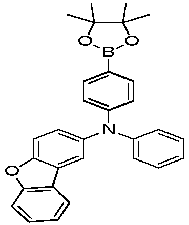 | 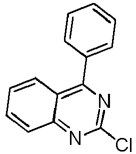 | 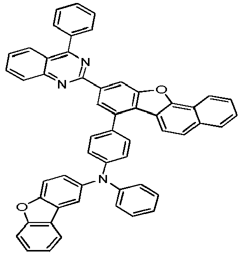 | 72 |
| 143 | D-1 | 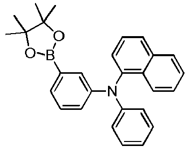 | 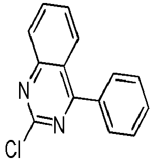 | 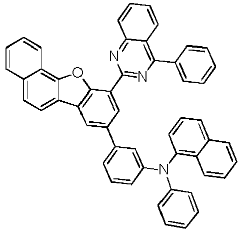 | 86 |
| 146 | G-1 | 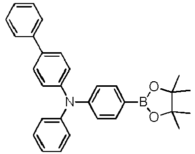 | 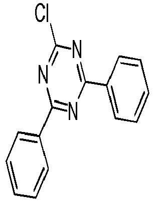 | 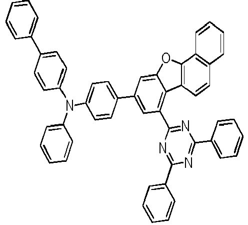 | 79 |
| 285 | C-1 | 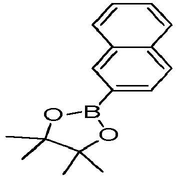 | 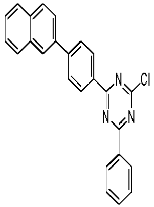 | 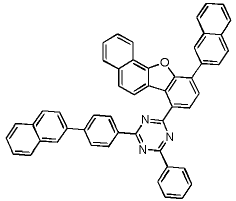 | 75 |
| 295 | E-1 | 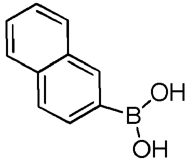 | 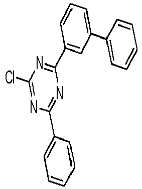 | 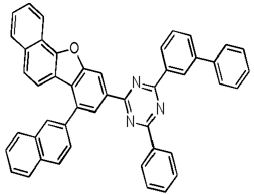 | 81 |

### [Preparation Example 5] Preparation of Compound 157

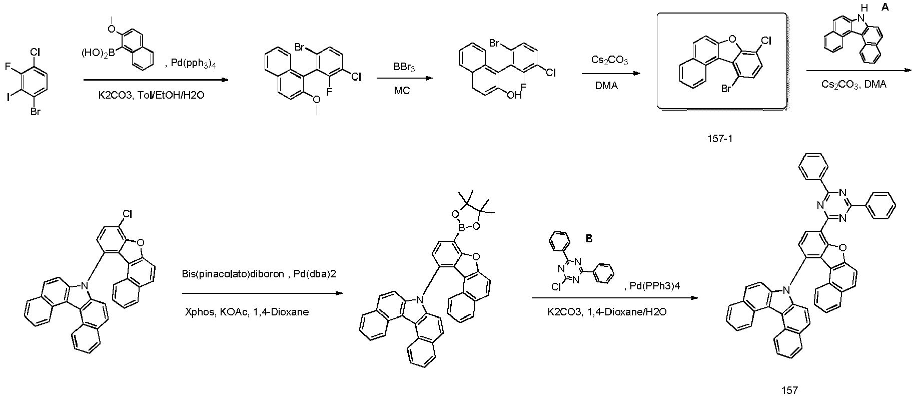

### 1) Preparation of Compound 157-1

Compound 157-1 was prepared in the same manner as in Preparation of Compound 1-3 of Preparation Example 1 except that (2-methoxynaphthalen-1-yl)boronic acid was used instead of (3-methoxynaphthalen-2-yl)boronic acid.

### 2) Preparation of Compound 157

Compound 157 (9.5 g, 84%) was prepared in the same manner as in Preparation of Compound 1 of Preparation Example 1 except that Compound 157-1 was used instead of Compound 1-3.

Target compounds of the following Table 9 were synthesized in the same manner as in Preparation of Compound 157 of Preparation Example 5 except that A of the following Table 9 was used instead of 7H-dibenzo[c,g]carbazole, and B of the following Table 9 was used instead of 2-chloro-4,6-diphenyl-1,3,5-triazine.

**[Table 9]**

| Compoun d | A | B | Target Compound | Yield (%) |
|---|---|---|---|---|
| 161 | 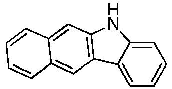 | 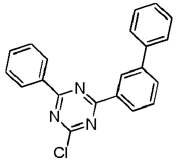 | 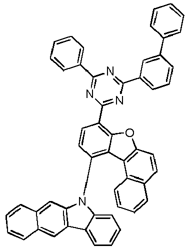 | 67 |

In Preparation Example 5, positions of -Br and -Cl in Compound 157-1 differ depending on -Br and -Cl of the 1-bromo-4-chloro-3-fluoro-2-iodobenzene, and intermediates to be synthesized accordingly are as follows.

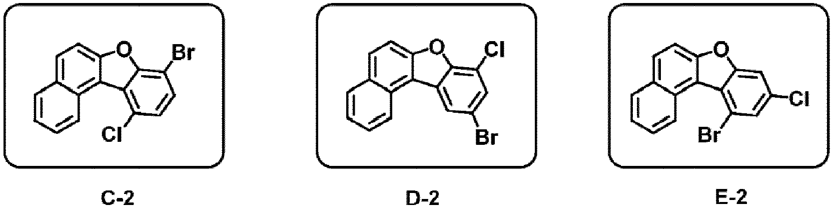

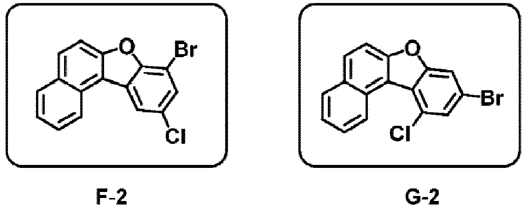

Target compounds of the following Table 10 were synthesized in the same manner as in Preparation of Compound 157 of Preparation Example 5 except that one of Intermediates C-2, D-2, E-2, F-2 and G-2 was used as described in the following Table 10 instead of Compound 157-1, and A and B of the following Table 10 were used.

**[Table 10]**

| Comp ound | Inter media te | A | B | Target Compound | Yiel d (%) |
|---|---|---|---|---|---|
| 172 | C-2 | 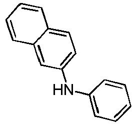 | 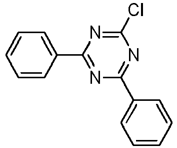 | 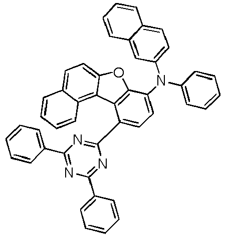 | 68 |
| 177 | C-2 | 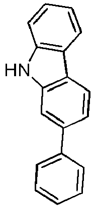 | 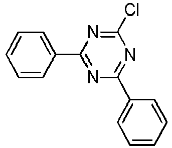 | 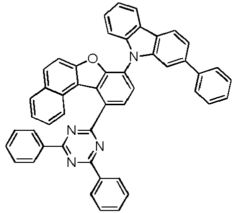 | 64 |
| 188 | F-2 | 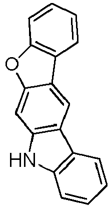 | 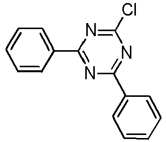 | 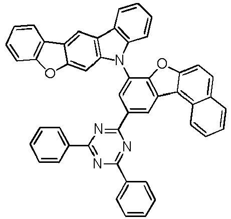 | 73 |
| 204 | E-2 | 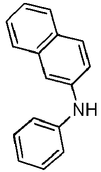 | 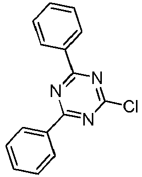 | 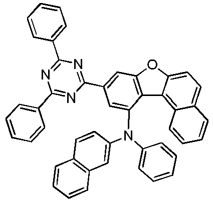 | 83 |
| 221 | G-2 | 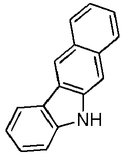 | 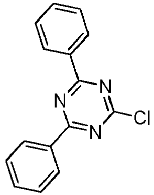 | 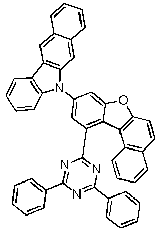 | 72 |
| 227 | D-2 | 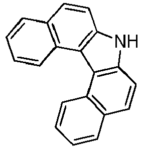 | 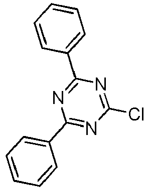 | 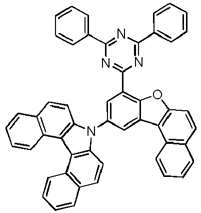 | 88 |

### [Preparation Example 6] Preparation of Compound 165

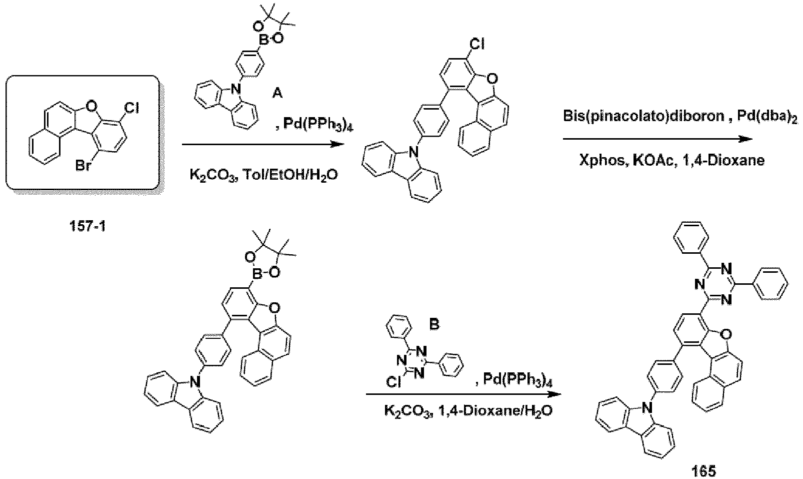

### 1) Preparation of Compound 165

Compound 165 (5.8 g, 68%) was obtained in the same manner as in Preparation Example 4 except that Compound 157-1 was used instead of Compound 77-1.

Target compounds of the following Table 11 were synthesized in the same manner as in Preparation of Compound 165 of Preparation Example 6 except that A and B of the following Table 11 were used.

**[Table 11]**

| Compoun d | A | B | Target Compound | Yield (%) |
|---|---|---|---|---|
| 318 | 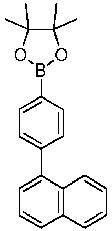 | 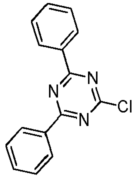 | 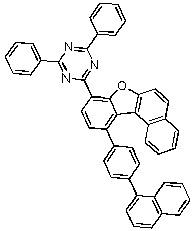 | 67 |

Similarly, the following intermediates may be used instead of Compound 157-1 in Preparation Example 6.

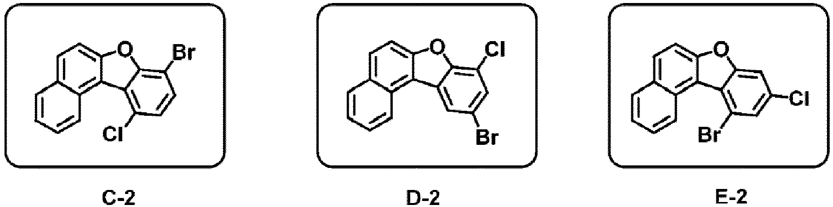

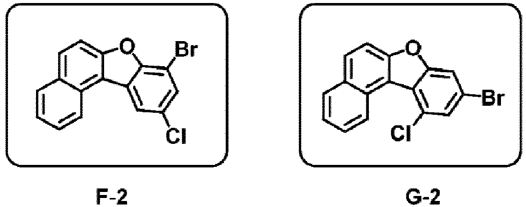

**[Table 12]**

| Compound | Intermediate | A | B | Target Compound | Yield (%) |
|---|---|---|---|---|---|
| 174 | C-2 | 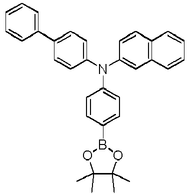 | 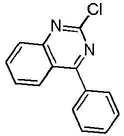 | 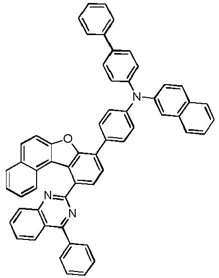 | 72 |
| 191 | F-2 | 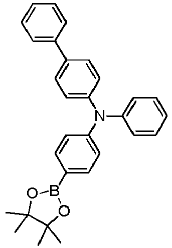 | 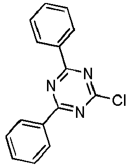 | 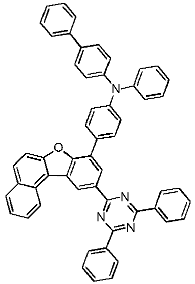 | 67 |
| 203 | E-2 | 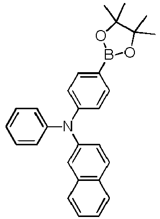 | 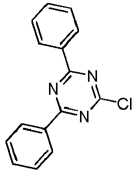 | 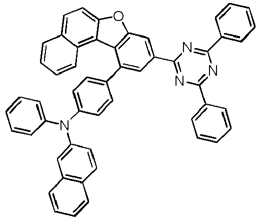 | 87 |
| 212 | D-2 | 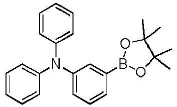 | 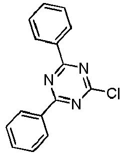 | 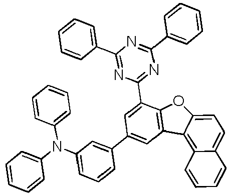 | 73 |
| 224 | G-1 | 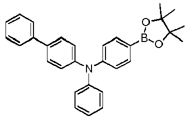 | 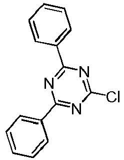 | 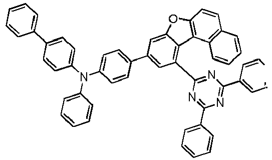 | 89 |
| 343 | D-2 | 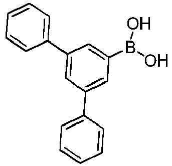 | 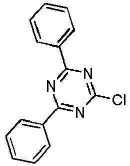 | 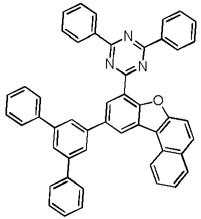 | 81 |

Compounds were prepared in the same manner as in the preparation examples, and the synthesis identification results are shown in the following Table 13 and Table 14. The following Table 13 shows measurement values of ¹H NMR (CDCl₃, 300 Mz), and the following Table 14 shows measurement values of FD-mass spectrometry (FD-MS: field desorption mass spectrometry).

**[Table 13]**

| **NO** | **¹H NMR (CDCl₃, 300 Mz)** |
|---|---|
| 1 | 8.54(m, 2H), 8.28(m, 4H), 8.16(m, 4H), 7.96∼7.94(m, 2H), 7.81(d, 1H), 7.67~7.65(m, 8H), 7.51∼7.41(m, 8H) |
| 9 | 8.45(d, 1H), 8.28(m, 2H), 8.16(m, 2H), 7.98(m, 1H), 7.87~7.81(m, 2H), 7.73∼7.67(m, 3H), 7.52~7.41(m, 15H), 7.20(m, 2H), 6.86~6.81(m, 2H), 6.69~6.63(m, 4H) |
| 14 | 8.28(m, 3H), 8.16(d, 2H), 7.87(m, 1H) 7.67~7.41(m, 14H), 7.28~7.20(m, 3H), 6.81∼6.75(m, 2H), 6.63(m, 3H), 6.39(d, 1H) |
| 21 | ,8.54(m, 2H), 8.23~8.16(m, 7H), 7.96~7.94(m, 2H), 7.75~7.41(m, 20H) |
| 22 | 8.55(d, 1H), 8.28(m, 4H), 8.16(m, 4H), 7.94(m, 1H). 7.75~7.67(m, 5H), 7.55∼7.25(m, 15H) |
| 23 | 8.28(d, 2H), 8.16(m, 2H), 7.89~7.81(m, 4H), 7.72~7.67(m, 5H), 7.52∼7.32(m, 15H), 7.20(m, 2H), 6.89~6.81(m, 3H), 6.69~6.59(m, 5H) |
| 30 | 8.16(m, 3H), 7.89~7.79(m, 5H), 7.66~7.38(m, 17H), 7.20(m, 2H), 6.81(t, 1H), 6.69~6.63(m, 4H), 6.39(d, 1H) |
| 31 | 8.28(d, 4H), 8.16(t, 2H), 7.67(m, 8H), 7.51~7.36(m, 13H), 7.20(m, 2H), 6.81(t, 1H), 6.69~6.63(m, 4H) |
| 34 | 8.55(d, 1H), 8.28(d, 4H), 8.16(m, 4H), 8.02(s, 1H), 7.94(d, 1H), 7.72~7.67(m, 5H), 7.51∼7.25(m, 12H) |
| 41 | 8.28(d, 4H), 8.16(t, 2H), 7.88~7.67(m, 6H), 7.51~7.36(m, 11H), 7.20(t, 2H), 7.00(s, 1H), 6.81(m, 1H), 6.63(m, 2H) |
| 44 | 8.28(d, 4H), 8.16(m, 2H), 7.88~7.36(m, 21H), 7.20(t, 2H), 6.81(m, 1H), 6.69~6.63(m, 4H) |
| 57 | 8.16~8.02(m, 5H), 7.83~7.79(m, 4H), 7.58~7.41(m, 15H), 7.20(t, 2H), 6.98(d, 1H), 6.89~6.81(m, 3H), 6.63~6.59(m, 3H) |
| 64 | 8.28(m, 3H), 8.16(t, 2H), 7.89(d, 1H), 7.67~7.32(m, 19H), 7.20(m, 2H), 6.81(t, 1H), 6.69~6.63(m, 4H), 6.39(d, 1H) |
| 67 | 8.28(d, 3H), 8.16(m, 2H), 7.84~7.74(m, 4H), 7.67(m, 2H), 7.52~7.36(m, 20H), 6.69(d, 2H) |
| 85 | 8.28(m, 4H), 8.16(m, 2H), 7.89~7.81(m, 4H), 7.67∼7.64(m, 4H), 7.51∼7.38(m, 12H), 7.20(m, 2H), 6.81(m, 1H), 6.69~6.63(m, 4H), 6.33(d, 1H) |
| 90 | 8.55(d, 1H), 8.28(m, 4H), 8.16~8.12(m, 3H), 7.94~7.79(m, 6H), 7.68~7.67(m, 5H), 7.51∼7.25(m, 11H) |
| 95 | 8.28(d, 2H), 8.16(d, 2H), 7.89~7.81(m, 5H), 7.72~7.67(m, 5H), 7.53∼7.41(m, 14H), 7.20(t, 2H), 6.88~6.81(m, 3H), 6.63~6.59(m, 5H) |
| 103 | 8.28(d, 4H), 8.16(d, 2H), 7.88~7.67(m, 7H), 7.50~7.36(m, 13H), 7.20(t, 2H), 6.81(t, 1H), 6.63(d, 2H), 6.45(d, 1H) |
| 107 | 8.54(m, 2H), 8.28(d, 4H), 8.16(m, 4H), 8.02~7.94(m, 4H), 7.72~7.63(m, 9H), 7.53~7.41(m, 7H) |
| 117 | 8.28(d, 4H), 8.16(m, 2H), 7.89~7.84(m, 3H), 7.77~7.74(m, 2H), 7.67(m, 2H), 7.54∼7.41(m, 19H), 6.69(d, 2H) |
| 119 | 8.28(d, 4H), 8.16(m, 2H), 7.89(d, 1H), 7.67(m, 4H), 7.54~7.41(m, 6H), 7.20(t, 2H), 6.81(t, 1H), 6.69~6.63(m, 6H) |
| 122 | 8.28(d, 4H), 8.16(t, 2H), 7.88~7.67(m, 7H), 7.53~7.36(m, 12H), 7.00(s, 1H), 6.81(t, 1H), 6.63(m, 2H) |
| 123 | 8.16(m, 3H), 7.89~7.79(m, 6H), 7.67~7.32(m, 16H), 7.20(m, 2H), 6.81(t, 1H), 6.69~6.63(m, 4H), 6.39(m, 1H) |
| 128 | 8.54(d, 2H), 8.28(d, 2H), 8.16(m, 4H), 7.96~7.85(m, 5H), 7.67~7.72(m, 10H), 7.52∼7.41(m, 9H), 7.25(d, 2H) |
| 130 | 8.55(d, 1H), 8.28(m, 4H), 8.16~8.12(m, 3H), 7.94~8.89(m, 2H), 7.76~7.41(m, 23H) |
| 146 | 8.28(d, 4H), 8.16(m, 2H), 7.89(d, 1H), 7.67(m, 2H), 7.54~7.41(m, 18H), 7.20(m, 2H), 6.81(t, 1H), 6.69~6.63(m, 6H) |
| 153 | 8.55(d, 1H), 8.28(d, 4H), 8.16(m, 4H), 7.94~7.89(m, 2H), 7.69~7.66(m, 6H), 7.55∼7.33(m, 11H) |
| 157 | 8.54(m, 3H), 8.28(d, 4H), 8.16(m, 3H), 7.96~7.94(m, 2H), 7.81(d, 1H), 7.67~7.41(m, 17H) |
| 161 | 8.54(m, 2H), 8.28~8.16(m, 6H), 7.67(d, 1H), 7.81(d, 1H), 7.67~7.41(m, 22H) |
| 165 | 8.55∼7.79(m, 2H), 8.28(d, 4H), 8.16~8.12(m, 2H), 7.94~7.81(m, 4H), 7.66~7.25(m, 17H) |
| 174 | 8.34(m, 1H), 8.16(m, 2H), 7.88~7.36(m, 30H), 6.69(d, 4H) |
| 177 | 8.55-8.54(m, 2H), 8.28(m, 4H), 8.18~8.16(m, 2H), 7.94(d, 1H), 7.79~7.25(m, 21H) |
| 188 | 8.55∼8.54(m, 2H), 8.28(d, 4H), 8.16(m, 1H), 8.02(s, 1H), 7.94~7.89(m, 2H), 7.66∼7.25(m, 18H) |
| 191 | 8.54(m, 8.54), 8.28(m, 4H), 8.16(t, 1H), 7.54~7.41(m, 21H), 7.20(t, 2H), 6.81(m, 1H), 6.69~6.63(m, 6H) |
| 203 | 8.54(d, 1H), 8.28(d, 4H), 8.16(m, 1H), 7.88~7.36(m, 11H), 7.20(m, 2H), 6.81(m, 1H), 6.69~6.63(m, 4H) |
| 204 | 8.54(m, 1H), 8.28(m, 4H), 8.16(m, 1H), 7.77~7.36(m, 18H), 7.20(m, 2H), 7.00(s, 1H), 6.81(m, 1H), 6.63(m, 2H) |
| 212 | 8.54(m, 1H), 8.28(d, 4H), 8.16(m, 1H), 7.51∼7.41(m, 13H), 7.20(t, 2H), 6.89~6.81(m, 4H), 6.63~6.59(m, 5H) |
| 221 | 8.55∼8.54(m, 2H), 8.28~7.67(m, 4H), 8.16(m, 3H), 7.94(d, 1H), 7.67~7.25(m, 18H) |
| 224 | 8.54(m, 1H), 8.28(d, 4H), 8.16(m, 1H), 7.66~7.41(m, 21H), 7.20(t, 2H), 6.81(t, 1H), 6.69~6.63(m, 6H) |
| 227 | 8.54(m, 3H), 8.28(d, 4H), 8.16(m, 3H), 7.96~7.94(d, 2H), 7.67~7.41(m, 18H) |
| 243 | 8.28(d, 4H), 8.16(m, 2H), 8.00~7.81(m, 5H), 7.67~7.41(m, 14H), 7.25(d, 4H) |
| 271 | 8.55(d, 1H), 8.42(d, 1H), 8.28(d, 4H), 8.16~8.04(m, 4H), 7.67~7.41(m, 15H), 7.25(d, 4H) |
| 285 | 8.28(m, 2H), 8.16(d, 2H), 8.00~7.81(m, 11H), 7.73~7.51(m, 13H), 7.25(d, 2H) |
| 295 | 8.28~8.16(m, 5H), 8.00~7.89(m, 4H), 7.67~7.51(m, 20H) |
| 318 | 8.55~8.54(d, 2H), 8.42(d, 1H), 8.28(d, 4H), 8.16~8.04(m, 3H), 7.87~7.81(m, 2H), 7.66~7.41(m, 13H), 7.25(d, 4H) |
| 343 | 8.54(d, 1H), 8.28(d, 4H), 8.16(t, 1H), 7.67~7.41(m, 25H) |
| 353 | 8.28(d, 4H), 8.16(t, 2H), 7.87~7.81(m, 2H), 7.67(m, 2H), 7.51∼7.41(m, 13H) |
| 354 | 8.28(d, 4H), 8.16(t, 2H), 7.87~7.81(m, 2H), 7.70~7.67(m, 3H), 7.57∼7.41(m, 16H) |
| 356 | 8.28(d, 2H), 8.16(t, 2H), 7.95~7.66(m, 9H), 7.51~7.32(m, 12H) |
| 360 | 8.55(d, 1H), 8.42(d, 2H), 8.16~7.98(m, 6H), 7.87~7.79(m, 4H), 7.67~7.41(m, 12H), 7.25(s, 4H) |
| 363 | 8.55(d, 1H), 8.42(d, 1H), 8.16~8.04(m, 2H), 7.87~7.79(m, 6H), 7.67~7.55(m, 11H), 7.25(s, 4H) |

**[Table 14]**

| Compoun d | FD-MS | Compoun d | FD-MS |
|---|---|---|---|
| 1 | m/z=714.81 (C51H30N4O=714.24) | 181 | m/z=714.81 (C51H30N4O=714.24) |
| 3 | m/z=713.82 (C52H31N30=713.25) | 183 | m/z=714.81 (C51H30N4O=714.24) |
| 5 | m/z=790.91 (C57H34N4O=790.27) | 185 | m/z=690.79 (C49H30N4O=690.24) |
| 7 | m/z=829.94 (C59H35N5O=829.28) | 187 | m/z=740.85 (C53H32N4O=740.26) |
| 9 | m/z=690.79 (C49H30N4O=690.24) | 189 | m/z=740.85 (C53H32N4O=740.26) |
| 11 | m/z=790.91 (C57H34N4O=790.27) | 191 | m/z=743.87 (C52H29N3OS=743.20) |
| 13 | m/z=690.79 (C49H30N4O=690.24) | 193 | m/z=664.75 (C47H28N4O=664.23) |
| 15 | m/z=663.76 (C48H29N3O=663.23) | 195 | m/z=754.83 (C53H30N4O2=754.24) |
| 17 | m/z=643.75 (C44H25N3OS=643.27) | 197 | m/z=664.75 (C47H28N4O=664.23) |
| 19 | m/z=858.98 (C61H38N4O2=858.30) | 199 | m/z=743.87 (C52H29N3OS=743.20) |
| 21 | m/z=714.81 (C51H30N4O=714.24) | 201 | m/z=740.85 (C53H32N4O=740.26) |
| 23 | m/z=714.81 (C51H30N4O=714.24) | 203 | m/z=704.77 (C49H28N4O2=704.22) |
| 25 | m/z=690.79 (C49H30N4O=690.24) | 205 | m/z=714.81 (C51H30N4O=714.24) |
| 27 | m/z=740.85 (C53H32N4O=740.26) | 207 | m/z=764.87 (C55H32N4O=764.26) |
| 29 | m/z=740.85 (C53H32N4O=740.26) | 209 | m/z=798.95 (C55H34N4OS=798.25) |
| 31 | m/z=743.87 (C52H29N3OS=743.20) | 211 | m/z=732.87 (C52H36N4O=732.29) |
| 33 | m/z=664.75 (C47H28N4O=664.23) | 213 | m/z=745.86 (C53H35N4O2=745.27) |
| 35 | m/z=754.83 (C53H30N4O2=754.24) | 215 | m/z=742.86 (C53H34N4O=742.27) |
| 37 | m/z=664.75 (C47H28N4O=664.23) | 217 | m/z=768.90 (C55H36N4O=768.29) |
| 39 | m/z=743.87 (C52H29N3OS=743.20) | 219 | m/z=742.86 (C53H34N4O=742.27) |
| 41 | m/z=740.85 (C53H32N4O=740.26) | 221 | m/z=731.88 (C53H37N3O=731.29) |
| 43 | m/z=704.77 (C49H28N4O2=704.22) | 223 | m/z=666.77 (C47H30N4O=666.24) |
| 45 | m/z=714.81 (C51H30N4O=714.24) | 225 | m/z=741.88 (C54H35N3O=741.28) |
| 47 | m/z=764.87 (C55H32N4O=764.26) | 227 | m/z=742.86 (C53H34N4O=742.27) |
| 49 | m/z=798.95 (C55H34N4OS=798.25) | 229 | m/z=732.87 (C52H36N4O=732.29) |
| 51 | m/z=732.87 (C52H36N4O=732.29) | 231 | m/z=722.85 (C49H30N4O=722.21) |
| 53 | m/z=745.86 (C53H35N4O2=745.27) | 233 | m/z=768.90 (C55H36N4O=768.29) |
| 55 | m/z=742.86 (C53H34N4O=742.27) | 235 | m/z=782.88 (C55H34N4O2=782.27) |
| 57 | m/z=768.90 (C55H36N4O=768.29) | 237 | m/z=782.88 (C55H34N4O2=782.27) |
| 59 | m/z=742.86 (C53H34N4O=742.27) | 239 | m/z=764.87 (C55H32N4O=764.26) |
| 61 | m/z=745.86 (C53H35N4O2=745.27) | 241 | m/z=727.85 (C53H33N3O=727.26) |
| 63 | m/z=666.77 (C47H30N4O=666.24) | 243 | m/z=650.76 (C48H30N2O=650.24) |
| 65 | m/z=755.86 (C54H33N3O2=755.26) | 245 | m/z=694.80 (C48H26N2O2S=694.17) |
| 67 | m/z=742.86 (C53H34N4O=742.27) | 247 | m/z=680.81 (C48H28N2OS=680.19) |
| 69 | m/z=732.87 (C52H36N4O=732.29) | 249 | m/z=701.81 (C51H31NO3=701.25) |
| 71 | m/z=722.85 (C49H30N4O=722.21) | 251 | m/z=650.76 (C48H30N2O=650.24) |
| 73 | m/z=768.90 (C55H36N4O=768.29) | 253 | m/z=651.75 (C47H29N3O=651.23) |
| 75 | m/z=742.86 (C53H34N4O=742.27) | 255 | m/z=650.76 (C48H30N2O=650.24) |
| 77 | m/z=782.88 (C55H34N4O2=782.27) | 257 | m/z=651.75 (C47H29N3O=651.23) |
| 79 | m/z=764.87 (C55H32N4O=764.26) | 259 | m/z=651.75 (C47H29N3O=651.23) |
| 81 | m/z=714.81 (C51H30N4O=714.24) | 261 | m/z=701.81 (C51H31NO3=701.25) |
| 83 | m/z=713.82 (C52H31N30=713.25) | 263 | m/z=751.87 (C55H33N3O=751.26) |
| 85 | m/z=790.91 (C57H34N4O=790.27) | 265 | m/z=717.85 (C52H35N3O=717.28) |
| 87 | m/z=829.94 (C59H35N5O=829.28) | 267 | m/z=651.75 (C47H29N3O=651.23) |
| 89 | m/z=690.79 (C49H30N4O=690.24) | 269 | m/z=651.75 (C47H29N3O=651.23) |
| 91 | m/z=790.91 (C57H34N4O=790.27) | 271 | m/z=664.75 (C48H28N2O2=664.22) |
| 93 | m/z=690.79 (C49H30N4O=690.24) | 273 | m/z=700.82 (C52H32N2O=700.25) |
| 95 | m/z=663.76 (C48H29N3O=663.23) | 275 | m/z=650.76 (C48H30N2O=650.24) |
| 97 | m/z=643.75 (C44H25N3OS=643.27) | 277 | m/z=751.87 (C55H33N3O=751.26) |
| 99 | m/z=858.98 (C61H38N4O2=858.30) | 279 | m/z=651.75 (C47H29N3O=651.23) |
| 101 | m/z=714.81 (C51H30N4O=714.24) | 281 | m/z=753.89 (C55H35N3O=753.28) |
| 103 | m/z=714.81 (C51H30N4O=714.24) | 283 | m/z=650.76 (C48H30N2O=650.24) |
| 105 | m/z=690.79 (C49H30N4O=690.24) | 285 | m/z=604.72 (C42H24N2OS=604.16) |
| 107 | m/z=740.85 (C53H32N4O=740.26) | 287 | m/z=680.81 (C48H28N2OS=680.19) |
| 109 | m/z=740.85 (C53H32N4O=740.26) | 289 | m/z=701.81 (C51H31NO3=701.25) |
| 111 | m/z=743.87 (C52H29N3OS=743.20) | 291 | m/z=650.76 (C48H30N2O=650.24) |
| 113 | m/z=664.75 (C47H28N4O=664.23) | 293 | m/z=651.75 (C47H29N3O=651.23) |
| 115 | m/z=754.83 (C53H30N4O2=754.24) | 295 | m/z=650.76 (C48H30N2O=650.24) |
| 117 | m/z=664.75 (C47H28N4O=664.23) | 297 | m/z=651.75 (C47H29N3O=651.23) |
| 119 | m/z=743.87 (C52H29N3OS=743.20) | 299 | m/z=651.75 (C47H29N3O=651.23) |
| 121 | m/z=740.85 (C53H32N4O=740.26) | 301 | m/z=701.81 (C51H31NO3=701.25) |
| 123 | m/z=704.77 (C49H28N4O2=704.22) | 303 | m/z=751.87 (C55H33N3O=751.26) |
| 125 | m/z=714.81 (C51H30N4O=714.24) | 305 | m/z=717.85 (C52H35N3O=717.28) |
| 127 | m/z=764.87 (C55H32N4O=764.26) | 307 | m/z=651.75 (C47H29N3O=651.23) |
| 129 | m/z=798.95 (C55H34N4OS=798.25) | 309 | m/z=651.75 (C47H29N3O=651.23) |
| 131 | m/z=732.87 (C52H36N4O=732.29) | 311 | m/z=651.75 (C47H29N3O=651.23) |
| 133 | m/z=745.86 (C53H35N4O2=745.27) | 313 | m/z=700.82 (C52H32N2O=700.25) |
| 135 | m/z=742.86 (C53H34N4O=742.27) | 315 | m/z=650.76 (C48H30N2O=650.24) |
| 137 | m/z=768.90 (C55H36N4O=768.29) | 317 | m/z=751.87 (C55H33N3O=751.26) |
| 139 | m/z=742.86 (C53H34N4O=742.27) | 319 | m/z=651.75 (C47H29N3O=651.23) |
| 141 | m/z=745.86 (C53H35N4O2=745.27) | 321 | m/z=767.87 (C55H33N3O2=767.26) |
| 143 | m/z=666.77 (C47H30N4O=666.24) | 323 | m/z=650.76 (C48H30N2O=650.24) |
| 145 | m/z=755.86 (C54H33N3O2=755.26) | 325 | m/z=604.72 (C42H24N2OS=604.16) |
| 147 | m/z=742.86 (C53H34N4O=742.27) | 327 | m/z=680.81 (C48H28N2OS=680.19) |
| 149 | m/z=732.87 (C52H36N4O=732.29) | 329 | m/z=701.81 (C51H31NO3=701.25) |
| 151 | m/z=722.85 (C49H30N4O=722.21) | 331 | m/z=650.76 (C48H30N2O=650.24) |
| 153 | m/z=768.90 (C55H36N4O=768.29) | 333 | m/z=651.75 (C47H29N3O=651.23) |
| 155 | m/z=715.84 (C52H33N3O=715.26) | 335 | m/z=650.76 (C48H30N2O=650.24) |
| 157 | m/z=732.87 (C52H36N4O=732.29) | 337 | m/z=651.75 (C47H29N3O=651.23) |
| 159 | m/z=764.87 (C55H32N4O=764.26) | 339 | m/z=651.75 (C47H29N3O=651.23) |
| 161 | m/z=714.81 (C51H30N4O=714.24) | 341 | m/z=701.81 (C51H31NO3=701.25) |
| 163 | m/z=713.82 (C52H31N30=713.25) | 343 | m/z=751.87 (C55H33N3O=751.26) |
| 165 | m/z=790.91 (C57H34N4O=790.27) | 345 | m/z=717.85 (C52H35N3O=717.28) |
| 167 | m/z=714.81 (C51H30N4O=714.24) | 347 | m/z=651.75 (C47H29N3O=651.23) |
| 169 | m/z=690.79 (C49H30N4O=690.24) | 349 | m/z=651.75 (C47H29N3O=651.23) |
| 171 | m/z=790.91 (C57H34N4O=790.27) | 351 | m/z=651.75 (C47H29N3O=651.23) |
| 173 | m/z=690.79 (C49H30N4O=690.24) | 353 | m/z=700.82 (C52H32N2O=700.25) |
| 175 | m/z=663.76 (C48H29N3O=663.23) | 355 | m/z=650.76 (C48H30N2O=650.24) |
| 177 | m/z=643.75 (C44H25N3OS=643.27) | 357 | m/z=751.87 (C55H33N3O=751.26) |
| 179 | m/z=858.98 (C61H38N4O2=858.30) | 359 | m/z=651.75 (C47H29N3O=651.23) |

### <Experimental Example 1>-Manufacture of Organic Light Emitting Device

### 1) Manufacture of Organic Light Emitting Device (Red Single Host)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4'4"-tris[2-naphthyl(phenyl)amino] triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 500 Å using a compound described in the following Table 15 as a red host and (piq)₂(Ir) (acac) as a red phosphorescent dopant by doping the (piq)₂(Ir) (acac) to the host in 3 wt%. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured (Examples 1 to 18 and Comparative Examples 1 to 6).

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 1 to 18 and Comparative Examples 1 to 6 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. T₉₀ means a lifetime (unit: h, time), a time taken to become 90% with respect to initial luminance.

Properties of the organic light emitting devices of the present disclosure are as shown in the following Table 15.

**[Table 15]**

| | Compound | Driving Voltage (V) | Efficiency (cd/A) | Color Coordinate (x, y) | Lifetime (T₉₀) |
|---|---|---|---|---|---|
| Comparative Example 1 | Comparative Compound A | 5.35 | 10.5 | 0.672, 0.328 | 55 |
| Comparative Example 2 | Comparative Compound B | 5.20 | 8.7 | 0.676, 0.324 | 58 |
| Comparative Example 3 | Comparative Compound C | 5.16 | 9.2 | 0.680, 0.320 | 49 |
| Comparative Example 4 | Comparative Compound D | 5.28 | 8.1 | 0.677, 0.322 | 47 |
| Comparative Example 5 | Comparative Compound E | 5.32 | 7.5 | 0.675, 0.325 | 48 |
| Comparative Example 6 | Comparative Compound F | 5.19 | 6.9 | 0.674, 0.326 | 52 |
| Example 1 | Compound 1 | 4.23 | 25.1 | 0.679, 0.321 | 138 |
| Example 2 | Compound 14 | 4.56 | 23.9 | 0.674, 0.326 | 141 |
| Example 3 | Compound 23 | 4.85 | 24.5 | 0.685, 0.315 | 129 |
| Example 4 | Compound 30 | 4.61 | 22.9 | 0.673, 0.327 | 131 |
| Example 5 | Compound 90 | 4.51 | 26.0 | 0.688, 0.312 | 140 |
| Example 6 | Compound 103 | 4.23 | 24.5 | 0.686, 0.314 | 142 |
| Example 7 | Compound 128 | 4.44 | 25.7 | 0.672, 0.328 | 132 |
| Example 8 | Compound 130 | 4.56 | 24.8 | 0.683, 0.317 | 129 |
| Example 9 | Compound 161 | 4.75 | 23.9 | 0.675, 0.325 | 119 |
| Example 10 | Compound 174 | 4.21 | 24.5 | 0.672, 0.328 | 120 |
| Example 11 | Compound 191 | 4.32 | 25.3 | 0.673, 0.32 | 138 |
| Example 12 | Compound 212 | 4.33 | 25.4 | 0.687, 0.313 | 129 |
| Example 13 | Compound 243 | 4.32 | 26.2 | 0.677, 0.323 | 131 |
| Example 14 | Compound 271 | 4.40 | 23.9 | 0.675, 0.325 | 140 |
| Example 15 | Compound 285 | 4.21 | 22.7 | 0.684, 0.316 | 142 |
| Example 16 | Compound 295 | 4.18 | 26.5 | 0.682, 0.318 | 139 |
| Example 17 | Compound 318 | 4.51 | 24.9 | 0.673, 0.327 | 132 |
| Example 18 | Compound 343 | 4.71 | 25.5 | 0.684, 0.316 | 128 |

Comparative Compounds A to F of Table 15 are as follows.

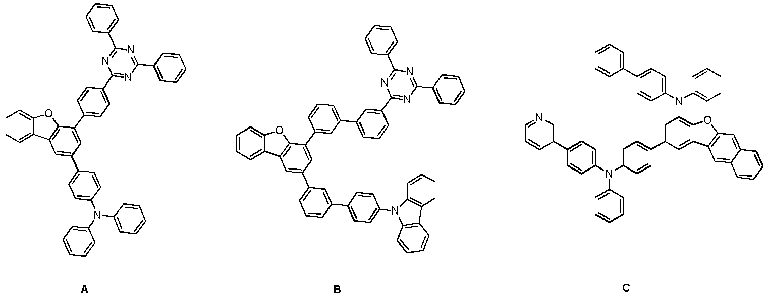

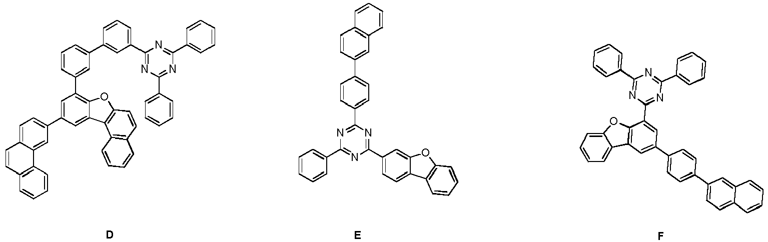

As seen from the results of Table 15, the organic light emitting device using the heterocyclic compound of the present disclosure had a lower driving voltage and significantly improved light emission efficiency and lifetime compared to Comparative Examples 1 to 6. This is considered to be due to the fact that the compound of the present disclosure has, while having high thermal stability, proper molecular weight and band gap by the heteroaryl group being linked without a linker. In other words, when using the heterocyclic compound of the present disclosure in a light emitting layer, a proper band gap is formed in the light emitting layer, which prevents losses of electrons and holes helping to form an effective recombination zone of the electrons and the holes.

Particularly, among the compounds of the present disclosure, the compound with an arylamine group had a relatively reduced lifetime and higher efficiency, and it was identified that strong HT properties of the arylamine group resolved a hole trap occurring in the dopant.

### <Experimental Example 2>-Manufacture of Organic Light Emitting Device (Red N+P Mixed Host)

### 1) Manufacture of Organic Light Emitting Device (Red N+P Mixed Host)

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4'4"-tris[2-naphthyl(phenyl)amino] triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 400 Å by mixing an arylamine compound and a unipolar compound or mixing an arylamine compound and a bipolar compound as described in the following Table 16 as a red host, and doping and depositing (piq)₂(Ir)(acac) in 3 wt% as a red phosphorescent dopant.

From device evaluation results on Compound 14 and Compound 295 (Examples 19 to 23), it was identified that the ratio of 1:1 resulted in most superior performance, and after that, the deposition ratio of the arylamine and the unipolar compound was fixed at 1:1. From device evaluation results on Compound 13 and Compound 22 (Examples 34 to 38), the deposition ratio of the arylamine and the bipolar compound was fixed at 1:2.

After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an an organic light emitting device was manufactured (Examples 19 to 41).

In addition, organic light emitting devices of Examples 42 to 65 were manufactured in the same manner as the organic light emitting devices of Examples 19 to 41 except that the compounds of the present disclosure and Compounds G to P were used as described in the following Table 17.

Specifically, through a deposition ratio test of Compound G and Compound 243, it was identified that most superior performance was obtained in the ratio of 1:1, and the ratio was fixed when conducting the device evaluation thereafter.

Meanwhile, all the organic compounds required to manufacture the OLED were vacuum sublimation purified under 10⁻⁸ torr to 10⁻⁶ torr for each material to be used in the OLED manufacture.

### 2) Driving Voltage and Light Emission Efficiency of Organic Light Emitting Device

For each of the organic light emitting devices of Examples 19 to 65 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. T₉₀ means a lifetime (unit: h, time), a time taken to become 90% with respect to initial luminance.

Properties of the organic light emitting devices of the present disclosure are as shown in the following Table 16 and Table 17.

**[Table 16]**

| | Light Emitting Layer Compound | Ratio (N:P) | Drivin g Voltag e (V) | Effici ency (cd/A) | Color Coordinate (x, y) | Lifet ime (T₉₀) |
|---|---|---|---|---|---|---|
| Example 19 | Compound 14:Compound 295 | 1:3 | 5.01 | 25.7 | 0.677, 0.323 | 171 |
| Example 20 | | 1:2 | 4.95 | 26.9 | 0.676, 0.324 | 186 |
| Example 21 | | 1:1 | 4.15 | 28.5 | 0.679, 0.321 | 241 |
| Example 22 | | 2:1 | 4.65 | 27.1 | 0.674, 0.326 | 191 |
| Example 23 | | 3:1 | 4.75 | 24.8 | 0.673, 0.327 | 188 |
| Example 24 | Compound 41:Compound 363 | 1:1 | 4.35 | 28.5 | 0.674, 0.326 | 190 |
| Example 25 | Compound 30:Compound 343 | 1:1 | 4.45 | 27.3 | 0.681, 0.319 | 181 |
| Example 26 | Compound 31:Compound 243 | 1:1 | 4.80 | 30.9 | 0.673, 0.327 | 179 |
| Example 27 | Compound 57:Compound 271 | 1:1 | 4.71 | 29.1 | 0.682, 0.318 | 250 |
| Example 28 | Compound 64:Compound 285 | 1:1 | 4.65 | 31.8 | 0.677, 0.323 | 195 |
| Example 29 | Compound 103:Compound 256 | 1:1 | 4.10 | 27.9 | 0.675, 0.325 | 234 |
| Example 30 | Compound 122:Compound 254 | 1:1 | 4.05 | 28.1 | 0.682, 0.318 | 179 |
| Example 31 | Compound 85:Compound 260 | 1:1 | 4.32 | 26.8 | 0.673, 0.327 | 185 |
| Example 32 | Compound 146:Compound 260 | 1:1 | 4.86 | 28.2 | 0.675, 0.325 | 248 |
| Example 33 | Compound 174:Compound 285 | 1:1 | 4.26 | 29.3 | 0.677, 0.323 | 239 |
| Example 34 | Compound 13:Compound 22 | 1:3 | 4.65 | 25.7 | 0.682, 0.318 | 201 |
| Example 35 | | 1:2 | 4.45 | 26.2 | 0.681, 0.319 | 222 |
| Example 36 | | 1:1 | 4.38 | 28.2 | 0.677, 0.323 | 218 |
| Example 37 | | 2:1 | 4.41 | 25.7 | 0.678, 0.322 | 196 |
| Example 38 | | 3:1 | 4.43 | 24.6 | 0.676, 0.324 | 175 |
| Example 39 | Compound 103:Compound 153 | 1:2 | 4.32 | 27.2 | 0.684, 0.316 | 219 |
| Example 40 | Compound 123:Compound 90 | 1:2 | 4.22 | 29.8 | 0.685, 0.315 | 215 |
| Example 41 | Compound 204:Compound 221 | 1:2 | 4.10 | 28.1 | 0.676, 0.324 | 194 |

**[Table 17]**

| | Light Emitting Layer Compound | Ratio (N:P) | Driving Voltage (V) | Efficie ncy (cd/A) | Color Coordinate (x, y) | Life time (T₉₀) |
|---|---|---|---|---|---|---|
| Example 42 | G:Compound 243 | 1:3 | 4.89 | 22 | 0.628, 0.312 | 108 |
| Example 43 | | 1:2 | 4.53 | 23 | 0.678, 0.322 | 112 |
| Example 44 | | 1:1 | 4.34 | 25 | 0.680, 0.320 | 120 |
| Example 45 | | 2:1 | 4.45 | 24 | 0.679, 0.321 | 109 |
| Example 46 | | 3:1 | 4.57 | 23 | 0.682, 0.318 | 105 |
| Example 47 | H:Compound 271 | 1:1 | 4.23 | 25 | 0.681, 0.319 | 119 |
| Example 48 | I:Compound 353 | 1:1 | 4.56 | 24 | 0.677, 0.323 | 109 |
| Example 49 | J:Compound 356 | 1:1 | 4.12 | 26 | 0.676, 0.324 | 110 |
| Example 50 | K:Compound 285 | 1:1 | 4.23 | 24 | 0.683, 0.317 | 112 |
| Example 51 | G:Compound 295 | 1:1 | 4.64 | 25 | 0.674, 0.326 | 116 |
| Example 52 | H:Compound 318 | 1:1 | 4.57 | 24 | 0.682, 0.318 | 120 |
| Example 53 | I:Compound 343 | 1:1 | 4.45 | 25 | 0.681, 0.319 | 117 |
| Example 54 | J:Compound 360 | 1:1 | 4.32 | 26 | 0.675, 0.325 | 115 |
| Example 55 | K:Compound 354 | 1:1 | 4.44 | 24 | 0.677, 0.323 | 109 |
| Example 56 | G:Compound 363 | 1:1 | 4.65 | 25 | 0.678, 0.322 | 120 |
| Example 57 | H:Compound 1 | 1:1 | 4.21 | 24 | 0.681, 0.319 | 121 |
| Example 58 | I:Compound 22 | 1:1 | 4.15 | 23 | 0.682, 0.318 | 110 |
| Example 59 | J:Compound 153 | 1:1 | 4.27 | 26 | 0.679, 0.321 | 104 |
| Example 60 | K:Compound 165 | 1:1 | 4.31 | 25 | 0.677, 0.323 | 109 |
| Example 61 | L:Compound 31 | 1:1 | 4.25 | 23 | 0.673, 0.327 | 170 |
| Example 62 | M:Compound 1 | 1:1 | 4.31 | 22 | 0.675, 0.325 | 181 |
| Example 63 | N:Compound 41 | 1:1 | 4.40 | 24 | 0.681, 0.319 | 169 |
| Example 64 | O:Compound 103 | 1:1 | 4.19 | 22 | 0.677, 0.323 | 175 |
| Example 65 | P:Compound 212 | 1:1 | 4.38 | 21 | 0.682, 0.318 | 184 |

Compounds G to P described in Table 17 are as follows.

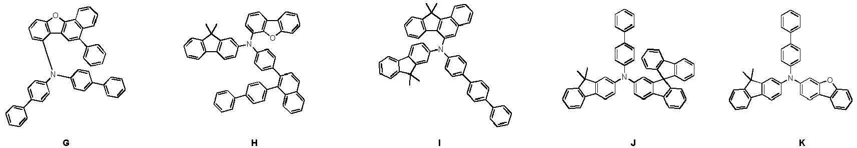

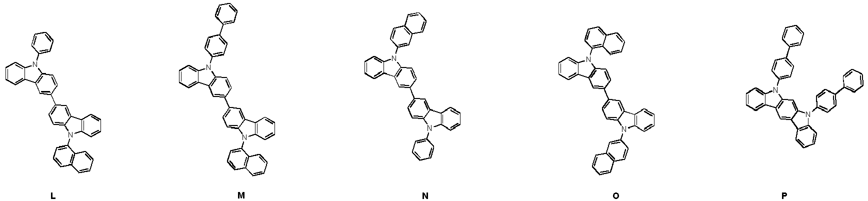

Compounds G to K are arylamine derivatives different from the compounds of the present disclosure, and Compounds L to P are compounds corresponding to common biscarbazole or indolecarbazole derivatives used as a P-type host.

As seen from the results of Table 16 and Table 17, effects of more superior efficiency and lifetime were obtained when depositing the compounds according to the present application after mixing as the light emitting layer of the organic light emitting device. This result is considered to be from a result of improving a phenomenon of reducing a lifetime generally occurring when using a compound comprising an arylamine group in a light emitting layer of an organic light emitting device.

In other words, it may be predicted that an exciplex phenomenon of N+P compounds occurs when comprising both a host having strong HT properties and the compound according to the present application. The exciplex phenomenon of N+P compounds is a phenomenon of releasing energy having sizes of a donor (p-host) HOMO level and an acceptor (n-host) LUMO level due to electron exchanges between two molecules. When a donor (p-host) having a favorable hole transfer ability and an acceptor (n-host) having a favorable electron transfer ability are used as a host of a light emitting layer, holes are injected to the p-host and electrons are injected to the n-host due the exciplex phenomenon of N+P compounds, and therefore, a driving voltage may be lowered, which resultantly helps with enhancement in the lifetime.

### <Reference Numeral>

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

## Claims

1. A heterocyclic compound represented by the following Chemical Formula 1: 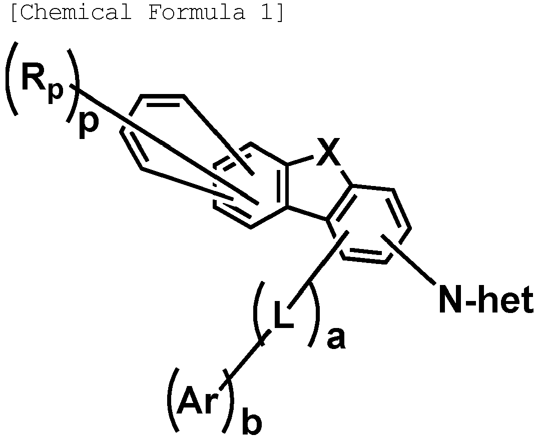 wherein, in Chemical Formula 1,
L is a direct bond; a substituted or unsubstituted arylene group having 6 to 60 carbon atoms; or a substituted or unsubstituted heteroarylene group having 2 to 60 carbon atoms;
X is O; or S;
N-het is a monocyclic or polycyclic heterocyclic group substituted or unsubstituted, and comprising one or more Ns;
Ar is a substituted or unsubstituted aryl group having 6 to 60 carbon atoms; a substituted or unsubstituted heteroaryl group having 2 to 60 carbon atoms; or an amine group unsubstituted or substituted with one or more selected from the group consisting of a substituted or unsubstituted aryl group having 6 to 60 carbon atoms and a substituted or unsubstituted heteroaryl group having 2 to 60 carbon atoms;
Rₚ is hydrogen; deuterium; a halogen group; a cyano group; a substituted or unsubstituted alkyl group having 1 to 30 carbon atoms; a substituted or unsubstituted cycloalkyl group having 3 to 30 carbon atoms; a substituted or unsubstituted aryl group having 6 to 40 carbon atoms; or a substituted or unsubstituted heteroaryl group having 2 to 40 carbon atoms;
a is an integer of 0 to 3, and when a is 2 or greater, substituents in the parentheses are the same as or different from each other;
b is an integer of 0 to 4, and when b is 2 or greater, substituents in the parentheses are the same as or different from each other; and
p is an integer of 0 to 6, and when p is 2 or greater, substituents in the parentheses are the same as or different from each other.

2. The heterocyclic compound of Claim 1, wherein the "substituted or unsubstituted" means being substituted with one or more substituents selected from the group consisting of a linear or branched alkyl group having 1 to 60 carbon atoms; a linear or branched alkenyl group having 2 to 60 carbon atoms; a linear or branched alkynyl group having 2 to 60 carbon atoms; a monocyclic or polycyclic cycloalkyl group having 3 to 60 carbon atoms; a monocyclic or polycyclic heterocycloalkyl group having 2 to 60 carbon atoms; a monocyclic or polycyclic aryl group having 6 to 60 carbon atoms; a monocyclic or polycyclic heteroaryl group having 2 to 60 carbon atoms; a silyl group; a phosphine oxide group; and an amine group, or being unsubstituted, or being substituted with a substituent linking two or more substituents selected from among the substituents illustrated above, or being unsubstituted.

3. The heterocyclic compound of Claim 1, wherein Chemical Formula 1 is represented by any one of the following Chemical Formulae 1-1 to 1-3: 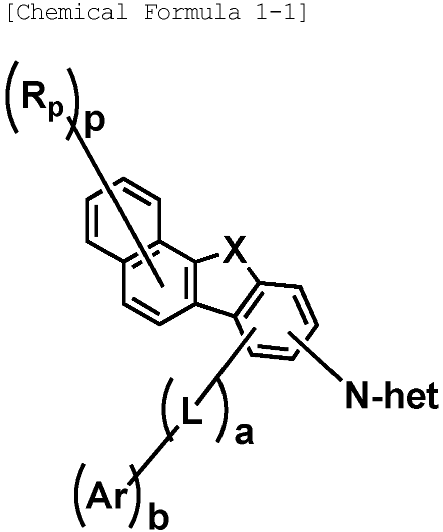 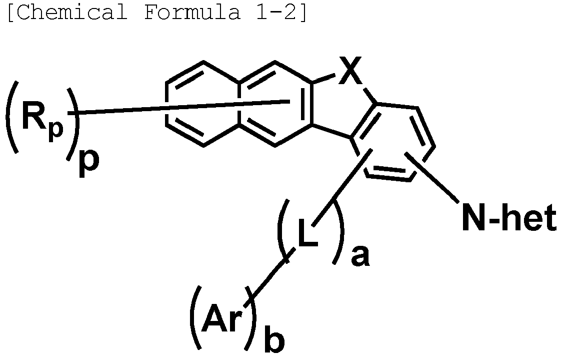 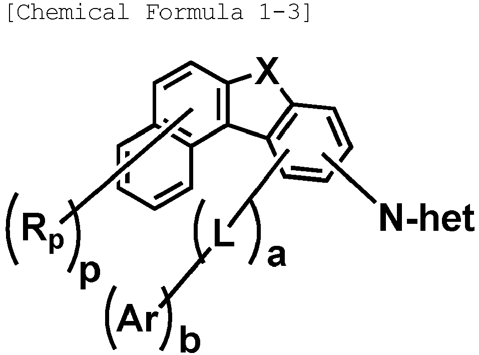 in Chemical Formulae 1-1 to 1-3,
each substituent has the same definition as in Chemical Formula 1.

4. The heterocyclic compound of Claim 1, wherein N-het is represented by the following Chemical Formula A: 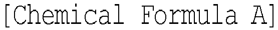 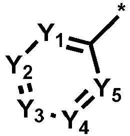 in Chemical Formula A,
* is a position at which Chemical Formula 1 and N-het bond;
Y₁ to Y₅ are the same as or different from each other and each independently N or CRa, at least one or more of Y₁ to Y₅ are N, and when there are two or more CRas, Ras are the same as or different from each other; and
Ra is selected from the group consisting of hydrogen; deuterium; a substituted or unsubstituted aryl group having 6 to 60 carbon atoms; and a substituted or unsubstituted heteroaryl group having 2 to 60 carbon atoms, or two or more groups adjacent to each other bond to each other to form a substituted or unsubstituted aromatic hydrocarbon ring having 6 to 60 carbon atoms or a substituted or unsubstituted heteroring having 2 to 60 carbon atoms.

5. The heterocyclic compound of Claim 4, wherein Chemical Formula A is represented by any one of the following Chemical Formulae A-1 to A-7: 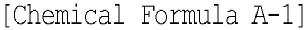 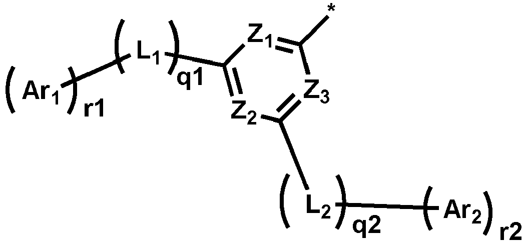 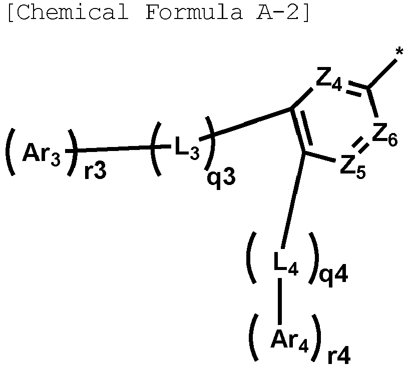 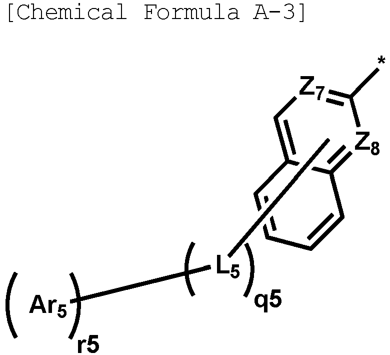 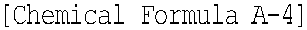 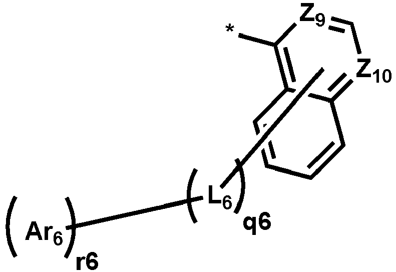 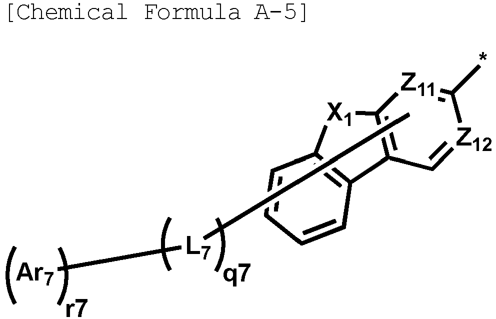 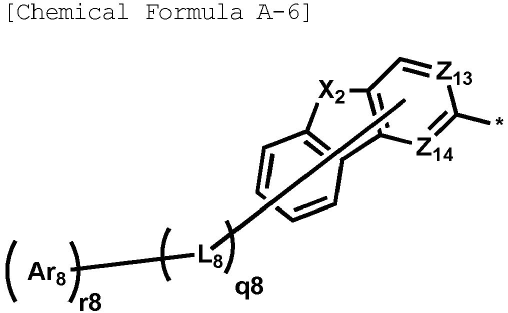 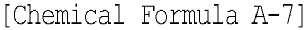 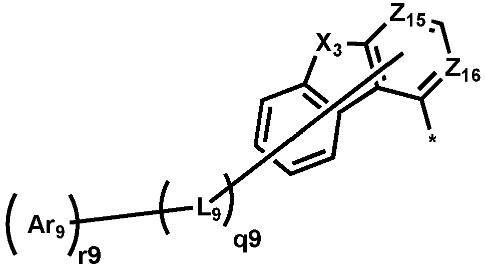 in Chemical Formulae A-1 to A-7,
* is a position at which Chemical Formula 1 and N-het bond;
Z₁ to Z₁₆ are the same as or different from each other and each independently N or CH, at least one or more of Z₁ to Z₃ are N, at least one or more of Z₄ to Z₆ are N, at least one or more of Z₇ and Z₈ are N, at least one or more of Z₉ and Z₁₀ are N, at least one or more of Z₁₁ and Z₁₂ are N, at least one or more of Z₁₃ and Z₁₄ are N, and at least one or more of Z₁₅ and Z₁₆ are N;
L₁ to L₉ are the same as or different from each other, and each independently a direct bond; a substituted or unsubstituted arylene group having 6 to 60 carbon atoms; or a substituted or unsubstituted heteroarylene group having 2 to 60 carbon atoms;
Ar₁ to Ar₉ are the same as or different from each other, and each independently hydrogen; deuterium; a substituted or unsubstituted aryl group having 6 to 60 carbon atoms; or a substituted or unsubstituted heteroaryl group having 2 to 60 carbon atoms;
X₁ to X₃ are the same as or different from each other, and each independently O; or S;
q1 to q9 are each independently an integer of 0 to 2, and when q1 to q9 are each 2, substituents in the parentheses are the same as or different from each other; and
r1 to r4 are each independently an integer of 1 to 3, r5 to r9 are each independently an integer of 1 to 5, and when r1 to q9 are each 2 or greater, substituents in the parentheses are the same as or different from each other.

6. The heterocyclic compound of Claim 1, wherein Ar is a substituted or unsubstituted aryl group having 6 to 60 carbon atoms; a substituted or unsubstituted heteroaryl group having 2 to 60 carbon atoms; or a group represented by the following Chemical Formula B: 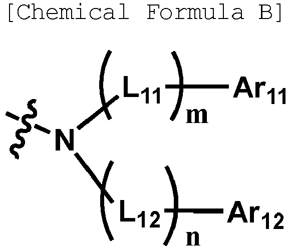 in Chemical Formula B,
L₁₁ and L₁₂ are the same as or different from each other, and each independently a direct bond; a substituted or unsubstituted arylene group having 6 to 40 carbon atoms; or a substituted or unsubstituted heteroarylene group having 2 to 40 carbon atoms;
Ar₁₁ and Ar₁₂ are the same as or different from each other, and each independently selected from the group consisting of a substituted or unsubstituted aryl group having 6 to 40 carbon atoms; and a substituted or unsubstituted heteroaryl group having 2 to 40 carbon atoms, or Ar₁₁ and Ar₁₂ bond to each other to form a substituted or unsubstituted aromatic hydrocarbon ring having 6 to 60 carbon atoms or a substituted or unsubstituted heteroring having 2 to 60 carbon atoms;
m and n are each 0 or 1; and 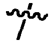
means a position bonding to L of Chemical Formula 1.

7. The heterocyclic compound of Claim 6, wherein Chemical Formula B is represented by any one of the following Chemical Formulae B-1 to B-5: 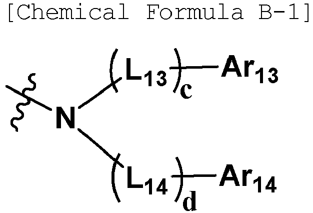 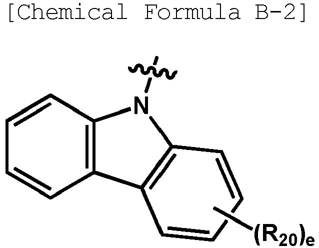 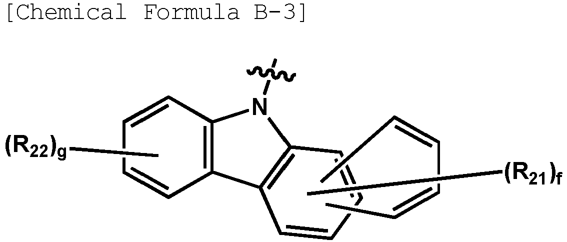 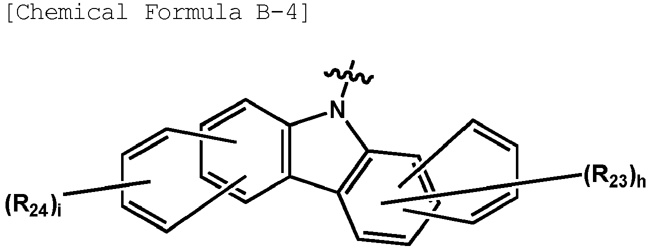 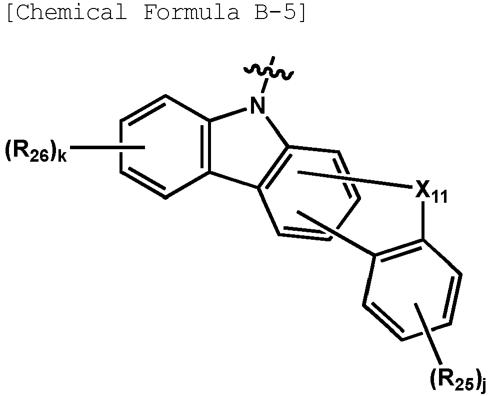 in Chemical Formulae B-1 to B-5,
L₁₃ and L₁₄ are the same as or different from each other, and each independently a direct bond; a substituted or unsubstituted arylene group having 6 to 40 carbon atoms; or a substituted or unsubstituted heteroarylene group having 2 to 40 carbon atoms;
Ar₁₃ and Ar₁₄ are the same as or different from each other, and each independently a substituted or unsubstituted aryl group having 6 to 40 carbon atoms; or a substituted or unsubstituted heteroaryl group having 2 to 40 carbon atoms;
R₂₀ to R₂₆ are each independently hydrogen; deuterium; a halogen group; a cyano group; a substituted or unsubstituted aryl group having 6 to 20 carbon atoms; or a substituted or unsubstituted heteroaryl group having 2 to 20 carbon atoms;
X₁₁ is O; S; or NRc, and Rc is a substituted or unsubstituted aryl group having 6 to 20 carbon atoms;
c and d are each 0 or 1;
e, g, j and k are each an integer of 0 to 4, f, h and i are each an integer of 0 to 6, and when e to k are each 2 or greater, substituents in the parentheses are the same as or different from each other; and 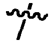
means a position bonding to L of Chemical Formula 1.

8. The heterocyclic compound of Claim 1, wherein Chemical Formula 1 is represented by any one of the following compounds:

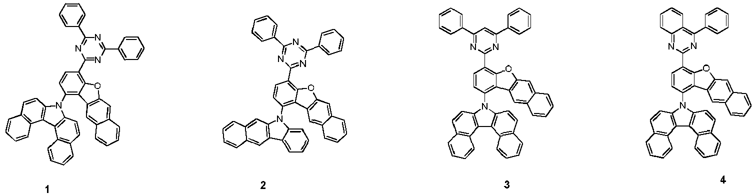

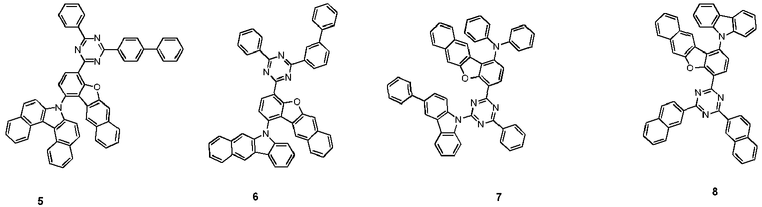

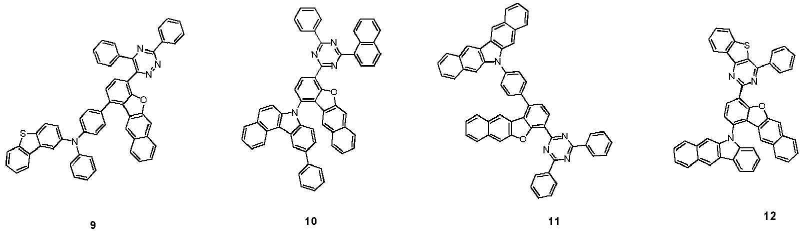

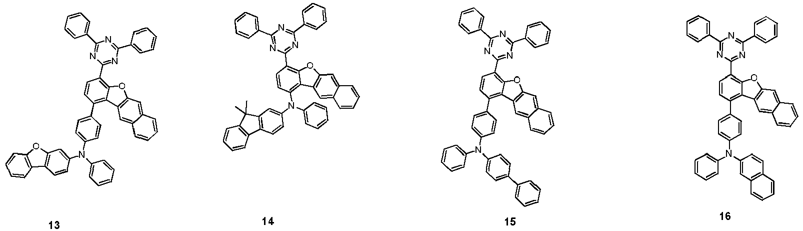

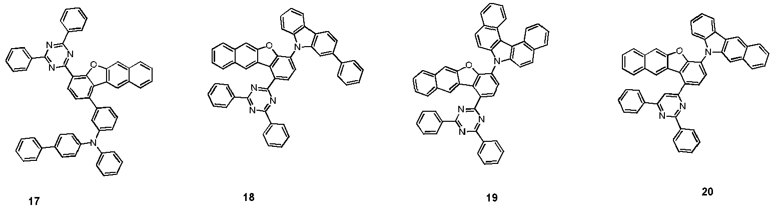

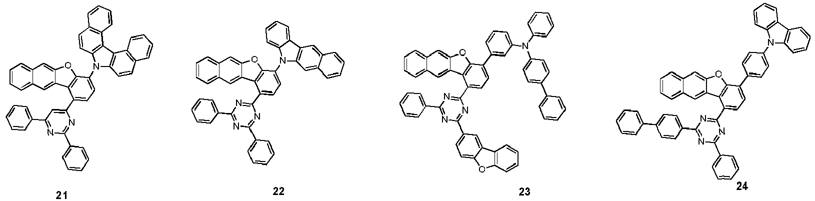

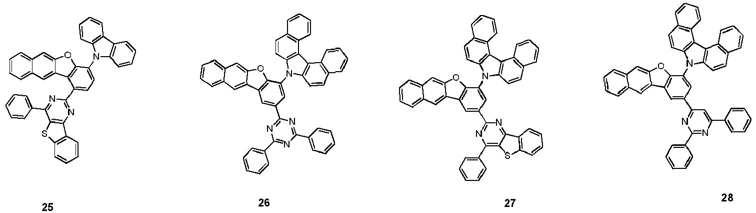

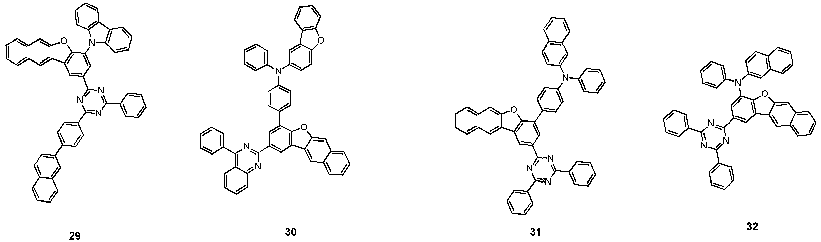

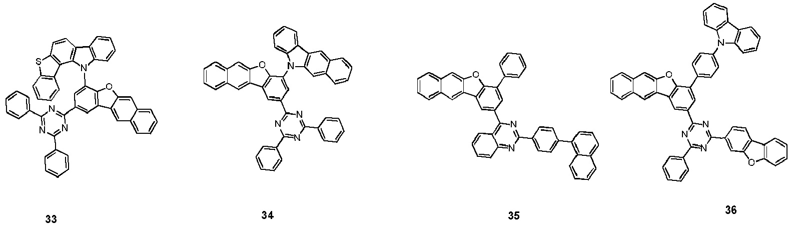

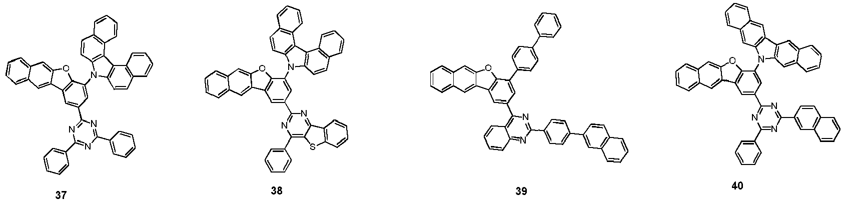

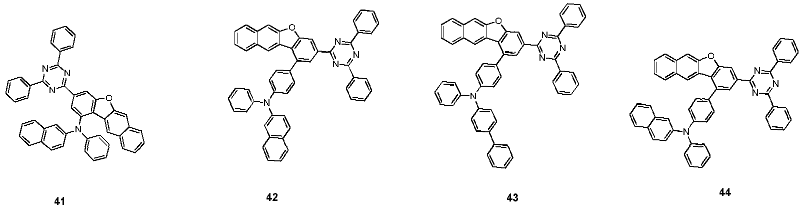

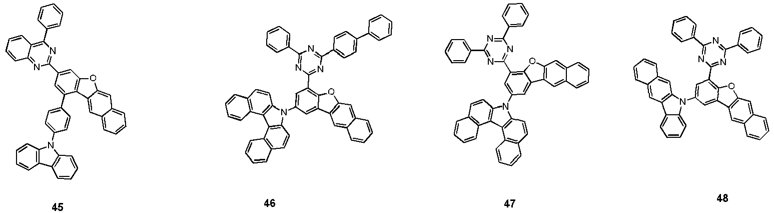

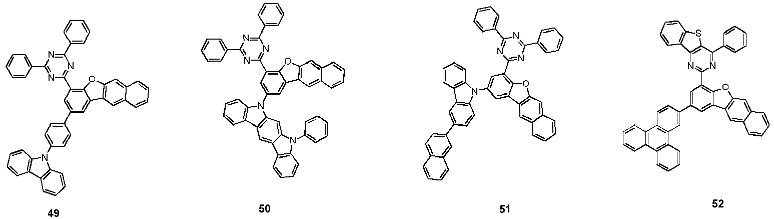

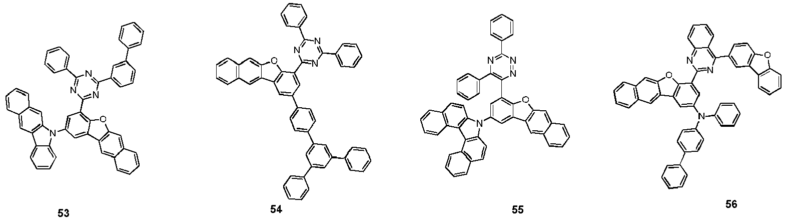

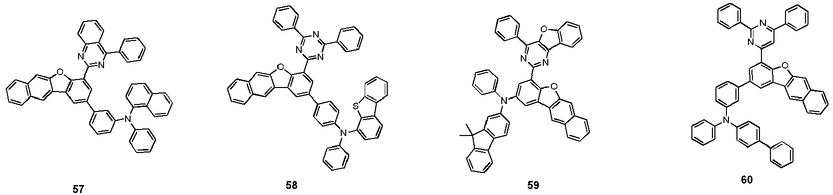

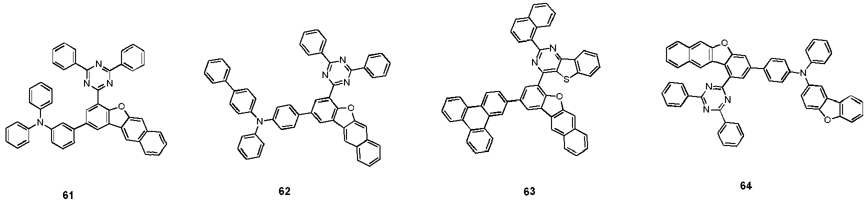

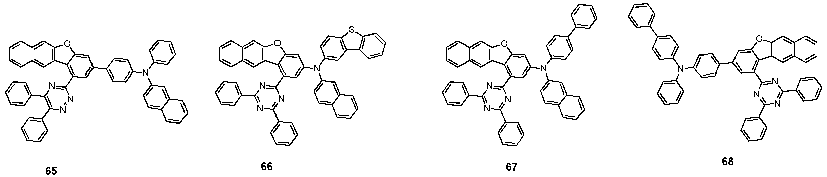

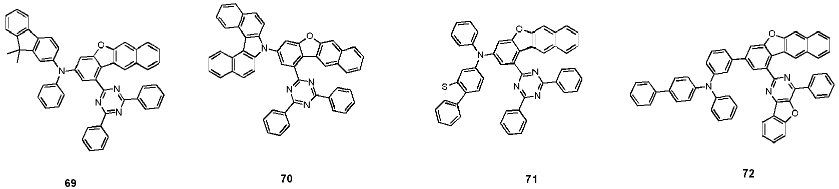

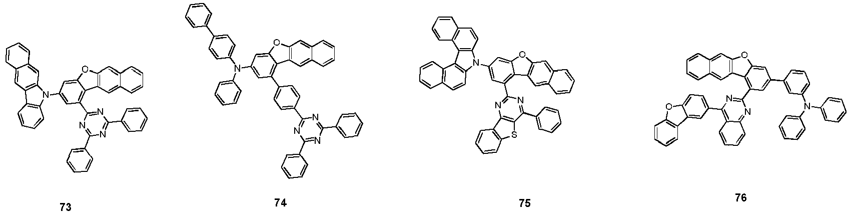

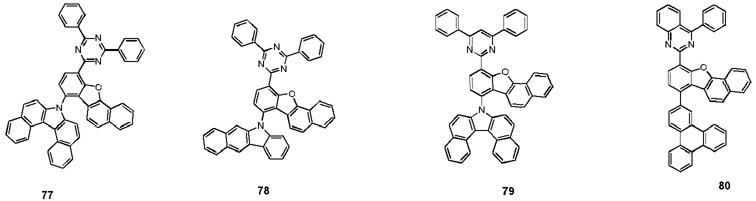

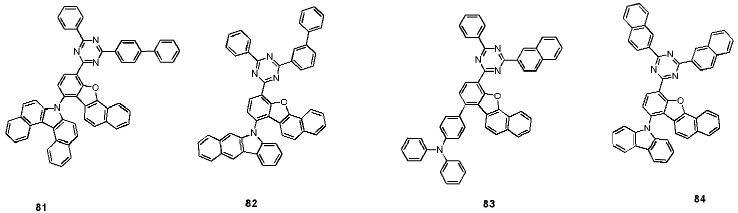

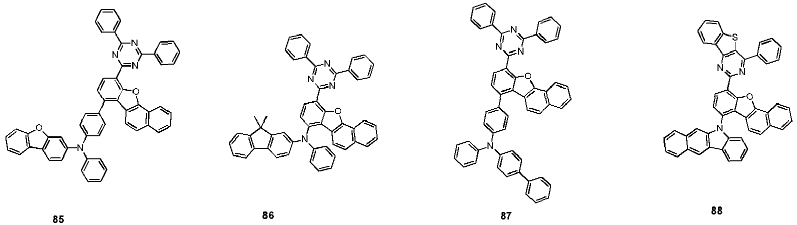

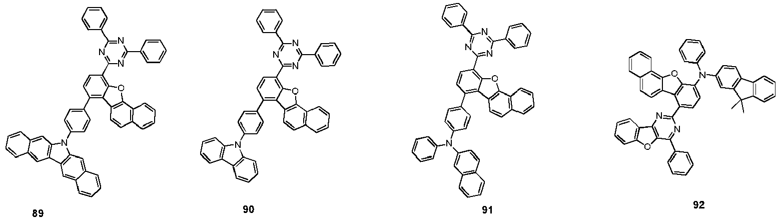

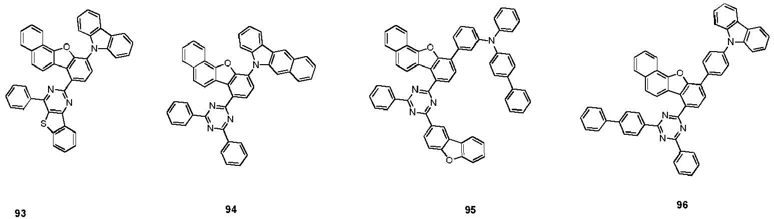

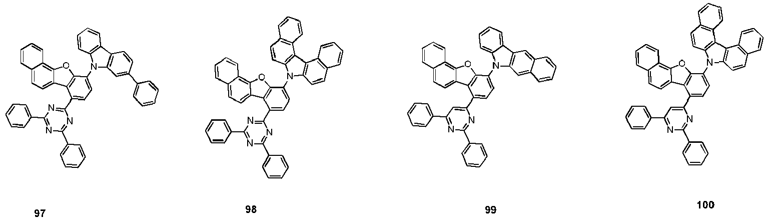

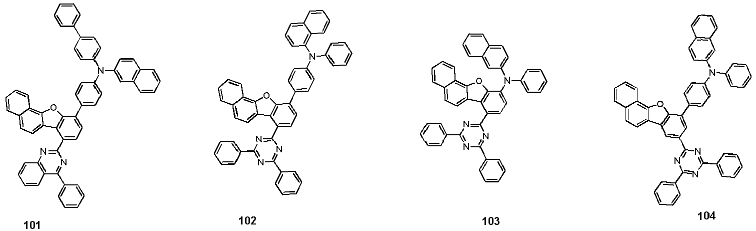

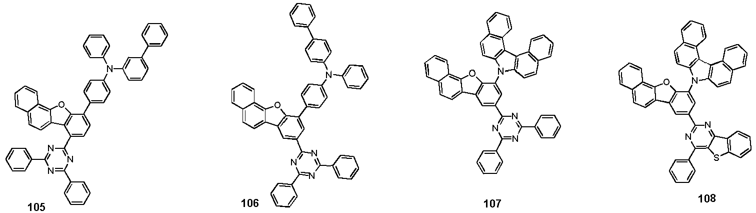

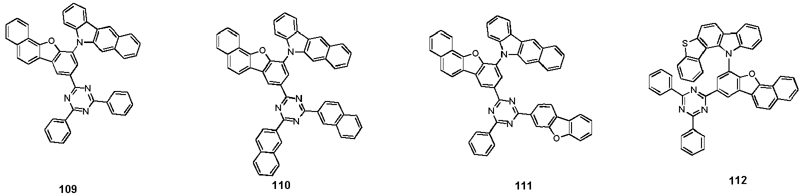

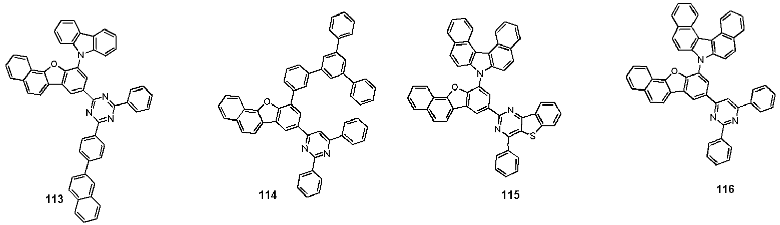

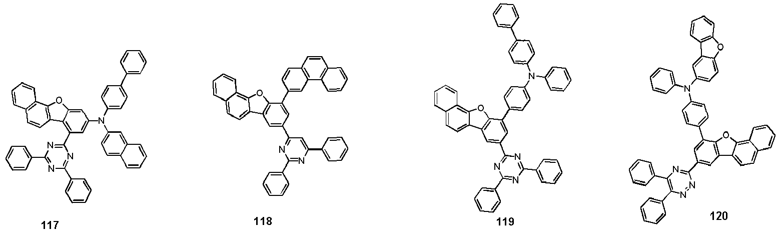

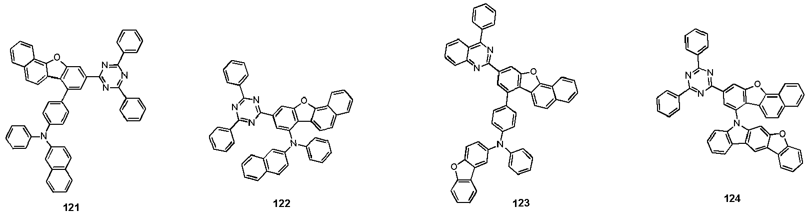

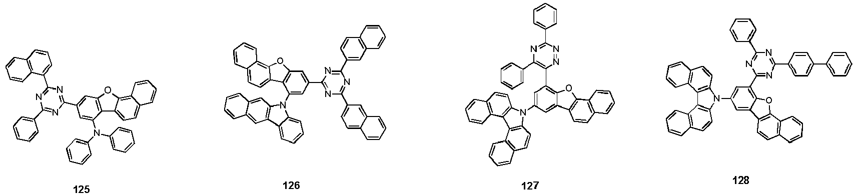

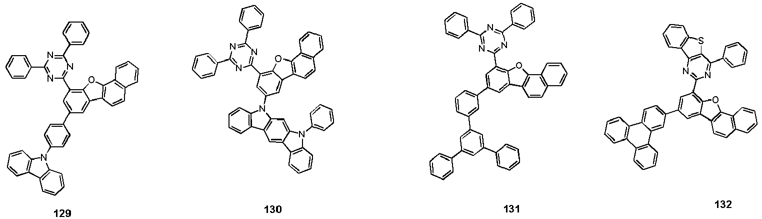

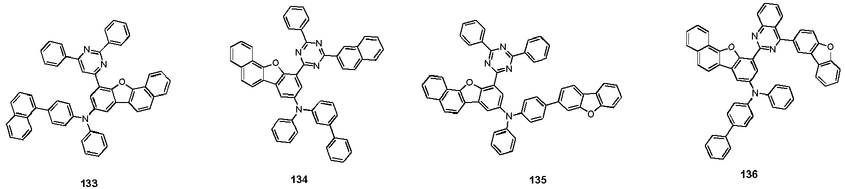

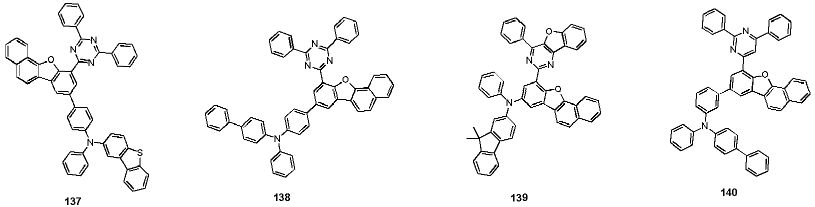

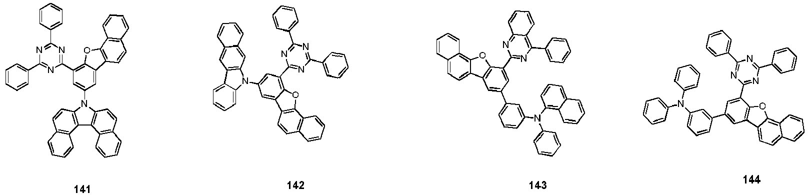

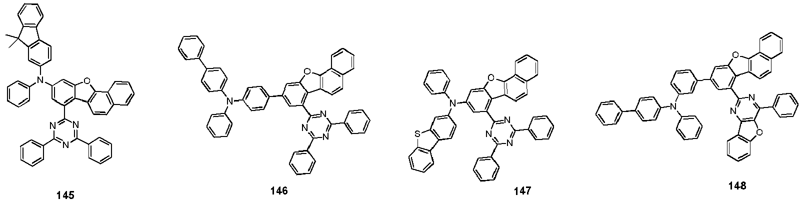

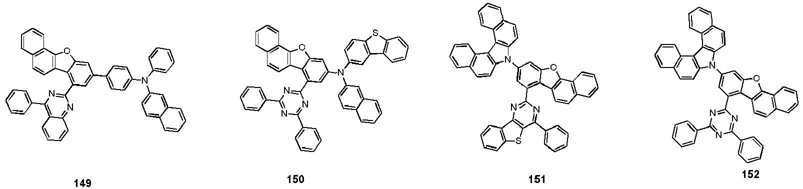

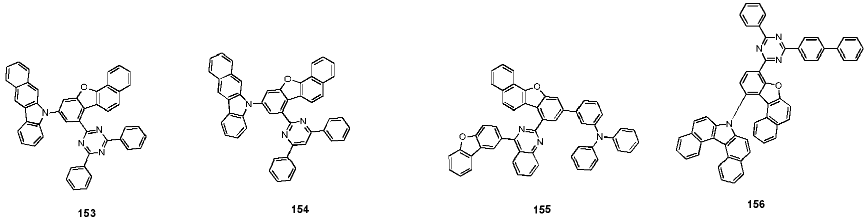

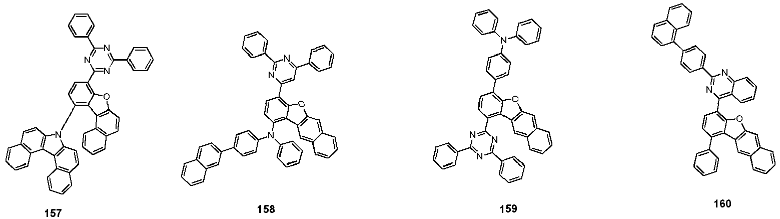

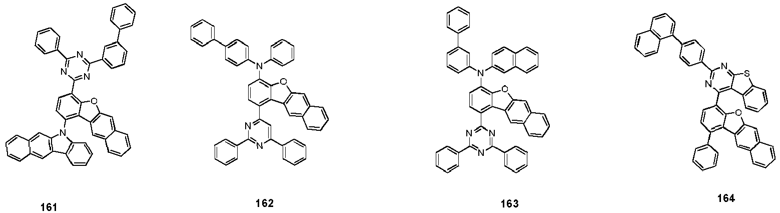

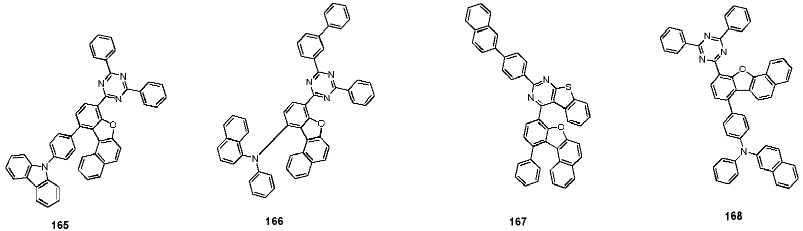

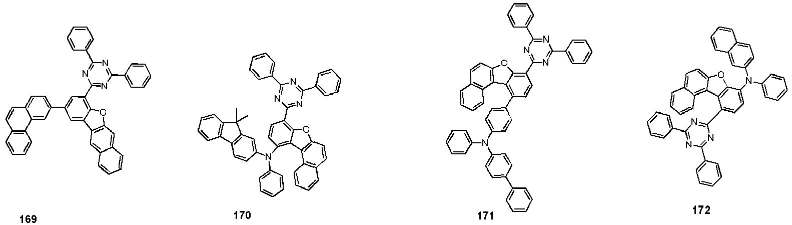

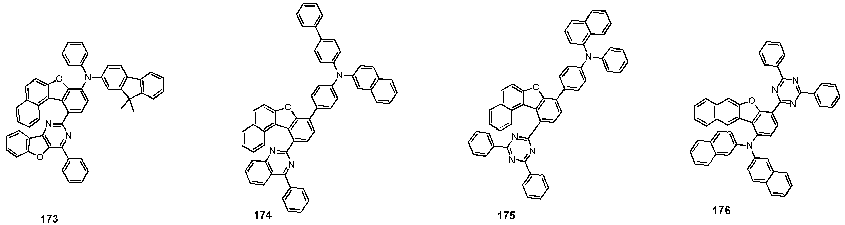

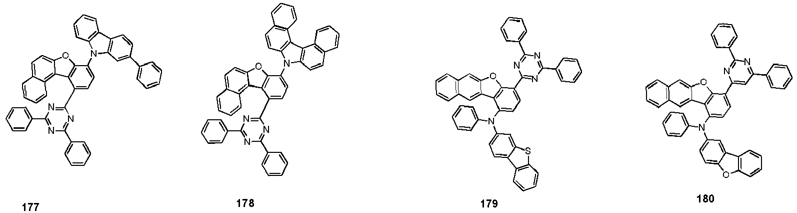

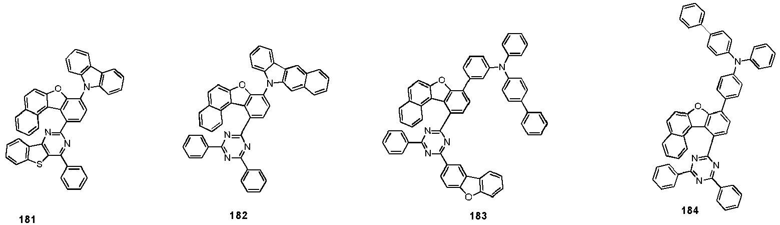

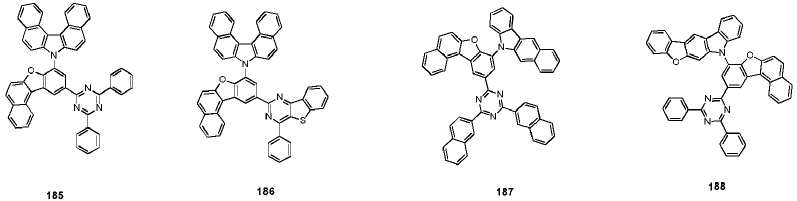

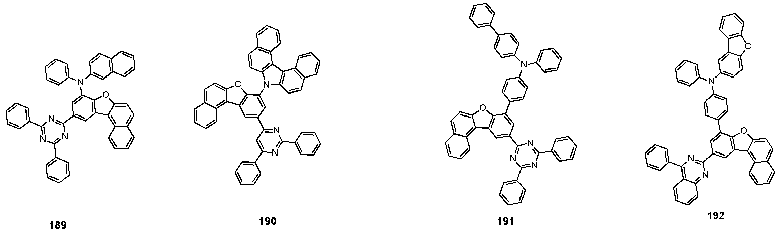

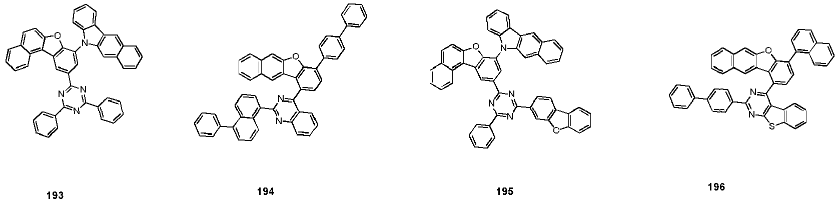

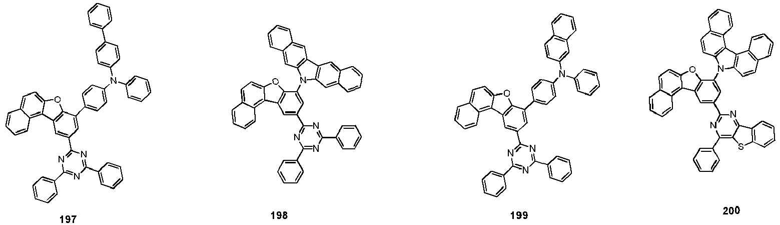

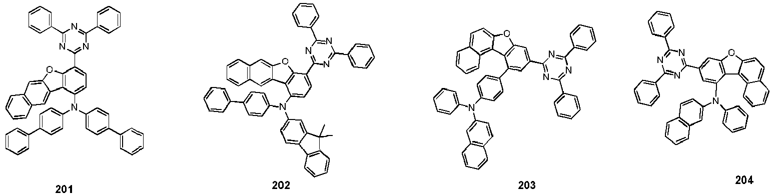

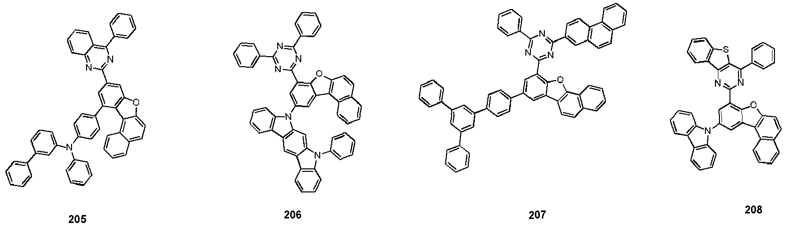

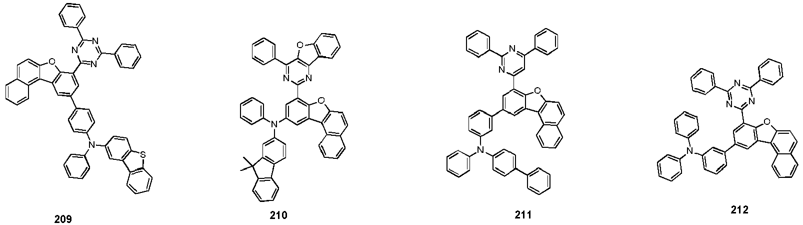

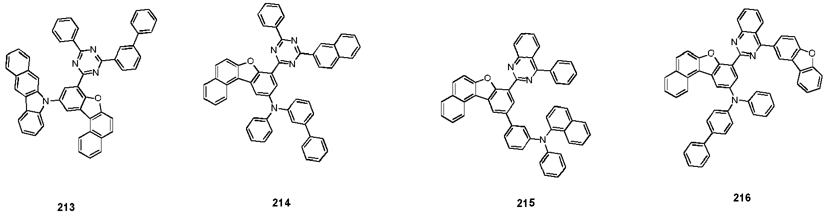

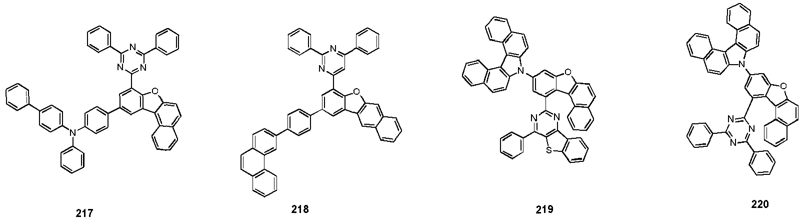

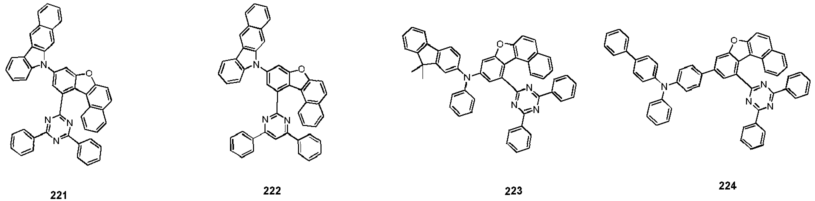

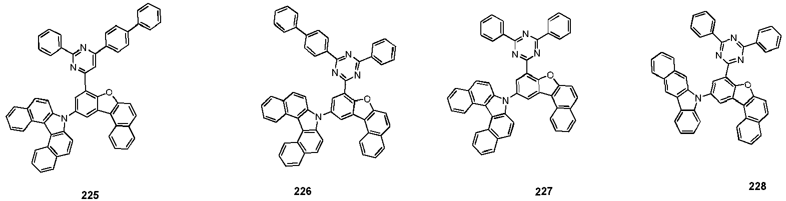

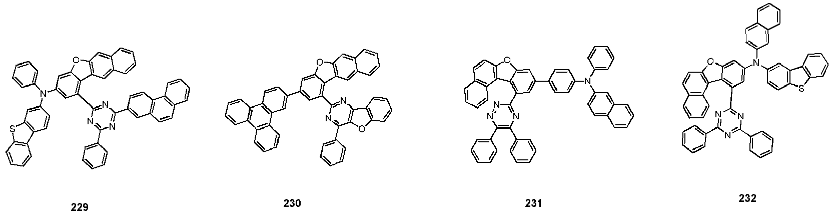

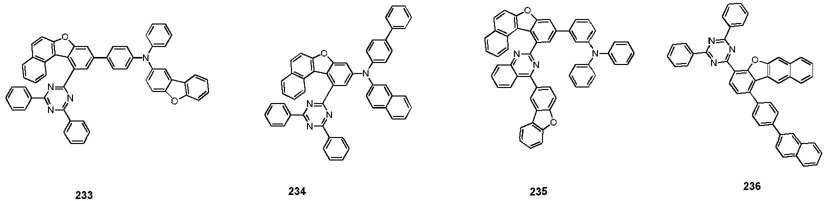

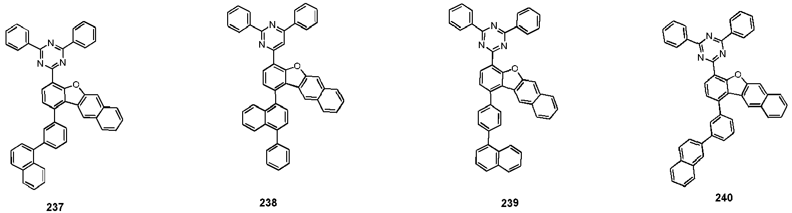

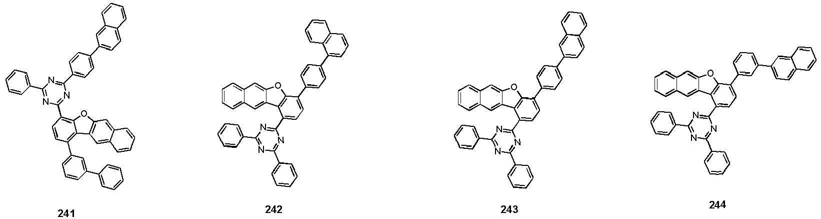

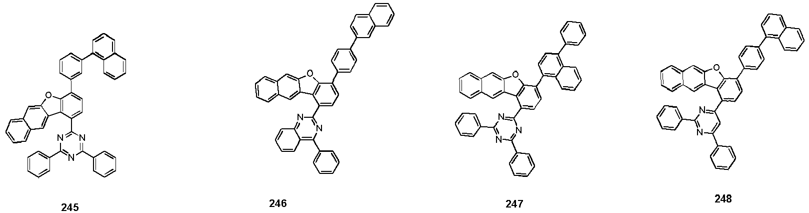

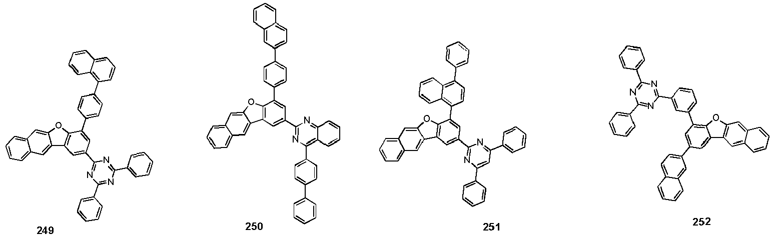

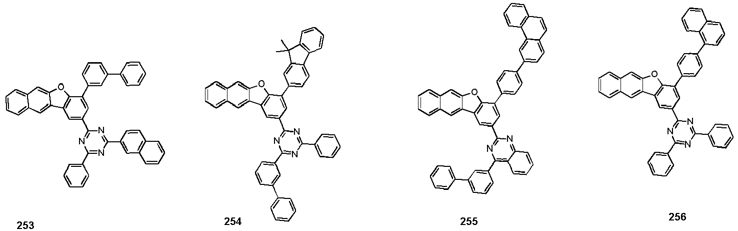

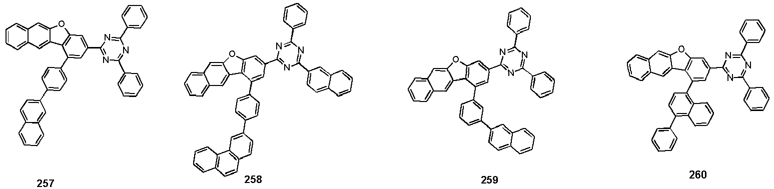

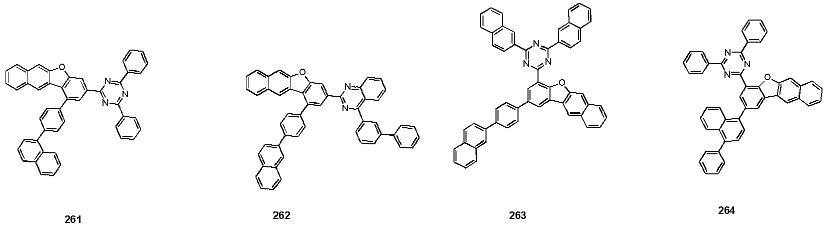

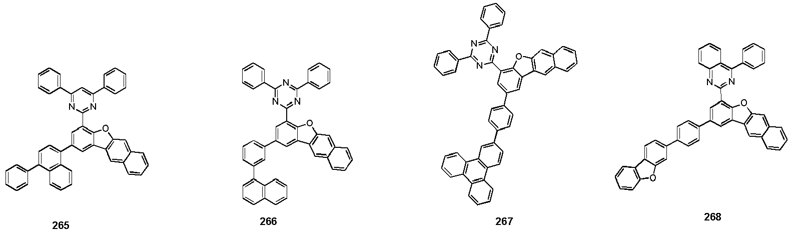

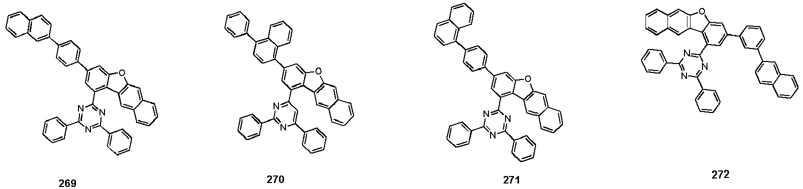

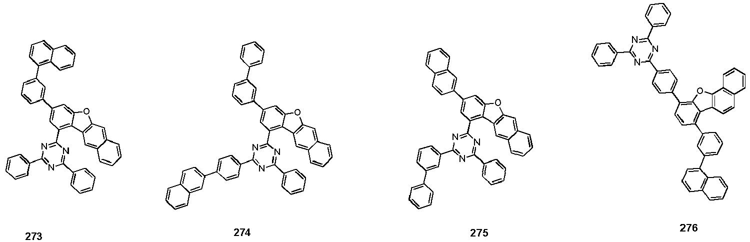

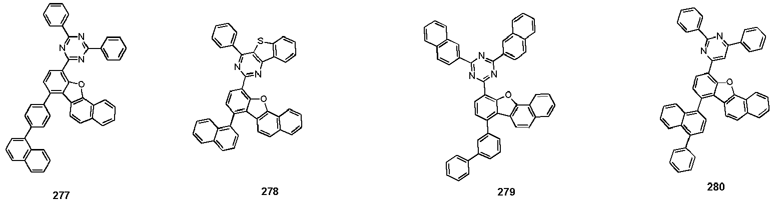

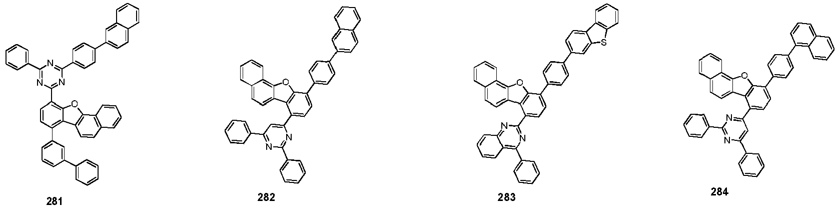

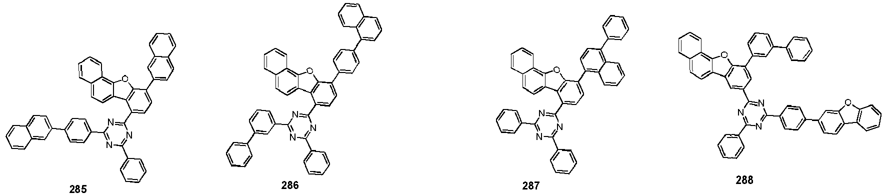

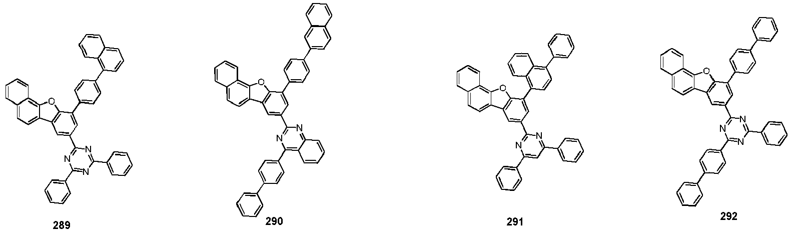

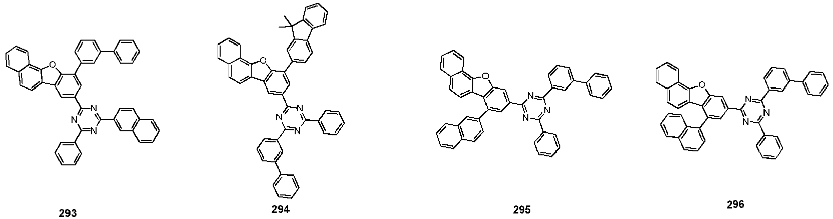

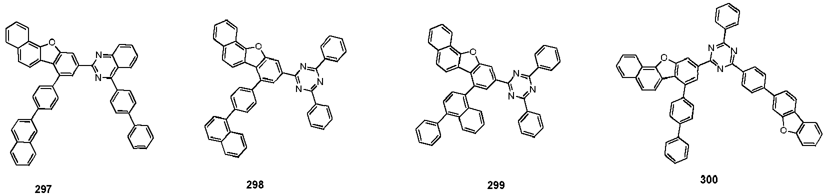

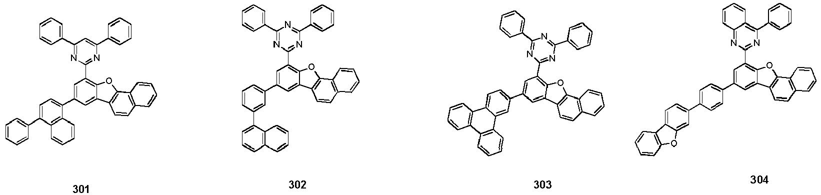

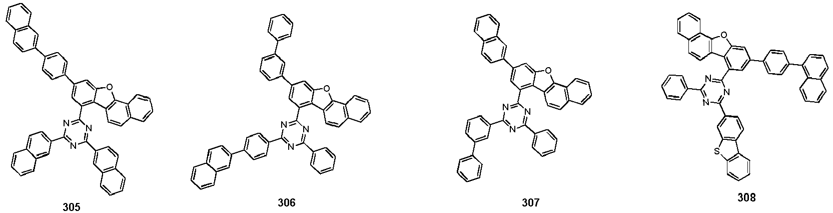

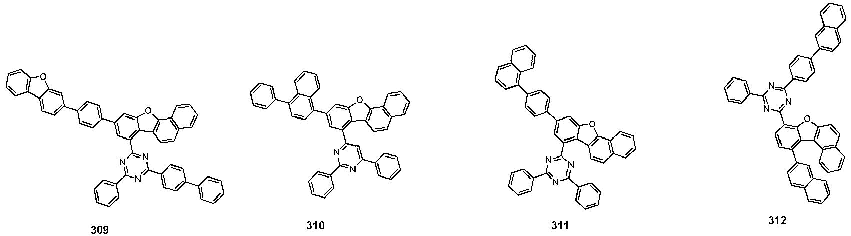

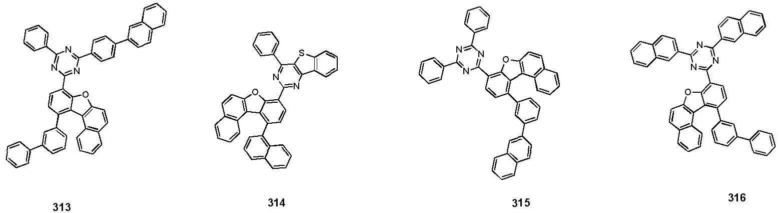

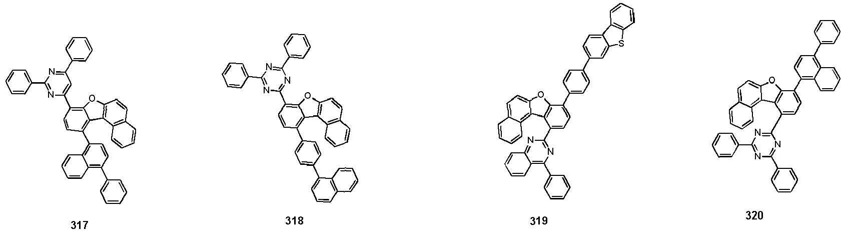

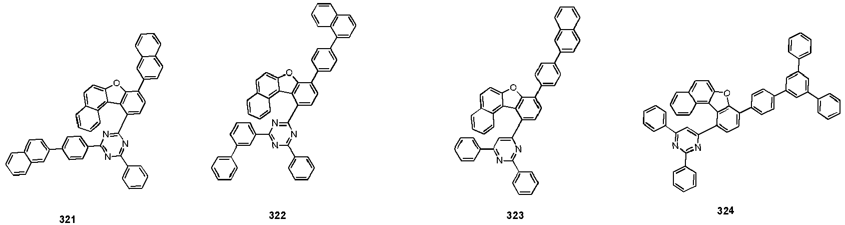

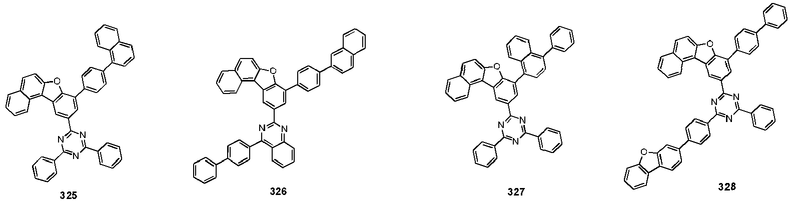

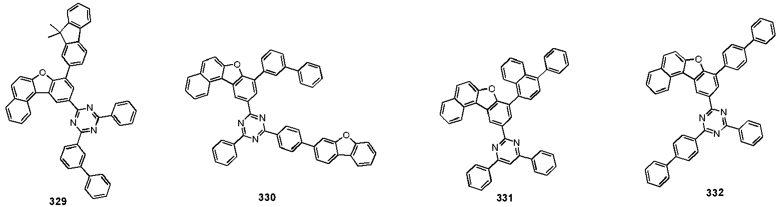

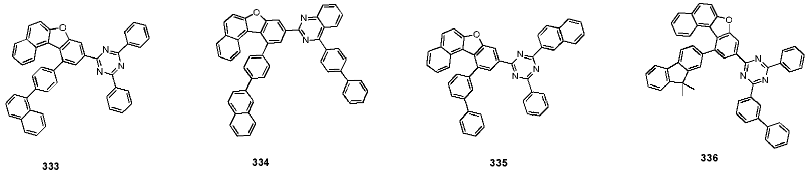

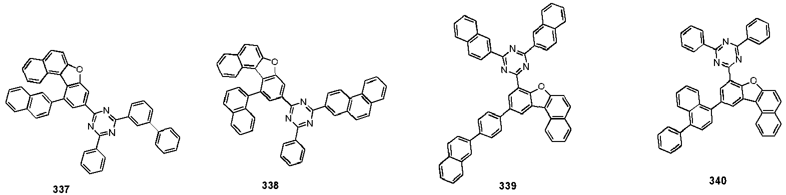

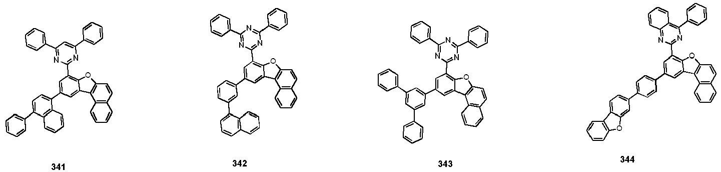

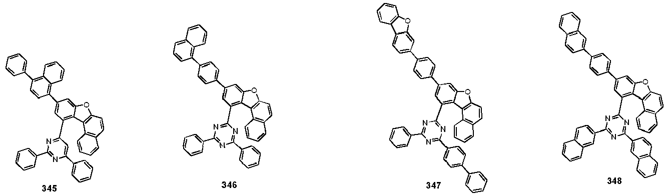

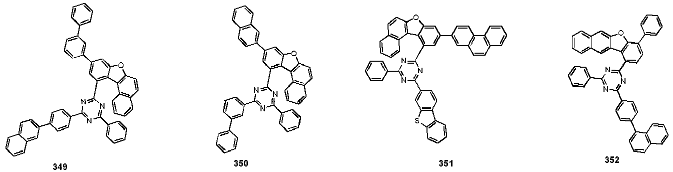

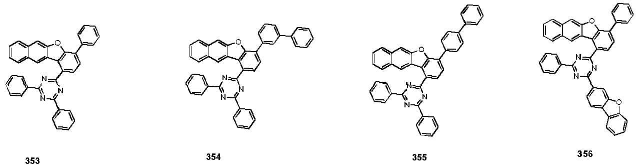

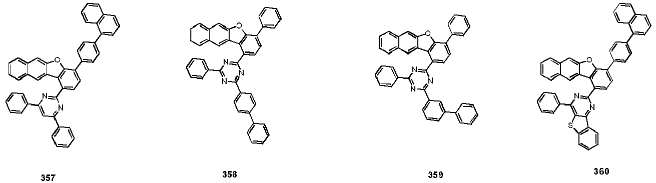

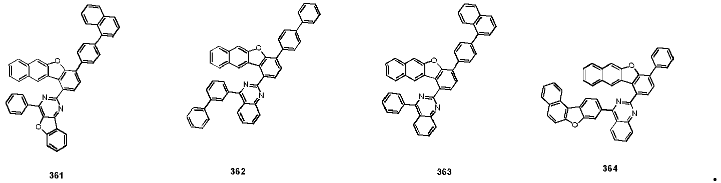

9. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 8.

10. The organic light emitting device of Claim 9, wherein the organic material layer comprises a light emitting layer, and the light emitting layer comprises the heterocyclic compound.

11. The organic light emitting device of Claim 9, wherein the organic material layer comprises a light emitting layer, and the light emitting layer comprises the heterocyclic compound as a host material of a light emitting material.

12. The organic light emitting device of Claim 9, further comprising one, two or more layers selected from the group consisting of a light emitting layer, a hole injection layer, a hole transfer layer, an electron injection layer, an electron transfer layer, a hole auxiliary layer and a hole blocking layer.

13. The organic light emitting device of Claim 9, wherein two or more host materials are pre-mixed in the light emitting layer, and at least one of the two or more host materials comprises the heterocyclic compound as a host material of a light emitting material.

14. A composition for an organic material layer of an organic light emitting device, the composition comprising two or more types of the heterocyclic compound represented by Chemical Formula 1 of Claim 1.

15. The composition for an organic material layer of an organic light emitting device of Claim 14, wherein, in the composition, two types of the heterocyclic compound represented by Chemical Formula 1 have a weight ratio of 1:10 to 10:1.
